(19) Europäisches Patentamt
European Patent Office
Office européen des brevets

(11) EP 3 912 629 A1

(12) EUROPEAN PATENT APPLICATION
published in accordance with Art. 153(4) EPC

(43) Date of publication:
24.11.2021 Bulletin 2021/47

(21) Application number: 19899759.5

(22) Date of filing: 16.12.2019

(51) Int Cl.:
*A61K 35/12* (2015.01) *C12N 15/12* (2006.01)
*A61P 35/00* (2006.01)

(86) International application number:
PCT/CN2019/125565

(87) International publication number:
WO 2020/125576 (25.06.2020 Gazette 2020/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 17.12.2018 CN 201811541631

(71) Applicant: Cure Genetics Co., Ltd
Suzhou, Jiangsu 215123 (CN)

(72) Inventors:
- WANG, Pei
  Suzhou, Jiangsu 215000 (CN)
- JIA, Luying
  Suzhou, Jiangsu 215000 (CN)
- LIN, Yanni
  Suzhou, Jiangsu 215000 (CN)
- NIU, Zhijie
  Suzhou, Jiangsu 215000 (CN)
- XU, Dan
  Suzhou, Jiangsu 215000 (CN)
- FANG, Jiaolong
  Suzhou, Jiangsu 215000 (CN)
- YUAN, Hui
  Suzhou, Jiangsu 215000 (CN)
- LIU, Ting
  Suzhou, Jiangsu 215000 (CN)

(74) Representative: Bardehle Pagenberg Partnerschaft mbB
Patentanwälte Rechtsanwälte
Prinzregentenplatz 7
81675 München (DE)

# (54) METHOD FOR DELIVERING GENE IN CELLS

(57) Disclosed is a method for delivering a gene into target cells, wherein immune cells are used as a vector for virus packaging and transportation to complete the delivery of biomacromolecules among cells, and thus change original characteristics of the target cells or generate new characteristics in the target cells. The gene delivery system, on one hand, can kill target cells such as tumor cells by utilizing the specificity of immune cells; and on the other hand, can modify genes of cells in a lesion by gene delivery to target cells, to directly kill the target cells.



Printed by Jouve, 75001 PARIS (FR)

## Description

### Technical Field

The present invention relates to a gene delivery method, in particular to a method for using immune cells as a vector for virus packaging and transportation to achieve gene delivery to target cells.

### Background Art

Immune cells are one of the most important constituent parts of the human circulatory system. The function of immune cells is to provide defense when pathological, toxicological and other disease processes appear in the body. Therefore, the modification of immune cells to enhance functions thereof has important value with regard to many diseases.

Redirecting an immune system to target and eliminate cancer cells is an important means for treating cancers. The specificity and totipotency of an immune response has always been the direction of this technology that needs to be continuously optimized. Immune cell-mediated tumor suppressor effects *in vivo* and *in vitro,* such as those mediated by T cells, were explored first. In primary T cells, exogenous genes are introduced through retroviral or lentiviral transduction, so that the T cells can specifically recognize tumor-associated antigens, and then, modified T cells are reintroduced into patients to achieve the aim of treatment. This adoptive T cell immunotherapeutic means has been widely studied and applied, wherein the most common means for modifying T cells focuses on a transgenic T cell receptor (tTCR) and a chimeric antigen receptor (CAR). A tTCR is modified from the natural TCR of a T cell, so that the modified T cell (TCR-T) maintains the original structure while also having the characteristics of antigen selectivity and high affinity. A CAR combines the characteristics of a B cell receptor recognizing an antigen and a T cell receptor responding upon stimulation, so that a modified T cell (CAR-T) can effectively attack cancer cells that express a corresponding antigen. To present, CAR therapeutic regimens have achieved a high response rate in the clinical treatment of chronic lymphocytic leukemia. The FDA has approved the application of this cellular immunotherapy regimen with regard to cancers, which has become a watershed in the application of cellular immunotherapy (Nathan S et al., Current Hematologic Malignancy Reports, 2017, 10).

Although CAR-Ts have achieved great success in the treatment of hematologic tumors, such individualized efficacy will vary greatly due to differences in the specific lesion environment and cell quality; especially in the treatment of solid tumors, due to the influence of tumor cell exposure limitations and tumor microenvironment complexity, the efficacy of CAR-T cells will not be as significant as for hematologic tumors. In order to maintain the persistent aggregation of CAR-T cells and the osmotic killing of tumors, this T cell treatment method will be modified in many different ways. This includes the directional guidance of CAR-T cell transportation and infiltration of tumor parenchyma through CCR4 or CCR2b, etc.; increasing the proliferation and persistence of CAR-T cells by co-expressing cytokines (IL-2, IL-7, etc.); in addition, the re-modification of an immunosuppressive microenvironment through the bioinformatics analysis of signal pathways, for example, through signaling pathways mediated by CAT, PD-1, CTL-4, etc. co-expressed in CAR-T cells, to achieve a modification that is more conducive to immunotherapy (Isabelle R et al., Molecular Therapy, 2017, Vol. 25, No 5).

In the above-mentioned methods, in most cases, the adoptively imported membrane surface molecules or secreted molecules of immune cells act on the extracellular molecules of host cells to achieve effects, and cannot enter the host cells and act on the intracellular molecules of the host cells.

Delivering genes to target cells through a viral system is a commonly used method in the field of gene therapy. Lentivirus is a subfamily of retrovirus and is considered to be one of the most effective means for gene delivery. Lentivirus can transfect both dividing cells and non-dividing cells. Viruses firstly need to be packaged in host cells before target cells are transfected with the viruses (Adam S. C et al., Molecular Biotechnology, 2007, 36(3): 184-204).

Lentivirus packaging involves co-transfecting cells with multiple plasmids containing viral elements, and then, after a period of time, the supernatant contains packaged virions secreted by the cells. The plasmid system for packaging viruses is constructed by separating cis-acting elements and sequences encoding trans-acting proteins in a viral genome. A three-plasmid system and a four-plasmid system are common. Taking a three-plasmid system as an example, the three-plasmid system includes a packaging plasmid, an envelope protein plasmid, and a transfer plasmid. Under the control of a CMV promoter, the packaging plasmid (such as psPAX2) expresses all trans-activating proteins required for HIV 21 replication, but does not produce viral envelope proteins and an accessory protein vpu; the envelope protein plasmid (such as pMD2.G) encodes vesicular stomatitis virus G protein (VSV2G), and the use of a pseudotype lentiviral vector of a VSV2G envelope expands the tropism range of the vector with regard to target cells and increases the stability of the vector; and transfer plasmid DNA can transcribe lentiviral genetic material RNA, which includes a target gene. The three plasmids are co-transfected into cells, and when the host genome is expressed, target gene RNA transcribed along with host genes and proteins translated from psPAX2 and pMD2.G genes are assembled into a lentivirus. Viruses are generally collected and purified 3-4 days after co-transfection.

When target cells are infected by viruses, after the viruses enter the target cells, the genetic material RNA of the viruses is reverse-transcripted into DNA, and the gene is then integrated into the genome of the target cells to complete an infection process. Since only the genetic material of the viruses can be integrated into the genome of the target cells and expressed, and the outer shell and membrane protein of the viruses cannot be integrated and expressed, after infection, the viruses cannot repeatedly proliferate in the target cells like normal viruses, and for this reason are harmless to hosts but can efficiently transfect target genes into the genome of the target cells.

Generally, cells used for virus packaging are derived from a HEK293 cell line, which is mainly a cell line obtained by means of the modification, screening or adaptation of human embryonic kidney cells. The most commonly used cell line, 293T, has been widely used in the packaging and production of lentivirus. There is no precedent for the use of immune cells for virus packaging and delivery to target cells.

Immune cells are generally used as target cells for viruses. In primary T cells, exogenous genes are introduced through retroviral or lentiviral transduction, so that the T cells can specifically recognize tumor-associated antigens, and then, modified T cells are reintroduced into patients to achieve the aim of treatment. This adoptive T cell immunotherapeutic means has been widely studied and applied, wherein a TCR-T and a CAR-T are most commonly used. In the preparation of CAR-T cells, a CAR sequence is used as a target gene and is included in the genetic material of a virus; and when a lentivirus infects a T cell, the CAR sequence will be integrated into the genome of the T cell, and other auxiliary co-expression molecules are also generally delivered in the same delivery manner as above, so that the modified T cell can continue to express exogenous genes such as CAR and auxiliary molecules.

In the field of cell therapy and gene therapy, at the present stage, there have been some scientific research attempts regarding intercellular delivery (Ryosuke K et al., Nature communications, 2017 9:1305; Chen-Yuan K et al., Sci. Adv. 2018; 4: eaau6762). However, there is always a need for a more efficient system that can specifically target and effectively attack target cells.

## Summary of the Invention

The present invention uses immune cells as a vector for virus packaging and transportation to complete the intercellular delivery of virus forms, and thus change original characteristics of receptor cells or generate new characteristics in receptor cells.

The present invention discloses the technical solutions shown by the following serial numbers:

1. A method for delivering biomacromolecules to target cells, the method comprising the following steps:

   a) introducing biomacromolecules into immune cells to assemble a delivery system containing biomacromolecules;
   b)bringing the delivery system of step a) into contact with target cells; and
   c)the target cells receiving the biomacromolecules delivered by the delivery system.

2. The method of technical solution 1, wherein the immune cells are T cells, B cells or NK cells.
3. The method of technical solution 2, wherein the immune cells are T cells.
4. The method of technical solution 3, wherein the T cells are CAR-T or TCR-T cells.
5. The method of any one of the preceding technical solutions, wherein with respect to the immune cells, the biomacromolecules are exogenous biomacromolecules.
6. The method of any one of the preceding technical solutions, wherein the biomacromolecules are selected from one or more of a polypeptide, a protein and a genetic material.
7. The method of technical solution 6, wherein the genetic material is DNA and/or RNA.
8. The method of technical solution 7, wherein the RNA is selected from one or more of mRNA, siRNA and gRNA.
9. The method of technical solution 7, wherein the RNA is gRNA.
10. The method of technical solution 9, wherein the gRNA is sgRNA or a duplex composed of crRNA and tracRNA.
11. The method of technical solution 9 or 10, wherein the gRNA and a coding gene encoding a Cas protein are delivered to the target cells.
12. The method of technical solution 7, wherein the DNA is selected from one or more of linear DNA, single-stranded DNA and plasmid DNA.
13. The method of technical solution 6, wherein the biomacromolecules are surface molecules, surface antigens, secreted molecules, or cytokines.
14. The method of technical solution 13, wherein the biomacromolecules are CD19 surface molecules or CCL19 secreted molecules.
15. The method of any one of the preceding technical solutions, wherein the method is performed *in vitro* or on *ex-*

*vivo* cells.

16. The method of any one of technical solutions 1-14, wherein step a) is performed *in vitro,* and steps b) and c) are performed *in vivo.*
17. The method of any one of technical solutions 12-16, wherein the plasmid is a plasmid for assembling a virus.
18. The method of any one of technical solutions 1-17, wherein the delivery system is a viral system.
19. The method of technical solution 18, wherein the viral system is selected from one or more of an adeno-associated virus, an adenovirus, a retrovirus, a lentivirus, a rabies virus and a herpes virus.
20. The method of technical solution 19, wherein the viral system is a lenti-virus.
21. The method of any one of technical solutions 1-16, wherein the delivery system is an exosome.
22. The method of technical solution 21, wherein the exosome, which encapsulates the polypeptide, protein and/or genetic material, is released to outside the immune cells and comes into contact with the target cells.
23. The method of any one of technical solutions 12-22, wherein the plasmid further comprises a regulatory element.
24. The method of any one of the preceding technical solutions, wherein the biomacromolecules of step a) are introduced into the immune cells by means of electrotransfection, or introduced into the immune cells by means of chemical reagents.
25. The method of any one of the preceding technical solutions, wherein the target cells are tumor cells, pathogens, stem cells or somatic cells.
26. The method of technical solution 11, wherein the Cas protein is selected from Cas1, Cas1B, Cas2, Cas3, Cas4, Cas5, Cas6, Cas7, Cas8, Cas9, Cas10, Cas12 and Cas13, and mutants of the above-mentioned proteins, and fusion proteins or protein complexes including these proteins or mutants thereof.
27. The method of technical solution 26, wherein the Cas9 protein is a Cas9 protein derived from *Streptococcus pyogenes.*

The delivery system of the present invention can be a viral system or an exosome system. According to the proposed method, an intercellular delivery system (such as virus forms) can be produced via immune cells. Viruses can infect target cells, deliver exogenous genetic materials carried by the viruses, label target cells or edit the genes of target cells, or regulate the microenvironment in which target cells are located. The method for delivering genetic materials to target cells of the present invention can be used in a variety of applications, including research applications, diagnostic applications, industrial applications, and therapeutic applications, and, for example, can be used for studying the effects on the development, metabolism, expression, etc. of downstream genes. The method can also be used for studying tumor models. After immune cells specifically recognize tumor cells, the immune cells can be activated to induce the production of a delivery system. The characteristics of tumor cells and a tumor microenvironment can be studied by means of materials, comprising, for example, active molecules, genetic materials, and even gene editing tools, packaged by the delivery system.

Compared with existing cell therapy methods, for example, CAR-Ts, which only act on extracellular targets, the method of the present invention can achieve a broader scope of utility; in addition, the broad applicable characteristics of a virus itself provide more choice for optimization tools for immune cell therapy. According to the virus packaging method and the method for delivering biomacromolecules to target cells proposed in the present invention, immune cells are used as an intermediary vector to deliver macromolecules or macromolecular complexes with biomedical effects, thereby realizing the modification of target cells and realizing disease monitoring or treatment. Specifically, there are the following three advantages:

(1) immune cells (for example, T cells) specifically targeting target lesions ensures virions, which are secondary delivery vectors produced by the immune cells, can take effect at critical lesions. Virions can contain tumor labeling agents such as Ig antibodies coupled to GFP (green fluorescent proteins) or related antibody drugs, so as to realize the labeling of lesions. The present invention can be used for diagnosis, or for adjuvant therapy assisting surgeries, or for the direct delivery of anti-tumor drugs, etc.
(2) immune cells (for example, T cells) specifically targeting target lesions ensures virions, which are secondary delivery vectors produced by the immune cells, can take effect at critical lesions. Virions can contain, for example, a CRISPR gene editing system, which edits the PDL1 gene, so as to regulate tumor survival/growth genes, or edits drug resistance genes, or anti-immune suppression genes, or genes in other cells in a tumor microenvironment which promote tumor growth. Such a gene-editing method with combined delivery by immune cells and secondary virus has the specificity rendered by immune cells, and at the same time, a secondary vector virus realizes the delivery of the CRISPR system, and then modifies the genes of the cells in the lesion, so as to achieve the direct killing of tumor cells, or enhance the efficacy of drugs such as tumor-killing drugs, especially targeted therapy, tumor immunotherapy, and cell therapies such as CART, achieving combination therapy, or enhancing the efficacy of existing drugs, or reducing the toxicity of existing drugs.
(3) immune cells (for example, T cells) specifically targeting target lesions ensures cell secretions such as exosomes, which are secondary delivery vectors produced by the immune cells, can be produced and take effect at critical lesions. Gene editing proteins/RNA molecules were linked to sequences that can enable localization to exosomes (such as exosome-associated tetraspanin protein CD9, or Lamp2b, or C-terminal fusion of the C1C2 domain from MFG-E8), which can make these exosomes contain, for example, a CRISPR gene editing system, which edits the PDL1 gene, so as to regulate tumor survival/growth genes, or edits drug resistance genes, or anti-immune suppression genes, or genes in other cells in a tumor microenvironment which promote tumor growth. Such a gene-editing method with combined delivery by immune cells and secondary exosomes has the specificity rendered by immune cells, and at the same time, a secondary vector exosome realizes the delivery of the CRISPR system, and then modifies the genes of the cells in the lesion, so as to achieve the direct killing of tumor cells, or enhance the efficacy of drugs such as tumor-killing drugs, especially targeted therapy, tumor immunotherapy, and cell therapies such as CART, achieving combination therapy, or enhancing the efficacy of existing drugs, or reducing the toxicity of existing drugs.

**Drawings of the Description**

Figure 1: pLenti-GFP plasmid map

Figure 2: A schematic view of the construction of stably transfected 293T-dsRed cell line

Figure 3: Fluorescence detection of experimental group cells, wherein column 1 is a photograph of cells in a white light field; column 2 is a photograph of cells with red fluorescence excitation; and column 3 is a photograph of cells with green fluorescence excitation.

Figure 4: Fluorescence detection of control group cells, wherein column 1 is a photograph of cells in a white light field; column 2 is a photograph of cells with red fluorescence excitation; and column 3 is a photograph of cells with green fluorescence excitation. See Table 2 for group number.

Figure 5: Flow cytometry analysis of a single-cell group, wherein column 1 relates to total cells; column 2 relates to red fluorescence sorting, wherein the boxes on the left show cells with a negative red fluorescence signal, and the boxes on the right show cells with a negative red fluorescence signal; and column 3 relates to green fluorescence sorting, wherein the boxes on the left show cells with a negative green fluorescence signal, and the boxes on the right show cells with a negative green fluorescence signal. See Table 2 for group number, wherein S represents cells in a suspension of the group, and A represents adherent cells of the group.

Figure 6: Flow cytometry analysis of an experimental group, wherein column 1 relates to total cells; column 2 relates to red fluorescence detection of the total cells, wherein the boxes on the left show cells with a negative red fluorescence signal, and the boxes on the right show cells with a negative red fluorescence signal; column 3 relates to green fluorescence detection of cells with a negative red fluorescence signal, wherein the boxes on the right show cells with a positive green fluorescence signal; and column 4 relates to green fluorescence detection of cells with a positive red fluorescence signal, wherein the boxes on the right show cells with a positive green fluorescence signal. See Table 2 settings for group number, wherein S represents cells in a suspension of the group, and A represents adherent cells of the group.

Figure 7: Flow cytometry analysis of a control group, wherein column 1 relates to total cells; column 2 relates to red fluorescence detection of the total cells, wherein the boxes on the left show cells with a negative red fluorescence signal, and the boxes on the right show cells with a negative red fluorescence signal; column 3 relates to green fluorescence detection of cells with a negative red fluorescence signal, wherein the boxes on the right show cells with a positive green fluorescence signal; and column 4 relates to green fluorescence detection of cells with a positive red fluorescence signal, wherein the boxes on the right show cells with a positive green fluorescence signal. See Table 2 for group number, wherein S represents cells in a suspension of the group, and A represents adherent cells of the group

Figure 8: The above table shows statistics compiled on the basis of results of flow cytometer analysis, and the following graph shows the efficiency of infecting 293T-dsRed with virions packaged by Jurkat cells calculated from GFP expression and a dsRed tag. See Table 2 for group number.

Figure 9: Shuttle plasmid map

Figure 10: Screening plasmid map for the construction of a 293T-Cas9 cell line

Figure 11: Editing effect of target sites in a 293T-Cas9 cell line. The control group is the analysis of the EGFR gene editing efficiency in cells which have not been transfected with sgRNA, and the experimental group is the analysis of the gene editing efficiency in cells that have been transfected with sgRNA targeting EGFR.

Figure 12: Cell fluorescence detection, wherein column 1 is a photograph of cells in a white light field; and column 2 is a photograph of cells with green fluorescence excitation

Figure 13: Gene editing efficiency detection (Surveyor assay)

Control G/C represents a positive control in a kit, which proves the feasibility of the detection method; NC represents a negative control, which only amplifies a target gene fragment to indicate the position of an original fragment; and G3-1-S, G3-2-S, G3-1-A, G3-2-A, G3-3 and G3-0 indicate a detection result of the gene editing efficiency of cells in each experimental group.

Figure 14: pELPs-CD19 plasmid map

Figure 15: A and B are the results of further culturing the adherent cells in Example 3 for one week and then analyzing the cells with a flow cytometer.

Figure 16: A, B and C are the results of further culturing the adherent cells in Example 4 for one week and then analyzing the cells with a flow cytometer.

Figure 17: The results of further culturing the adherent cells in Example 5 for one week and then analyzing the cells with flow cytometer.

Figure 18: pBD30-CD63-Nluc plasmid map

Figure 19: pBD60-Booster plasmid map

Figure 20: Exosomal luciferase detection: Figure A is the fluorescence value in the wavelength range of 400-600 nm; and Figure B is the fluorescence value at 460 nm, wherein a vertical coordinate RLU (relative light unit) represents the relative test value of the amount of light produced in a sample.

## Detailed Description of the Invention

The present invention is described in detail herein with reference to the following definitions and examples. The content of all the patents and published documents referred to herein, comprising all the sequences disclosed in these patents and published documents, are expressly incorporated herein by way of reference.

### Immune cells

"Immune cells" are used as "donor cells" in the present invention.

The immune cells described herein refer to cells that can recognize antigens and produce specific immune responses, such as T cells, B cells, natural killer cells (NK) cells, macrophages, modified immune cells, etc.

In tumor immune response, cellular immunity mediated by T cells plays a major role. T cells recognize tumor antigens via T cell receptors (TCRs), thus activating themselves and killing tumor cells. The most common means for modifying T cells focuses on a transgenic T cell receptor (tTCR) and a chimeric antigen receptor (CAR). A tTCR is modified from the natural TCR on a T cell, so that the tTCR maintains the original structure while also having the characteristics of antigen selectivity and high affinity. The modified T cell is TCR-T. A CAR combines the characteristics of a B cell receptor recognizing an antigen and a T cell receptor responding upon stimulation, so that a modified T cell (CAR-T) can effectively attack cancer cells that express a corresponding antigen.

In a classic CAR, scFv fragments of monoclonal antibodies that specifically recognize tumor antigens are implanted on T cells or NK cells. For example, retroviral vectors can be used to introduce CAR-encoding nucleic acids into T cells, NK cells, or NKT cells. In this way, a large number of cancer-specific T cells, NK cells, or NKT cells can be produced for adoptive cell transfer. Early clinical studies of this method have shown efficacy in some cancers.

### Target cell

A "target cell" is also referred to as a "host cell" or a "receptor cell" in the present invention.

A target cell is a cell that can be specifically recognized by an immune cell, for example, the target cell can be a tumor cell, an allogeneic cell, and can also be a pathogen, or other immune cells that present alloantigens. The surfaces of the target cell all have antigen expression different from an autoantigen.

Any type of cells may become a target cell, for example, a stem cell, fsuch as an embryonic stem (ES) cell, an induced pluripotent stem (iPS) cell, and a germ cell; and a somatic cell, such as a fibroblast, a hematopoietic cell, a neuron, a muscle cell, a bone cell, a liver cell, and a pancreatic cell.

### Biomacromolecule

In the present invention, biomacromolecules refer to a polypeptide, a protein and a genetic material.

The term "genetic material" refers to a polymerized form of nucleotides having any length, that is, a polynucleotide sequence, which is a polymerized form of deoxyribonucleotides or ribonucleotides, or analogs thereof. A polynucleotide can have any three-dimensional structure and can perform any known or unknown function. The following are non-limiting examples of a polynucleotide: a coding region or a non-coding region of a gene or a gene fragment, an exon, an intron, messenger RNA (mRNA), transfer RNA, ribosomal RNA, short hairpin RNA (shRNA), micro-RNA (miRNA), silent RNA (siRNA), guide RNA (gRNA), a ribozyme, cDNA, a recombinant polynucleotide, a branched polynucleotide, a plasmid, a vector, isolated DNA of any sequence, isolated RNA of any sequence, a nucleic acid probe and a primer. A polynucleotide may contain one or more modified nucleotides, such as methylated nucleotides and nucleotide analogs. If present, modification of a nucleotide structure can be carried out before or after the assembly of a polymer. The sequence of a nucleotide can be interrupted by non-nucleotide components. Polynucleotides can be further modified after polymerization, for example, by conjugation with labeled components.

The protein, which acts as a biomacromolecule, of the present invention can be a surface molecule, a surface antigen (such as surface antigen CD19), a secreted molecule, and a cytokine, for example, CCL19, etc.

A surface molecule refers to a molecule located near the surface of a cell. Generally, a microscope combined with staining or other positioning techniques can be used to observe that the molecule is located on the cell membrane, or the molecule can be detected by means of flow cytometry when the cell membrane is intact. The surface molecule can be a membrane protein, and the membrane protein can be integrated on the cell membrane or interact/bind with the cell membrane. The membrane protein can be modified, e.g. by means of adding fatty acid chains or through prenylation. The membrane proteins are divided into several categories, including an integral membrane protein and a peripheral membrane protein. The integral membrane protein is a protein integrated on the membrane. Generally, surfactants (such as SDS) or other non-polar solvents need to be added before the protein can be separated from the membrane. A transmembrane protein belonging to the integral membrane protein can have a transmembrane domain, and a unidirectional integral membrane protein can be bound to the membrane from only one direction (outside or inside the membrane) and partially inserted into the membrane. The peripheral membrane protein is a protein that can temporarily bind to or interact with membrane or other membrane proteins. A class of common surface molecules are clusters of differentiation, also called population of differentiation or CD for short, which provides a target for immunophenotypic analysis/immunotyping. Many molecules in cluster of differentiation are ligands, receptors, or cell adhesion molecules. Surface antigen CD19 is one type of molecule in a cluster of differentiation.

A secreted molecule refers to a molecule secreted by a cell and can be secreted through different secretory pathways. A secreted molecule generally has the ability to leave a cell, and includes a cytokine, a hormone, a growth factor, etc. A secreted molecule can be located in a vesicle and transported via a vesicle. Cytokines are a class of important secreted molecules, and are usually a class of proteins that play a role in cell signal transduction. There are many types of cytokines, including chemokines, interferons, interleukins, lymphokines, tumor necrosis factors, etc. CCL19 is one type of cytokine. In the present invention, the biomacromolecule introduced into an immune cell can be a biomacromolecule different from those in the immune cell, i.e., an exogenous biomacromolecule; and the biomacromolecule can also be the same as the biomacromolecule produced and expressed by the immune cell itself.

## Plasmid

The genetic material of the present invention can be directly introduced into an immune cell, for example, linear DNA can be directly introduced into an immune cell; or the genetic material of the present invention can be introduced into an immune cell via a constructed plasmid.

The plasmid contains the genetic material that needs to be delivered. The genetic material can be DNA or RNA, and the genetic material is used by an immune cell for the production or enhancement of a delivery system.

The plasmid may contain a helper plasmid required for virus packaging and a shuttle plasmid with a target molecule gene; the target molecule gene can be a tracer protein (such as GFP) or other effector molecules related to cell functions (such as cytokines), and can also be a gene editing system (such as a CRISPR system);
and the plasmid can also contain a helper plasmid needed to promote exosome packaging, secretion, and orientation, and a plasmid with a target molecule gene.

The plasmid may also contain one or more regulatory elements selected on the basis of a host cell to be used for expression. The regulatory elements are operably linked to a nucleic acid sequence to be expressed. In a recombinant plasmid, "operably linked" is intended to mean that a nucleotide sequence of interest is linked to the one or more regulatory elements in a manner that allows the expression of the nucleotide sequence (for example, in an *in vitro* transcription/translation system or in a host cell when the vector is introduced into the host cell).

## Viral system

The viral system used as a delivery system in the present invention can be a lentivirus, an adeno-associated virus (AAV), an adenovirus, a retrovirus, a rabies virus and a herpes virus.

Viruses are infectious microorganisms that can generally only replicate in living cells or other biological organisms. Viruses have a wide and varied spectrum of infection, including animals, plants, and microorganisms. Different viruses have very different shapes and sizes. Generally, a complete virion will contain a long nucleic acid strand, which acts as genetic material thereof, and a protective protein shell called a capsid, wherein capsid protein is generally encoded by viral genetic material. In addition, viruses can generally also obtain a lipid envelope from the membrane of the infected host cell. This envelope contains a protein encoded by a viral genome or a host cell genome, and this envelope plays an important role in the infectivity of the virus.

The genetic material of viruses can be a DNA or RNA genome; therefore, viruses can be divided into DNA viruses and RNA viruses. Viral genomic DNA or RNA can be either single-stranded or double-stranded, and at the same time, can also be circular, linear or even fragmented. Adenoviruses are double-stranded DNA (dsDNA) viruses. In view of the infectivity thereof with regard to dividing and non-dividing cells and the ability thereof to carry a large number of exogenous genes, adenoviruses are often used as a viral vector in gene therapy. In addition, the use of oncolytic adenovirus in cancer treatment has been approved in China. Adeno-associated virus (AAV) is a single-stranded DNA (ssDNA) virus. The immune response induced thereby in the human body is very weak, and same has a variety of serological subtypes that are specifically infectious to different tissues and organs, and therefore, adeno-associated virus has obvious advantages in gene therapy. At present, AAV has been widely used in clinical trials of gene therapy, including hemophilia, spinal muscular atrophy, and Parkinson’s disease, etc. Some common viruses such as herpes simplex virus (HSV) are also DNA viruses, and are considered to be useful for the potential treatment of cancers due to the oncolytic ability thereof.

In addition, there are also many types of RNA viruses, for example, rabies virus is a highly pathogenic and highly infectious RNA virus. The use of the virus with reduced toxicity as a vaccine can bring about immunity to the virus in the body. Retroviruses are common single-stranded RNA viruses. After invading a host cell, a retrovirus transcripts its own RNA genome in reverse to generate DNA, and integrates the DNA into the genome of the host cell. The genetic information of the virus will be transcribed and translated together with the genes of the host cell, and then assembled into a new virus. Retroviruses have been successfully used in gene delivery systems due to the high capacity and wide spectrum of infection thereof. Lentivirus is a genus of retrovirus. Lentivirus is named because lentivirus can lead to chronic fatal diseases after undergoing a long latent period. The most well-known lentivirus is human immunodeficiency virus (HIV). A lentivirus can integrate a large amount of genes into a host cell genome to obtain stable expression, and can efficiently infect a variety of cell types, and therefore, lentiviruses have become the most effective method for gene delivery.

Exosome

An exosome is an important constituent part of an extracellular vesicle, and is a membrane structure of 30-100 nanometers. An exosome originates from an endosome. In view of a membrane closed structure of the exosome and encapsulated cytoplasmic components, the exosome plays an important role in intercellular interactions.

Exosomes can be shed off from and produced by almost all cells in the human body, and exosomes have many functions under different physiological and pathological conditions. Compared with non-cancer cells, cancer cells will produce more exosomes. These exosomes can promote tumor generation and metastasis by regulating anti-tumor immune mechanisms, e.g. by inducing drug resistance, promoting angiogenesis, changing a tumor microenvironment to cause immunosuppression, autocrine to promote cell proliferation and metastasis, etc. In contrast, it has also been reported that exosomes use immunostimulatory effects to produce beneficial therapeutic effects, such as activating an anti-tumor innate immune response and promoting the presentation of tumor antigens to the immune system. Therefore, exosomes, as an important form of intercellular communication, and due to the special performance thereof with regard to tumor cells, are not only an important means for assisting treatment, but also a candidate as drug targets.

At present, there are many application studies about and clinical trials on exosomes, mainly including using same as vaccines or delivery tools. For example, exosomes carrying tumor-specific antigens can be produced by autoimmune cells (such as dendritic cells, DC) or tumor cells of patients by means of spontaneous secretion or secretion via stimulation, and these exosomes can be reintroduced into the patient to activate an immune response against the corresponding condition; in addition, exosomes, as an intercellular transport vector with extremely high biocompatibility, can be used to directionally package therapeutic drugs, including chemical small molecule drugs, therapeutic biomacromolecules and even therapeutic viral particles. There have been many studies on the packaging of specific biomacromolecules by means of exosomes, including the transfection of production cells using specific exogenous genes and the enhancement of the packaging directionality and secretion modification of exosomes, and the subsequent collection and purification of exosomes to further promote the application and evaluation thereof in disease treatment. The packaging of virions is based on using cells to produce virions, promoting the production, by cells, of exosomes that can encapsulate the virus and using a special process to collect the exosomes and verify the function thereof.

CRISPR/Cas System

The CRISPR/Cas system is a nuclease system formed by a clustered regularly interspaced short palindromic repeat (CRISPR) sequence and CRISPR-binding protein (i.e., a Cas protein), which system is capable of cleaving almost all genomic sequences adjacent to protospacer-adjacent motifs (PAM) in eukaryotic cells (Cong et al., Science, 2013, 339: 819-823). "CRISPR/Cas system" collectively refers to transcripts involved in CRISPR-associated ("Cas") genes, and other elements involved in the expression of or directing the activity of CRISPR-associated genes, including sequences encoding a Cas gene, a tracr (trans-activated CRISPR) sequence (e.g. tracrRNA or active partial tracrRNA), a tracr mate sequence (encompassing a "direct repeat" and a processed partial direct repeat in the context of an endogenous CRISPR system), a guide sequence or other sequences and transcripts from a CRISPR locus. In general, a CRISPR system is characterized by elements that promote the formation of a CRISPR complex at the site of a target sequence (also referred to as a protospacer in the context of an endogenous CRISPR system).

CAS proteins can be divided into four different functional modules: a target recognition module (interval acquisition), an expression module (crRNA processing and target binding), a interference module (target cleavage) and an auxiliary module (regulatory and other CRISPR-related functions). A CRISPR-associated endonuclease Cas protein can target specific genomic sequences through guide RNA (gRNA). The Cas protein of the present invention is a Cas protein with a DNA binding function, such as a natural Cas protein, a mutated Cas protein, such as a Cas protein in which, after mutation, nuclease is inactivated (dead Cas, dCas).

Non-limiting examples of Cas proteins include: Cas1, Cas1B, Cas2, Cas3, Cas4, Cas5, Cas6, Cas7, Cas8, Cas9 (also known as Csn1 and Csx12), Cas10, Cas12, Cas13, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, and Csf4, homologous proteins in different species thereof, endonuclease-inactivated mutant proteins (dCas proteins), or modified versions thereof.

In some embodiments, the Cas protein is a Cas9 protein. A Cas9 variant can be a Cas9 endonuclease that does not naturally exist in nature and that is obtained by means of protein engineering or by means of random mutagenesis. Cas9 variants of the present invention can, for example, be obtained by means of mutations, i.e. the deletion of, or insertion of or substitution of at least one residue in the amino acid sequence of a *Streptococcus Pyogenes* Cas9 endonuclease (COG3513). In some embodiments, the Cas9 protein is *Streptococcus pneumoniae, Streptococcus pyogenes,* or *Streptococcus thermophilus* Cas9, and may include mutated Cas9 derived from these organisms, or variants with other amino acid sequences linked to Cas9, and these Cas9 are known; for example, the amino acid sequence of the *Streptococcus pyogenes* Cas9 protein may be found in the SwissProt database under the accession number Q99ZW2; the amino acid sequence of *Neisseria meningitidis* Cas9 protein may be found in the UniProt database under number A1IQ68; the amino acid sequence of the *Streptococcus thermophilus* Cas9 protein may be found in the UniProt database under number Q03LF7; and the amino acid sequence of *Staphylococcus aureus* Cas9 protein may be found in the UniProt database under number J7RUA5.

Nuclease-inactivated mutant proteins (dCas proteins) are a variant, obtained through mutation, of the Cas protein. The endonuclease activity of the mutant Cas protein is inactivated or substantially inactivated, so that the Cas protein loses or substantially loses endonuclease activity, and is thus unable to cleave a target sequence. The non-limiting examples of the Cas proteins listed above can all be modified, via mutation, into a dCas protein by means of the inactivation of endonuclease, said mutation comprising the insertion, deletion or substitution, etc. of one or more amino acid residues.

For example, certain Cas9 mutations can cause a Cas9 protein to lose or substantially lose endonuclease activity, thereby making the protein unable to cleave a target sequence. For Cas9 of a certain species, such as SpCas9, exemplary mutations that reduce or eliminate endonuclease activity comprise one or more mutations at the following positions: D10, G12, G17, E762, H840, N854, N863, H982, H983, A984, D986 or A987. The literature proves that guide RNA (gRNA)-mediated endonuclease-inactivated Cas9 (called dCas9) can lead to the inhibition of the expression of E. coli-specific endogenous genes and EGFP reporter genes in human cells (Qi et al. Cell, 2013, 152: 1173-1183). This study proves that the use of a gRNA-mediated dCas9 technology can accurately recognize and bind to the corresponding genome.

A dCas protein can be a variant that does not naturally exist in nature and that is obtained by means of protein engineering or by means of random mutagenesis, and the endonuclease activity thereof is inactivated or substantially inactivated. The corresponding dCas9 protein can, for example, be obtained by means of mutations, i.e. the deletion of, or insertion of or substitution of at least one residue in the amino acid sequence of an *Streptococcus Pyogenes* Cas9 endonuclease.

A Cas protein that acts on two strands of DNA generally has two endonuclease active sites. If there is only one site that is mutated or deleted to cause the enzymatic activity of the site to be deactivated, then Nickase, such as Cas9-nickase, that cleaves a single strand of DNA can be obtained, and is widely used because of the high fidelity rate thereof and the characteristics of cleaving a DNA single-strand.

In addition, a Cas protein variant which is screened by means of mutation having one or more point mutations is proved to be able to improve the specificity of the Cas protein variant, reduce the off-target rate of genome editing, or make the Cas protein variant compatible with more PAM sequences, such as eSpCas9, SpCas9-HF, HeFSpCas9 and HIFI-SpCas9, xCas9 (Christopher A. V et al., Nature Medicine, 2018(24), pp 1216-1224; Johnny H. H et al., Nature, 2018, 556 (7699), pp 57-63), etc.

Considering that a Cas protein contains multiple functional modules and the protein sequence thereof is relatively long, in order to facilitate packaging and transportation and function control, the protein sequence thereof will also be truncated by means of protein engineering research to form a Cas protein system, for example, a Split-SpCas9 system containing a complex formed by truncated proteins (Jana Murovec et al., Plant Biotechnology Journal, 2017, 15, pp. 917-926).

In some embodiments, the Cas protein is a fusion protein or a protein complex containing the above-mentioned proteins or mutants thereof, including but not limited to a variant in which other amino acid sequences are linked to Cas. Fusing other functional proteins on the basis of the above Cas proteins or the variants thereof can increase the specificity and effectiveness of the function of the Cas proteins, and can also produce effects other than cleavage of the genome. For example, the fusion of FokI to Cas or dCas can increase the specificity of the Cas9 protein for genome cleavage. Since FokI has cleavage activity only upon dimerization, a pair of recognition regions are required to complete the cleavage, thereby reducing the off-target rate. As another example, FokI is linked to a Cas protein from which some functional domains have been truncated but which retains the DNA binding ability thereof (Ma et al., ACS Synth. Biol., 2018, 7 (4), pp 978-985). As another example, the fusion of base modification enzymes (such as deaminase, cytosine deaminase and adenine deaminase) on Cas-nickase or dCas can efficiently perform targeted base modification on the target region of the genome (Komor et al., Sci. Adv. 2017, 3: eaao4774). As another example, the fusion of some protein domains that can regulate gene expression to dCas9 can effectively regulate the expression of target genes, for example, dCas9 fused with transcription activators such as VP64 and VPR can bind to nearby the target gene under the guidance of gRNA to activate expression; conversely, dCas9 fused with transcription inhibitors (such as SRDX) has a down-regulation effect on target genes. dSpCas9-Tet1 and dSpCas9-Dnmt3a can be used to modify epigenetic status, and regulate the methylation status of endogenous gene promoters to regulate protein expression.

<u>Guide RNA (gRNA)</u>

An RNA component included in the CRISPR is referred to as a guide RNA (gRNA). Guide RNA generally comprises a guide sequence and a backbone sequence, wherein the guide sequence and the backbone sequence may be in the same molecule or in different molecules. The role of the guide RNA is to direct the Cas protein to cleave a DNA site complementary to the guide sequence, i.e., the target sequence. In general, a guide sequence is any polynucleotide sequence having sufficient complementarity with a target sequence to hybridize with this target sequence and direct the specific binding of a CRISPR complex to the target sequence. The degree of complementarity between a guide sequence and its corresponding target sequence is about or more than about 50%, or more. In general, a guide sequence is about or more than about 12 nucleotides in length. The backbone sequence is necessary for guide RNA. The sequences other than the guide sequence generally comprise a tracr sequence and a tracr mate sequence, which generally do not vary with changes in the target sequence.

The guide RNA of the present invention includes single-stranded guide RNA (sgRNA) and double-stranded guide RNA composed of crRNA and tracrRNA. In certain embodiments, the guide RNA has a double-stranded structure composed of crRNA (CRISPR RNA) and tracrRNA (trans-activated crRNA). crRNA generally comprises a guide sequence and a tracr mate sequence, and tracrRNA generally comprises a tracr sequence. In some embodiments, the guide RNA is a single-stranded molecule that generally comprises a guide sequence, a tracr mate sequence, and a tracr sequence, and the single-stranded molecule is also referred to as chimeric single-stranded guide RNA (sgRNA). When the tracr sequence and tracr mate sequence are contained within a single transcript, hybridization between the two produces a transcript having a secondary structure (such as a hairpin). Preferred loop forming sequences for use in hairpin structures are four nucleotides in length, and most preferably have the sequence GAAA. However, longer or shorter loop sequences may be used, as may alternative sequences. The sequences preferably include a triplet (for example, AAA), and an additional nucleotide (for example, C or G). Examples of loop forming sequences include CAAA and AAAG. In one embodiment, the transcript or transcribed polynucleotide sequence has at least two or more hairpins. In preferred embodiments, the transcript has two, three, four or five hairpins. In a further embodiment, the transcript has at most five hairpins. In some embodiments, the transcript may contain one or more nucleic acid structures that bind to biomolecules other than the Cas protein. For example, certain RNA motifs can recruit specific protein domains, such as inserting MS2 RNA sequences into sgRNA sequences, and the RNA sequences can be used to recruit MCP proteins (MS2 bacteriophage coat protein) and proteins that fused or form complexes with MCP proteins (Zalatan et al., Cell, 2015, 160: 339-350). In some embodiments, the single transcript further includes a transcription termination sequence; preferably, this is a Poly-U sequence, for example six U nucleotides.

In some embodiments, one or more vectors driving the expression of one or more elements of a CRISPR system are introduced into a host cell, and the guide RNA enables expression of these elements of the CRISPR system to direct the formation of a CRISPR complex at one or more target sites.

Delivery method

Firstly, an exogenous genetic material is introduced into an immune cell, assembling a delivery system for the genetic material. Any method known in the art can be considered for delivering genetic material into immune cells. Non-limiting examples include viral transduction, transfection by means of electroporation, liposome delivery, polymeric carriers, chemical carriers, lipid complexes, polymeric complexes, dendrimers, nanoparticles, emulsions, a natural endocytosis or phagocytosis pathway, a cell-penetrating peptide, microinjection, microneedle delivery, particle bombardment, etc.

A preferred embodiment is transfection means of by electroporation, and non-limiting examples of instruments which can be used in electroporation include: the Neon transfection system (Thermo Fisher Scientific), a Gemini instrument and an AgilePulse/CytoPulse instrument (BTX-Harvard apparatus), the 4D-Nucleofector system, Amaxa Nucleofector II, the Nucleofector 2b device (Lonza), CTX-1500A (Celetrix), MaxCyte GT or VLX (MaxCyte), and Gene Pulser Xcell (Biorad). Based on guidance from manufacturers, the duration and intensity of pulses, interval between pulses, number of pulses, and optimal conditions for high transfection efficiency with low mortality can be modified.

In another preferred embodiment, biological macromolecules are introduced into cells via chemical reagents. The chemical reagents may include liposomes, polymeric carriers, lipid complexes, polymeric complexes, dendrimers, nanoparticles, emulsions, polyethylenimine (PEI), calcium phosphate, etc.

Secondly, immune cells can spontaneously produce a delivery system or be affected by the environment or target cells to produce a delivery system. Immune cells have been proven to be useful for spontaneously packaging virions or producing exosomes, which virions or exosomes act as a delivery system. At the same time, since immune cells are a type of cells that can recognize antigens and produce immune responses, the controllability of the delivery system produced by the immune cells can be increased according to the immune response mechanism of the immune cells. Receptors of immune cells can form diverse protein receptors through the free combination of coding genes, so that a variety of antigens can be recognized. These antigens can be membrane surface molecules of pathogens or allogeneic cells, can also be specific marker proteins on the surface of tumor cells, and can also be antigen polypeptides processed and presented by other immune cells for pathogen tag molecules. The specific recognition of antigens can activate immune cells, thereby achieving the regulation of gene expression and the synthesis and release of effector molecules through an intracellular signal pathway network, thus producing an immune killing effect on pathogens, allogeneic cells and pathological cells carrying the antigens. Therefore, immune cells can be recruited to a lesion area, and can also be modified to specifically recognize markers on target cells and thus be activated, so that the immune cells can be controlled to produce a delivery system in a specific area and a specific environment, and can directionally affect the target cells more efficiently.

Finally, the delivery system is brought into contact with the target cells, and the contact can be carried out directly *in vivo,* or *in vitro* or in *ex-vivo* cells. The delivery system is used to transfect the target cells, or fuse with the target cells. The target cells receive the genetic materials delivered by the delivery system, and these genetic materials can function by means of integrating or not integrating into the target cell genome. For example, the genetic material delivered by some integrated viruses will first be integrated into the genome of the target cell, and the exogenous gene carried by the genetic material will be transcribed and translated along with the genetic material of the target cell itself, such that same can be stably expressed in the target cell. The genetic material may be some tracer proteins that can track the state of the target cell; and may also be some functional molecules, such as siRNA, which can continuously inhibit the expression of specific genes in the target cell; or some regulatory protein molecules which can change or regulate the function of the target cell; and even some gene editing tools, which achieve the reconstruction or regulation of target cell function by editing the genome of the target cell. Genetic material delivered in the form of non-integrated viruses or exosomes will also have the above functional applications. The difference is that in addition to the long-lasting effect on target cell gene editing, the other effects on the function of target cells will gradually attenuate after a certain period of time.

## Examples

The examples are merely illustrative and are not intended to limit the present invention in any way.

The meanings of the abbreviations are as follows: "H" refers to hours, "min" refers to minutes, "s" refers to seconds, "ms" refers to milliseconds, "d" refers to days, "μL" refers to microliters, "mL" refers to milliliters, "L" refers to liters, "bp" refers to base pairs, "mM" refers to millimoles, and "μM" refers to micromoles.

**Example 1. Lentiviral particles packaged and released by immune T cells complete the intercellular delivery of protein tags**

1. Experimental design and results:

1.1. Experimental design

In this example, donor and receptor cells were selected, the donor cells were processed to make same able to produce a virus that delivers the green fluorescent protein (GFP) gene; by means of detecting whether the receptor cells express green fluorescent protein, virus infection was confirmed and material delivery in the form of a virus between cells was verified.

1.2. Experimental method

Step (1) Construction of a receptor 293T-dsRed cell line:
a) construction of an sgRNA and Cas9 co-expression vector: DNA sequences (SEQ ID No: 1 and SEQ ID NO: 2) were synthesized, two DNA strands were phosphorylated (NEB: M0201S) and annealed to form a double strand, a px330 vector (Addgene, #42230) was digested with BbsI (NEB: R3539S), and the digested product was recovered with a gel recovery kit (Qiagen, 28706). The phosphorylated DNA double-stranded chains were ligated (NEB: M0202L) to the digested px330. The ligated product was transformed into competence DH5$\alpha$ (purchased from GeneWiz), and a single clone was picked and verified by means of Sanger sequencing.
b) A donor DNA gene expressing dsRed was constructed into a plasmid: a PCR reaction was set up as shown in Table 1, wherein the plasmid templates used were a pEASY-T3 cloning plasmid (TransGen: CT301) and pCMV-N-DsRed (Beyotime: D2705), and a 293T cell genome was obtained by lysing a 293T cell (Shanghai Institutes for Biological Sciences: GNHu17). The amplified and purified fragments were constructed together using a Gibson assembly method (NEB, E2611L) to form a circular plasmid, which was transformed into competent DH5$\alpha$, and a single clone was picked and verified by means of Sanger sequencing. The obtained clone was a plasmid containing dsRed DNA. The schematic diagram of the donor in figure 2 shows the final ligation of the donor elements, not comprising a donor backbone.

Table 1: Primers and templates for donor DNA construction

| Primer sequence | Template | PCR product |
|---|---|---|
| SEQ ID NO: 3, SEQ ID NO: 4 | pEASY-T3 clone plasmid | Donor backbone |
| SEQ ID NO: 5, SEQ ID NO: 6 | pCMV-N-DsRed | CMV-dsRed |
| SEQ ID NO: 7, SEQ ID NO: 8 | pCMV-N-DsRed | PolyA |
| SEQ ID NO: 9, SEQ ID NO: 10 | 293T cell genome | HAL |
| SEQ ID NO: 11, SEQ ID NO: 12 | 293T cell genome | HAR |

Note: HAL stands for a left homologous arm, HAR stands for a right homologous arm, CMV-dsRed is a target gene and a promoter fragment thereof, PolyA is a polyadenosine tail, and a donor backbone provides the origin of replication required for plasmid replication, and a resistance gene element.

c) An sgRNA and Cas9 co-expression vector and donor DNA were delivered to a 293T cell using liposome lipofectamine 3000 (Thermo: L3000001). One week after transfection, a cell population was digested into single cells with trypsin (Gibco: 12605028) and counted. Cells with red fluorescence (DsRed positive) were sorted into single cells in a 96-well plate using a flow sorter (FACSAria II), 300 monoclones were obtained and placed in a 5% $CO_2$ cell incubator at 37°C and cultured for two weeks. The grown monoclonal cells were subjected to expanded culture, and genomic DNA was extracted from the monoclone with red fluorescence detected by a fluorescence microscope. A red-light-emitting monoclone was subjected to PCR, and the PCR product was subjected to Sanger sequencing. For sequencing primers, see primer lists (SEQ ID NO: 13-18). The sequencing results verified that the constructed cell line had an exogenous gene expression cassette inserted at the target site (in this example, the site AAVS1 was selected) (Figure 2 is a schematic diagram of site-specific integration into a genome), that is, a cell line with stable transcription of 293T-dsRed was prepared.

Step (2) Preparation of donor and receptor cells before electrotransfection

The receptor cell 293T-dsRed prepared in step (1) was cultured for 2-3 generations, and digested with trypsin, and the cells were counted. The cells were diluted to a concentration of 1 $\times$ $10^5$/mL with a DMEM medium (10% serum, 1% P/S), and added to a 24-well plate (0.5 mL/well), and placed in a cell incubator and cultured for 5-6 hours, and then, the medium was changed to a 1640 medium (10% serum, 1% P/S).

Donor Jurkat T cells (Cell Bank of Chinese Academy of Sciences (Shanghai) (TCHU123)) were resuscitated in a 1640 medium (10% serum, 1% P/S) and cultured for 2-3 generations. The cells were counted and centrifuged, and then resuspended to $5.6 \times 10^7$/mL with OPTI MEM (Life technologies: 31985070).

Step (3) Electrotransfection and infection of receptor cells by means of viruses after electrotransfection

Electrotransfection and virus assembly: 9 μL of Jurkat T cell suspension in 2.1 was taken, and the amount of plasmid was as follows: 225 ng of pLenti-GFP (Figure 1), 180 ng of psPAX2 (Addgene: 12260), and 135 ng of pM2.G (Addgene: #12259). After mixing, the volume was 12 μL, and the remaining amount was made up with OPTI MEM. Electrotransfection was repeated twice with NEON electrotransfection (ThermoFisher: MPK5000, MPK1025) parameters (1500V, 3 pulses, 10 milliseconds), and the obtained product was added to 200 μL of a 1640 medium (Gibco: C11875500BT) (10% serum (Gibco 15140-122), 1% P/S (Cellman: SA212.02)).

Infection of receptor cells by means of viruses: after electrotransfection was completed, 70 μL of electrotransfected Jurkat T cells was taken each time and added to the 293T-dsRed receptor cell culture system in step (2) and a blank medium so that the virus infected the receptor cells.

See Table 2 for the design of the experimental groups (G1-1 and G1-2) and various control groups.

Table 2

| Groups | Donor cell | Electrotransfection plasmid | Receptor cell |
|---|---|---|---|
| G1-1 | Jurkat T | pLenti-GFP, psPAX2, pM2.G | 293T-dsRed |
| G1-2 | Jurkat T | pLenti-GFP, psPAX2, pM2.G | 293T-dsRed |
| G1-3 | Jurkat T | pLenti-GFP, psPAX2, pM2.G | None |
| G2-1 | Jurkat T | pLenti-GFP, psPAX2 | 293T-dsRed |
| G2-2 | Jurkat T | pLenti-GFP, psPAX2 | 293T-dsRed |
| G2-3 | Jurkat T | pLenti-GFP, psPAX2 | None |
| G1-0-N | None | None | 293T |
| G1-0 | None | None | 293T-dsRed |

2. Detection and analysis

After the cells were co-cultured in a cell incubator at 37 degrees Celsius under 5% $CO_2$ for 66 hours, the 24-well plate was photographed (inverted fluorescence microscope Eclipse Ti-U (Nikon)). After taking a photograph, a supernatant cell suspension in each well was sucked out. After centrifugation, the pellet was resuspended with 500 μL PBS, wherein adherent cells were rinsed twice with PBS, then digested with trypsin, and resuspended with 500 μL PBS. The cells in the supernatant and especially the cell suspension were respectively analyzed by means of a flow cytometer (flow cytometer FACSCelesta (BD Biosciences)).

If viruses can be successfully packaged and released, cells successfully infected with the viruses express GFP.

3. Experiment results

Donor and receptor cells can be distinguished by means of growth and tag proteins, wherein 293T-dsRed cells grow adherently and show an red irregular shape in fluorescence photography (Figure 3, column 2 of row 1), and in flow cytometry analysis, the expression of dsRed can be detected in up to 95% of 293T-dsRed cells, which is clearly distinguished from natural 293T cell lines (Figure 5, column 2 of row 1 and row 2). A red light signal can hardly be detected in a Jurkat cell line (Figure 5, column 2 of row 3, and column 2 of row 4), and in addition, the cells are a cell line grown in suspension and have a regular circular shape. In the process of co-cultivation, as determined by the red fluorescence signal, the cells in the supernatant are almost all Jurkat cells (> 90%); there is a certain proportion of Jurkat cells (7%-16%) in adherent cells, and most of the adherent cells are 293T-dsRed (Figure 6 and Figure 7).

Jurkat cells can receive electrotransfection plasmids and express exogenous genes. pLenti-GFP is used as a shuttle plasmid in virus packaging, carries a complete expression cassette for an exogenous target gene, and can be expressed independently. The plasmid enters Jurkat cells by means of electrotransfection, resulting in the expression of the target gene, GFP, in the Jurkat cells (Figure 3, column 3 of row 4, and Figure 4, column 3 of row 3), which showed green and round cells in fluorescence photography, and the expression proportion of GFP is about 20%, as quantified through flow cytometry analysis (Figure 6, column 3 of rows 1 and 3, and Figure 7, column 3 of rows 1 and 3).

When the electrotransfection plasmid meets virus packaging conditions, Jurkat cells can produce effective virions to infect receptor cells. Only when virus plasmids are complete (three-plasmid system: pLenti-GFP, psPAX2, pM2.G) can Jurkat cells produce effective virions to infect the receptor cell, 293T-dsRed. After being infected by viruses produced by Jurkat cells, 293T-dsRed expressed exogenous gene GFP, green irregular cells were detected in fluorescence photography (Figure 3, row 2, and column 3 of row 3), and the proportion of infected cells was about 7% (Figure 6, row 2, and column 4 of row 4). In the control groups G2-1 and G2-2, when an electrotransfection plasmid lacks a virus packaging helper plasmid, 293T-dsRed does not express GFP (Figure 7, row 2, and column 4 of row 4). The data of Figure 5 to Figure 7 are summarized in Table 3. In Table 3, S represents the cells in the suspension of the group, and A represents the adherent cells of the group.

Table 3

| | Group number | Proportion of red fluorescent cells in total cells | Proportion of green fluorescent cells in total cells | Proportion of green fluorescent signal in non-red fluorescent cells | Proportion of green fluorescent signal in red fluorescent cells | Note |
|---|---|---|---|---|---|---|
| Single-cell group | G1-0-N | 0.13% | 0.00% | 0,00% | 0,00% | 293T |
| | G1-0 | 95.70% | 0.00% | 0.00% | 0.00% | 293T-dsRed |
| | G1-3 | 0.60% | 19.60% | 19.72% | N/A | Jurkat |
| | G2-3 | 0.30% | 18.60% | 18.66% | N/A | Jurkat |
| Experimental group | G1-1-S | 0.73% | 0.16% | 21.60% | N/A | Repetition |
| | G1-2-S | 8.08% | 1.54% | 19.10% | N/A | |
| | G1-1-A | 77.70% | 16.24% | 18.80% | 7.30% | Repetition |
| | G1-2-A | 80.70% | 15.11% | 17.00% | 7.21% | |
| Control group | G2-1-S | 1.88% | 0.40% | 21.50% | N/A | Repetition |
| | G2-2-S | 8.08% | 1.62% | 20.00% | N/A | |
| | G2-1-A | 77.70% | 14.53% | 18.60% | 0.35% | Repetition |
| | G2-2-A | 80.70% | 15.96% | 19.70% | 0.30% | |

The proportion of receptor cells in each group receiving GFP genetic material delivered by donor cells is shown in Figure 8. Compared with the single-cell group and the control groups, only the experimental group has an obvious genetic material delivery phenomenon.

This example demonstrates that effective virions can be produced by immune T cells, and a tag protein packaged by the virus can be delivered to a target cell by means of the virus, so that the tag protein is expressed in the target cell.

**Example 2. Lentiviral particles packaged and released by immune T cells can complete the intercellular delivery of a gene editing system**

Donor and receptor cells were selected, after the donor cells were processed to make same able to produce viruses for the delivery of a gene editing material (sgRNA), and the intercellular delivery of the gene editing system was verified by detecting the gene editing in a 293T receptor cell containing a Cas9 gene.

1. Experimental method

Step (1) Preparation of receptor cells

Construction of a 293T-Cas9 cell line: A LentiCRISPRv2 plasmid (Addgene, 52961; see Figure 10 for the plasmid map) was delivered to a 293T cell using liposome lipofectamine 3000 (Thermo: L3000001). After 48 hours of transfection, puromycin (Thermo Fisher, A11138-03) was added, and the cells were screened at a final concentration

of 1 μg/mL and subjected to expanded culture. The cell population screened via puromycin was digested with trypsin into single cells and counted. A flow sorter (FACSAria II) was used to sort cells into single cells in a 96-well plate. 300 monoclones were obtained and placed in a 5% $CO_2$ cell incubator at 37°C, and cultured for 10 days under a puromycin-free condition. Surviving monoclonal cells were observed under a microscope. The grown monoclonal cells were subjected to expanded culture, and further screened with 1 μg/mL of puromycin. Monoclonal cells that were screened via puromycin and subjected to expanded culture were digested with trypsin and plated, and sgRNA (SEQ ID NO: 19) was delivered to the cells by means of a liposome lipofectamine Messenger MAX (ThermoFisher, LMRNA003). The monoclone transfected with sgRNA was subjected to PCR (PCR primers: SEQ ID NO: 20 and SEQ ID NO: 21), and whether gene editing had occurred was analyzed by means of Tide sequencing (https://tide.nki.nl/). Monoclonal cells with relatively high editing efficiency were further subjected to expanded culture, that is, as a stable expression cell line of 293T-Cas9.

Step (2) Preparation of donor and receptor cells before electrotransfection

293T-Cas9 cells were resuscitated and cultured for 2-3 generations, and digested with trypsin, and the cells were counted. The cells were diluted to a concentration of 1 $\times$ $10^5$/mL with a DMEM medium (10% serum, 1% P/S), added to a 24-well plate (0.5 mL/well), and placed in a cell incubator and cultured for 5-6 hours, and then, the medium was changed to a 1640 medium (10% serum, 1% P/S).

Jurkat T cells were resuscitated in a 1640 medium (10% serum, 1% P/S) and cultured for 2-3 generations. The cells were counted and centrifuged, and then resuspended to 5.6 $\times$ $10^7$/mL with OPTI MEM (Life technologies: 31985070).

Step (3) Electrotransfection and infection of receptor cells by means of viruses after electrotransfection

a U6 promoter-sgRNA-U6 terminator sequence of pLenti-sgRNA-GFP was added to the pLenti-GFP plasmid in Example 1; see Figure 9 for map of the sequence.

9 μL of the Jurkat T cell suspension in step (2) was taken, and the amount of plasmid was as follows: pLenti-sgRNA-GFP (225 ng), psPAX2 (180 ng) and pM2.G (135 ng). The volume after mixing was 12 μL, and the remaining amount was made up with OPTI MEM. Electrotransfection was repeated twice with NEON electrotransfection (Neon Transfection System, ThermoFisher MPK5000, MPK1025) parameters (1500 V, 3 pulses, 10 ms) and the obtained product was added to 200 μL 1640 medium (10% serum, 1% P/S). After electrotransfection was completed, 70 μL of electrotransfected Jurkat T cells was taken each time and added to a 293T-Cas9 cell or a blank medium.

See Table 4 for the cells and plasmid usage of specific groups.

Table 4

| Groups | Donor cell | Electrotransfection plasmid | Receptor cell |
|---|---|---|---|
| G3-1 | Jurkat | pLenti-sgRNA-GFP, psPAX2, pM2.G | 293T-Cas9 |
| G3-2 | Jurkat | pLenti-sgRNA-GFP, psPAX2, pM2.G | 293T-Cas9 |
| G3-3 | Jurkat | pLenti-sgRNA-GFP, psPAX2 | None |
| G3-0 | None | None | 293T-Cas9 |

Step (4) Detection and analysis

After the cells were co-cultured for 66 hours, the 24-well plate was photographed (inverted fluorescence microscope Eclipse Ti-U (Nikon)). After taking a photograph, a supernatant cell suspension in each well was sucked out. After centrifugation, the pellet was resuspended with 500 μL PBS, wherein adherent cells were rinsed twice with PBS, then digested with trypsin, and resuspended with 500 μL PBS. A precipitate was collected by centrifuging the cells in the supernatant and especially the cell suspension, 80 μL of a QE buffer was respectively added, and the cells were lysed at 80°C for 10 minutes. The cell lysate was used as a template, and primers (SEQ ID NO: 20 and SEQ ID NO: 21) for target genes were used for fragment amplification. The above PCR products were purified and recovered, and 400 ng of each sample was taken for surveyor detection (IDT: 706020).

2. Experiment results

Donor and receptor cells can be distinguished according to the respective genotype and morphology. 293T-Cas9 cells are monoclonal adherent growth cell lines that can stably express Cas9. Direct sgRNA transfection is performed on the cell line, and the target gene sequence is analyzed, which verifies that there is obvious target gene editing (Figure 11). Morphologically, receptor cell line 293T-Cas9 has an irregular shape, and donor Jurkat T cells are small in shape and have a regular round shape, and most grow in suspension.

In this example, Lenti-sgRNA-GFP is used as a shuttle plasmid in virus packaging, and contains a complete exogenous gene GFP expression cassette and a specific sgRNA expression cassette. The plasmid enters Jurkat cells by means of electrotransfection, resulting in the expression of the target gene, GFP, in the Jurkat cells (Figure 12, row 3), but since there is no Cas9 expression system in the Jurkat cells, gene editing does not occur (Figure 13). However, when an electrotransfection plasmid meets virus packaging conditions, Jurkat cells can produce effective virions and infect receptor cells, and the expression of GFP can be detected in the receptor cells (Figure 12, column 2 of row 1 and row 2), which are mostly green irregular cells; at the same time, the receptor cells also receive an sgRNA expression cassette simultaneously delivered via viruses, and the sgRNA expression cassette binds to a Cas9 protein that is stably expressed by the receptor cells, producing cleaving and gene editing effects on the target genes in the genome of the receptor cells. Obvious editing effects can be seen with a Surveyor mutation detection kit and agarose gel electrophoresis (Figure 13). The gene editing efficiency of the sample in Figure 13, also known as Indel%, is the proportion of insertions and deletions (Indel) produced by gene editing, and is calculated by measuring the gray-scale value of each band in each electrophoresis channel and putting the gray-scale value into a formula.

For one electrophoresis channel, the calculation formula is as follows:

$$\text{Indel\%} = 100 \times \left(1 - \sqrt{1 - \frac{\textit{Sum of gray-scale value of bands produced by SURVEYOR enzyme cleavage}}{\textit{Sum of gray-scale value of all bands}}}\right)$$

The editing effect can reach an efficiency of about 9% under existing conditions through quantification. This example demonstrates that effective virions can be produced by immune T cells, and a gene editing system can be delivered to a target cell by means of a virus, so that a target gene is edited in the target cell.

Example 3. Lentiviral particles packaged and released by immune T cells complete the delivery of a surface antigen

1. Experimental design and method

1.1 Experimental design

In this example, donor and receptor cells were selected, and the donor cells were processed to make same able to produce viruses that deliver a cell surface antigen (in this example, CD19 is taken as an example). Intercellular material delivery in which viruses are taken as media was confirmed by means of detecting whether the receptor cells express the surface antigen.

1.2 Experimental method

Step (1) Construction of a receptor 293T-dsRed cell line: the same as step (1) of Example 1

Step (2) Construction of a lentiviral expression plasmid:

A Plasmid, pELPs (sequence refers to GenBank ID MP123113.1), was obtained using a complete synthesis method. The synthesized CD19 sequence is as shown in GenBank ID BC006338.2 (45-1715). The CD19 was ligated downstream of the EF-1$\alpha$ promoter in pELPs to obtain the plasmid pELPs-CD19 as shown in Figure 14.

Step (3) Preparation of donor and receptor cells before electrotransfection:

the same as step (2) of Example 1

Step (4) Electrotransfection and infection of receptor cells by means of viruses after electrotransfection:

the same as step (3) of Example 1. The electrotransfection plasmid was shown in Table 5.

Table 5

| Groups | Donor cell | Electrotransfection plasmid | Receptor cell |
|---|---|---|---|
| **G 1-0-N** | None | None | 293T |
| **G 1-0** | None | None | 293T-dsRed |
| **G 1-1** | Jurkat T | pELPs-CD19, pM2.G | 293T-dsRed |
| **G 1-2** | Jurkat T | pELPs-CD19, pM2.G | 293T-dsRed |
| **G 1-3** | Jurkat T | pELPs-CD19, pM2.G | None |
| **G 2-1** | Jurkat T | pELPs-CD19, psPAX2 | 293T-dsRed |

(continued)

| Groups | Donor cell | Electrotransfection plasmid | Receptor cell |
|---|---|---|---|
| **G 2-2** | Jurkat T | pELPs-CD19, psPAX2 | 293T-dsRed |
| **G 2-3** | Jurkat T | pELPs-CD19, psPAX2 | None |
| **G 3-1** | Jurkat T | pELPs-CD19, psPAX2, pM2.G | 293T-dsRed |
| **G 3-2** | Jurkat T | pELPs-CD19, psPAX2, pM2.G | 293T-dsRed |
| **G 3-3** | Jurkat T | pELPs-CD19, psPAX2, pM2.G | None |

2. Detection and analysis

The cells co-cultured in the 24-well plate were placed in a cell incubator at 37°C under 5% $CO_2$ for 66 hours. Then, the cells were gently shaken and a supernatant cell suspension was taken, centrifuged, and resuspended with 500 μL of PBS to form a suspension cell sample (such as G 1-1-S, etc.). Adherent cells were digested with trypsin and then resuspended with 500 μl of PBS to form an adherent cell sample (such as G 1-1-A, etc.). The suspension cell sample and the adherent cell sample were respectively analyzed with a flow cytometer (the flow cytometer model is Invitrogen Attune NxT). 1/10 of the adherent cell sample was taken and further cultured for one week, then digested with trypsin and analyzed with a flow cytometer. Receptor cell 293T had a dsRed fluorescent tag. If viruses were successfully packaged by and released from Jurkat T cells and infected receptor cells, it was also possible to detect the expression of the cell surface antigen CD19 in dsRed-positive cells.

3. Experiment results

The results of flow cytometer analysis are summarized in Table 6. Donor cells and receptor cells can be distinguished by means of a dsRed fluorescent tag in flow cytometry analysis. Comparing G 1-0-N (293T), G 1-0 (293T-dsRed), and G 1-3-S (Jurkat T), it can be seen that both 293T cells and Jurkat T cells have no dsRed fluorescence, and 293T-dsRed has no less than 99% dsRed-positive cells.

A pELPs-CD19 plasmid is expressed after entering Jurkat T cells by means of electrotransfection. When Jurkat T cells have both the expression plasmid pELPs-CD19 and the packaging plasmids psPAX2 and pM2.G, the Jurkat T cells produce effective virions and infect receptor cells. The three plasmids are indispensable for the production of virions. If the lentivirus-mediated delivery of cell surface antigen CD19 os completed, stable CD19 expression can be detected in 293T-dsRed cells infected by virions. Comparing the data in column 3 (dsRed-Jurkat T cells) and column 4 (dsRed+ 293T-dsRed cells), which relate to experimental groups G 3-1 and G 3-2 and control groups G 1-1, G 1-2, G 2-1 and G 2-2, which lack a packaging plasmid, in Table 6, it can be seen that, in a suspension cell sample (G x-x-S), dsRed-Jurkat T cells have substantially consistent CD19 expression efficiency upon electrotransfection. In adherent cell samples (G x-x-A), when a virus packaging plasmid is absent (G 1-1-A, G 1-2-A, G 2-1-A and G 2-2-A), a small amount of CD19 expression can be detected in dsRed+ cells, which may be caused by cytoplasmic communication produced by contact between cells. Only when the three plasmids are transfected at the same time (G 3-1, G 3-2) can CD19+ cells forming a cell population be detected.

The adherent cells are further cultured for one week before being subjected to detection (Figure 15A and Figure 15B, and Table 6, columns 5 and 6), and the dsRed+ cell population accounts for not less than 90% of all cells (Figure 15A and Figure 15B, column 1, and Table 6, column 5), and in dsRed+ cells (column 2 in Figure 15A and Figure 15B), CD19-expressing cells in experimental groups (G 3-1-A and G 3-2-A) form an obvious cell population, in contrast, the proportion of CD19-expressing cells in control groups (G 1-1-A, G 1-2-A, G 2-1-A, G 2-2-A) is close to zero. The experimental results confirm that in experimental groups where Jurkat T cells are electrotransfected with three plasmids, CD19 genes were delivered to receptor cells by lentiviruses, stably inserted into the receptor cell genome and persistently expressed.

Table 6

| Groups | dsRed+ % | CD19+% in dsRed- cells | CD19+% in dsRed+ cells | dsRed+ % after one week | CD19+% in dsRed+ cells after one week |
|---|---|---|---|---|---|
| **G 1-0-N** | 0 | 0.0089 9 | 0 | NA | NA |
| **G 1-0** | 99.8 | 0 | 0 | 99.1 | 0 |
| **G 1-1-S** | 90.0 | 75.5 | 0.65 | NA | NA |

(continued)

| Groups | dsRed+ % | CD19+% in dsRed- cells | CD19+% in dsRed+ cells | dsRed+ % after one week | CD19+% in dsRed+ cells after one week |
|---|---|---|---|---|---|
| **G 1-1-A** | 44.6 | 77.8 | 0.90 | 95.9 | 0.00845 |
| **G 1-2-S** | 5.91 | 74.9 | 0 | NA | NA |
| **G 1-2-A** | 47.6 | 79.1 | 0.88 | 90.6 | 0.063 |
| **G 1-3-S** | 0 | 78.0 | 0 | NA | NA |
| **G 2-1-S** | 3.62 | 71.3 | 0 | NA | NA |
| **G 2-1-A** | 49.7 | 71.3 | 0.77 | 92.9 | 0.066 |
| **G 2-2-S** | 4.9 | 70.8 | 0 | NA | NA |
| **G 2-2-A** | 47.8 | 70.4 | 0.80 | 96.5 | 0.033 |
| **G 2-3-S** | 0 | 68.8 | 0 | NA | NA |
| **G 3-1-S** | 5.71 | 78.8 | 1.56 | NA | NA |
| **G 3-1-A** | 58.0 | 82.0 | 1.95 | 96.4 | 1.46 |
| **G 3-2-S** | 7.84 | 78.5 | 0 | NA | NA |
| **G 3-2-A** | 55.7 | 81.8 | 1.76 | 96.9 | 1.09 |
| **G 3-3-S** | 0 | 81.8 | 0 | NA | NA |

**Example 4.** Lentiviral particles packaged and released by immune T cells complete the delivery of a secreted protein such as a cytokine

1. Experimental design and method

1.1 Experimental design

In this example, donor and receptor cells were selected, and the donor cells were processed to make same able to produce viruses that deliver a secreted cytokine (in this example, CCL19 is taken as an example). Intercellular material delivery in which viruses are taken as media was confirmed by means of detecting whether the receptor cells express the cytokine.

1.2 Experimental method

Step (1) Construction of a receptor 293T-dsRed cell line: the same as step (1) of Example 1
Step (2) Construction of a lentiviral expression plasmid:
The CD19 in the pELPs-CD19 (Figure 14) used in Example 3 was replaced with CCL19. The synthesized CCL19 sequence is GenBank ID CR456868.1.
Step (3) Preparation of donor and receptor cells before electrotransfection: the same as step (2) of Example 1
Step (4) Electrotransfection and infection of receptor cells by means of viruses after electrotransfection: the same as step (3) of Example 1. The electrotransfection plasmid is shown in Table 7.

12 hours before flow cytometer detection, the drug Brefeldin A (eBioscience, 00-4506-51), which inhibits the transportation of secreted proteins, was added to half of the experimental groups and the control groups, such as G 1-1-B.

Table 7

| Groups | Donor cell | Electrotransfection plasmid | Receptor cell | Drug added |
|---|---|---|---|---|
| G 1-0-N | None | None | 293T | None |
| G 1-0 | None | None | 293T-dsRed | None |

(continued)

| Groups | Donor cell | Electrotransfection plasmid | Receptor cell | Drug added |
|---|---|---|---|---|
| G 1-1 | Jurkat T | pELPs-CCL19, pM2.G | 293T-dsRed | None |
| G 1-1-B | Jurkat T | pELPs-CCL19, pM2.G | 293T-dsRed | Brefeldin A |
| G 1-2 | Jurkat T | pELPs-CCL19, pM2.G | 293T-dsRed | None |
| G 1-2-B | Jurkat T | pELPs-CCL19, pM2.G | 293T-dsRed | Brefeldin A |
| G 2-1 | Jurkat T | pELPs-CCL19, psPAX2 | 293T-dsRed | None |
| G 2-1-B | Jurkat T | pELPs-CCL19, psPAX2 | 293T-dsRed | Brefeldin A |
| G 2-2 | Jurkat T | pELPs-CCL19, psPAX2 | 293T-dsRed | None |
| G 2-2-B | Jurkat T | pELPs-CCL19, psPAX2 | 293T-dsRed | Brefeldin A |
| G 3-1 | Jurkat T | pELPs-CCL19, psPAX2, pM2.G | 293T-dsRed | None |
| G 3-1-B | Jurkat T | pELPs-CCL19, psPAX2, pM2.G | 293T-dsRed | Brefeldin A |
| G 3-2 | Jurkat T | pELPs-CCL19, psPAX2, pM2.G | 293T-dsRed | None |
| G 3-2-B | Jurkat T | pELPs-CCL19, psPAX2, pM2.G | 293T-dsRed | Brefeldin A |

2. Detection and analysis

The cells co-cultured in a 24-well plate were placed in a cell incubator at 37°C under 5% $CO_2$ for 54 hours. The drug Brefeldin A, which inhibits the transportation of secreted proteins, was added to a G x-x-B sample. After further culturing for 12 hours, the cells were gently shaken and suspension cells were collected. Then, adherent cells were digested with trypsin, and 1/10 of same were taken for further culture. The remaining adherent cells were mixed with suspension cells and analyzed with a flow cytometer (the flow cytometer model was Invitrogen Attune NxT). After one week, the adherent cells that were further cultured were digested with trypsin and analyzed with a flow cytometer. Receptor cell 293T had a dsRed fluorescent tag. If viruses were successfully packaged by and released from Jurkat T cells and infected receptor cells, it was also possible to detect the expression of the cytokine CCL19 in dsRed-positive cells.

3. Experimental results

The results of flow cytometer analysis are summarized in Table 8. Donor cells and receptor cells can be distinguished by means of a dsRed fluorescent tag in flow cytometry analysis. Comparing data in column 2 (dsRed+%) of G 1-0-N (293T) and G 1-0 (293T-dsRed) in Table 8, it can be seen that 293T cells have no dsRed fluorescence, and 293T-dsRed has no less than 99% dsRed-positive cells.

A pELPs-CCL19 plasmid is expressed after entering Jurkat T cells by means of electrotransfection. When Jurkat T cells have both the expression plasmid pELPs-CCL19 and the packaging plasmids psPAX2 and pM2.G, the Jurkat T cells produce effective virions and infect receptor cells. The three plasmids are indispensable for the production of virions. If the lentivirus-mediated delivery of cytokine CCL19 is completed, CCL19 is stably expressed in 293T-dsRed cells infected by virions. Since CCL19 is secreted outside the cell, CCL19 staining can only be detected in samples to which Brefeldin A, which inhibits transportation, is added. Comparing column 3 (dsRed- Jurkat T cells) and column 4 (dsRed+ 293T-dsRed cells), which relate to experimental groups G 3-1, G 3-1-B, G 3-2 and G 3-2-B and control groups G 1-1, G 1-1-B, G 1-2, G 1-2-B, G 2-1, G 2-1-B, G 2-2 and G 2-2-B, which lack a packaging plasmid, in Table 8, it can be seen that obvious CCL19+ cells can be detected only in samples to which Brefeldin A has been added, and Jurkat T cells have a substantially consistent CCL19 expression efficiency. In 293T-dsRed cells, when a virus packaging plasmid is absent (the data in column 4, which relates to G 1-1, G 1-1-B, G 1-2, G 1-2-B, G 2-1, G 2-1-B, G 2-2 and G 2-2-B), CCL19 expression is almost undetectable in dsRed+ cells. Only when the three plasmids are transfected at the same time and Brefeldin A is added (the data in column 4 of G 3-1-B and G 3-2-B) can CCL19+ cells be detected.

The adherent cells are further cultured for one week before being subjected to detection (Figure 16A, Figure 16B and Figure 16C, and Table 8, columns 5 and 6), and the dsRed-positive cell population accounts for not less than 99% of all cells (Figure 16, column 1, and Table 8, column 5), and in dsRed-positive cells, CCL19+ cells in experimental groups (G 3-1-B and G 3-2-B) form an obvious cell population (Figure 16C, column 2), in contrast, the proportion of CCL19-expressing cells in control groups (G 1-1-B, G 1-2-B, G 2-1-B, G 2-2-B) is close to the background. The experimental results confirm that in experimental groups where Jurkat T cells are transfected with the three plasmids, CCL19 genes were delivered to receptor cells by lentiviruses, stably inserted into the receptor cell genome and persistently expressed.

Table 8

| Groups | dsRed+% | CCL19+% in dsRed-cells | CCL19+% in dsRed+ cells | dsRed+ % after one week | CCL19+% in dsRed+ cells after one week |
|---|---|---|---|---|---|
| **G 1-0-N** | 0.00479 | 0.00383 | 0 | NA | NA |
| **G 1-0** | 99.4 | 0.77 | 0.012 | 99.8 | 0.046 |
| **G 1-1** | 84.0 | 2.84 | 0.24 | 99.8 | 0.053 |
| **G 1-1-B** | 83.3 | 12.9 | 0.27 | 99.6 | 0.092 |
| **G 1-2** | 82.2 | 3.49 | 0.30 | 99.8 | 0.10 |
| **G 1-2-B** | 83.1 | 10.4 | 0.28 | 99.7 | 0.10 |
| **G 2-1** | 88.5 | 6.56 | 0.22 | 99.9 | 0.10 |
| **G 2-1-B** | 89.1 | 21.3 | 0.37 | 99.8 | 0.15 |
| **G 2-2** | 88.1 | 9.69 | 0.37 | 99.9 | 0.13 |
| **G 2-2-B** | 90.2 | 21.2 | 0.26 | 99.7 | 0.18 |
| **G 3-1** | 91.1 | 9.29 | 0.35 | 99.9 | 0.18 |
| **G 3-1-B** | 90.8 | 23.1 | 1.36 | 99.9 | 0.46 |
| **G 3-2** | 90.0 | 9.41 | 0.46 | 99.9 | 0.16 |
| **G 3-2-B** | 91.7 | 21.9 | 1.06 | 99.9 | 0.66 |

**Example 5. Lentiviral particles packaged and released by immune T cells complete the delivery of a CRISPR system**

1. Experimental design and method

1.1 Experimental design

In this example, donor and receptor cells were selected, and the donor cells were processed to make same able to produce viruses that deliver a CRISPR system (in this example, Cas9 and gRNA are taken as examples). Intercellular material delivery in which viruses are taken as media was confirmed by means of detecting whether the corresponding gene was knocked out in the receptor cells. Unlike Example 2, in this example, a Cas9 enzyme and gRNA were delivered at the same time, so there is no need for target cells to express Cas9.

1.2 Experimental method

Step (1) Construction of a plasmid expressing RQR8:

A protein tag RQR8 gene (SEQ ID No: 22) was synthesized, and the PuroR gene in pLenti SpBsmBI sgRNA Puro (Addgene #62207) was replaced with an RQR8 gene to obtain a pLenti-RQR8 plasmid.

Step (2) Construction of a receptor 293T-dsRed-RQR8 cell line:

For the construction of a 293T-dsRed cell line, see Example 1. The pLenti-RQR8 plasmid obtained in step (1) was transfected into 293T-dsRed cells with liposome PEI (Polysciences, 24765-2). After 24 hours, single cells were divided into four 96-well plates using a gradient dilution method. After confirming a single-cell well under a microscope, the cells were subjected to expanded culture at 37°C under 5% $CO_2$. The expression of dsRed and RQR8 was detected via a flow cytometer (Invitrogen Attune NxT), and double-positive clones were selected as receptor cells of the experiment.

Step (3) Construction of a gRNA and Cas9 co-expression vector:

The synthesized DNA sequence is as shown in Table 9. The two DNA strands a and b in each group were phosphorylated with a T4 nucleotide kinase (NEB, M0201S) and then annealed to form a double strand. After the vector LentiCRISPRv2GFP (Addgene #82416) was digested with BsmBI (NEB, R0580L) and recovered, the phosphorylated DNA double-stranded chain was ligated into the vector with a T4 DNA ligase (NEB, M0202L) to obtain a plasmid LentiCRISPR-RQR8 gRNA. The plasmid expressed both Cas9 and gRNA targeting the RQR8 protein.

Table 9

| No. | Sequence | SEQ ID No: |
|---|---|---|
| 1-a | CACCTCCTCCGCCGCCAGAACAC | 22 |
| 1-b | AAACGTGTTCTGGCGGCGGAGGA | 23 |
| 2-a | CACCATCCTAGCCTGTGTAGCGG | 24 |
| 2-b | AAACCCGCTACACAGGCTAGGAT | 25 |
| 3-a | CACCGGATCTGAACTGCCTACAC | 26 |
| 3-b | AAACGTGTAGGCAGTTCAGATCC | 27 |
| 4-a | CACCCCAATCCTAGCCTGTGTAG | 28 |
| 4-b | AAACCTACACAGGCTAGGATTGG | 29 |
| 5-a | CACCGATCTGAACTGCCTACACA | 30 |
| 5-b | AAACTGTGTAGGCAGTTCAGATC | 31 |
| 6-a | CACCCAAGCGGTGGTGGTAGGCT | 32 |
| 6-b | AAACAGCCTACCACCACCGCTTG | 33 |
| 7-a | CACCTTGGTGGACACGTTGCTGA | 34 |
| 7-b | AAACTCAGCAACGTGTCCACCAA | 35 |
| 8-a | CACCCAGGGCCATCCAACACAGC | 36 |
| 8-b | AAACGCTGTGTTGGATGGCCCTG | 37 |

Step (4) Preparation of donor and receptor cells before electrotransfection:
the same as step (2) of Example 1, but the receptor cells were the 293T-dsRed-RQR8 cell line obtained in step (2) of this example.
Step (5) Electrotransfection and infection of receptor cells by means of viruses after electrotransfection
the same as step (3) of Example 1. The electrotransfection plasmid is as shown in Table 10, and the co-cultured receptor cells are 293T-dsRed-RQR8.

Table 10

| Groups | Donor cell | Electrotransfection plasmid | Receptor cell |
|---|---|---|---|
| G 1-0-N | Jurkat | None | None |
| G 1-0 | None | None | 293T-dsRed-RQR8 |
| G 1-1 | Jurkat T | LentiCRISPR-RQR8 gRNA, pM2.G | 293T-dsRed-RQR8 |
| G 1-2 | Jurkat T | LentiCRISPR-RQR8 gRNA, pM2.G | 293T-dsRed-RQR8 |
| G 1-3 | Jurkat T | LentiCRISPR-RQR8 gRNA, pM2.G | None |
| G 2-1 | Jurkat T | LentiCRISPR-RQR8 gRNA, psPAX2 | 293T-dsRed-RQR8 |
| G 2-2 | Jurkat T | LentiCRISPR-RQR8 gRNA, psPAX2 | 293T-dsRed-RQR8 |
| G 2-3 | Jurkat T | LentiCRISPR-RQR8 gRNA, psPAX2 | None |
| G 3-1 | Jurkat T | LentiCRISPR-RQR8 gRNA, psPAX2, pM2.G | 293T-dsRed-RQR8 |
| G 3-2 | Jurkat T | LentiCRISPR-RQR8 gRNA, psPAX2, pM2.G | 293T-dsRed-RQR8 |
| G 3-3 | Jurkat T | LentiCRISPR-RQR8 gRNA, psPAX2, pM2.G | None |

2. Detection and analysis

The cells co-cultured in the 24-well plate were placed in a cell incubator at 37°C under 5% $CO_2$ for 66 hours. Then, the cells were gently shaken and suspension cells were collected. Then, adherent cells were digested with trypsin, and 1/10 of same were taken for further culture. The remaining adherent cells were mixed with suspension cells and analyzed with a flow cytometer (the flow cytometer model was Invitrogen Attune NxT). The adherent cells were further cultured for one week and then digested with trypsin and analyzed with a flow cytometer.

The receptor cell 293T-dsRed-RQR8 expresses both a dsRed tag and an RQR8 tag at the same time, but the donor cell Jurkat T does not express these two tags, so in this experiment, RQR8 gRNA was used to verify the delivery of the CRISPR system. The expression of Cas9 and RQR8 gRNA in Jurkat T cells does not lead to gene knockout, nor does it affect the detection of gene editing efficiency. Only when Jurkat T cells successfully package and release a virus and Cas9 and RQR8 gRNA are delivered to receptor cells is the RQR8 tag expressed in the receptor cell knocked out, which is reflected in the detection results of the flow cytometer, namely, dsRed-positive and RQR8-negative cell populations can be detected.

3. Experimental results

The results of flow cytometer analysis are summarized in Table 11. Donor cells and receptor cells can be distinguished by means of dsRed and RQR8 tags in flow cytometry analysis. Comparing the data in column 2 of G 1-0-N (Jurkat T) and G 1-0 (293T-dsRed-RQR8), it can be seen that dsRed and RQR8 are almost undetectable in Jurkat T cells, whereas in 293T-dsRed-RQR8, there is not less than 96% dsRed+RQR8+ cells. Since the experiment detects the RQR8 knockout efficiency in 293T-dsRed-RQR8, and LentiCRISPR-RQR8 gRNA has a GFP tag, in order to detect the transfection efficiency, the GFP expression efficiency in Jurkat T cells (dsRed-RQR8-) will be counted. The efficiency of the intercellular delivery of the CRISPR system is demonstrated by a GFP+RQR8- cell population in a dsRed+ cell population.

When the expression plasmid LentiCRISPR-RQR8 gRNA and the packaging plasmids psPAX2 and pM2.G are simultaneously transfected into Jurkat T cells, the cells produce effective virions and infect receptor cells. The three plasmids are indispensable for the production of virions. If the lentivirus-mediated delivery of a CRISPR system is completed, the knockout of the RQR8 gene can be detected in 293T-dsRed-RQR8 cells infected with virions. Comparing data in column 3 (percentage of GFP+ cells in dsRed-RQR8- Jurkat T cells) and column 4 (percentage of GFP+RQR8- cells in dsRed+ 293T-dsRed-RQR8 cells), which relate to experimental groups G 3-1 and G 3-2 and control groups G 1-1, G 1-2, G 2-1 and G 2-2, which lack a packaging plasmid, in Table 11, it can be seen that Jurkat T cells have substantially consistent electrotransfection and expression efficiency. In dsRed+ cells, when a virus packaging plasmid is absent (G 1-1, G 1-2, G 2-1, G 2-2), the proportion of GFP+RQR8- cells is less than that of three-plasmid groups (G 3-1, G 3-2). Since the expression plasmid in this experiment is relatively large, the intercellular delivery efficiency is relatively low, and the difference between the experimental group and the control group is relatively small.

Adherent cells were further cultured for one week before being subjected to detection (Figure 17, data in column 5 of Table 11). There are obvious dsRed+RQR8- cell populations in the experimental groups (G 3-1, G 3-2), whereas the cell proportion of dsRed+RQR8- in the control groups (G 1-1, G 1-2, G 2-1, G 2-2) is close to the background, and there is no obvious cell population. The experimental results confirm that in experimental groups where Jurkat T cells are transfected with the three plasmids, the CRISPR system is delivered to receptor cells by lentiviruses, thereby achieving the aim of knocking out a gene in the receptor cells.

Table 11

| Groups | dsRed+ RQR8+% | GFP+% in dsRed-RQR8-cells | GFP+RQR8-% in dsRed+ cells | dsRed+RQR8-% after one week |
|---|---|---|---|---|
| **G 1-0-N** | 0 | 0 | 0 | NA |
| **G 1-0** | 96.6 | 0 | 0 | 0.89 |
| **G 1-1** | 25.1 | 31.2 | 0.12 | 0.86 |
| **G 1-2** | 24.4 | 31.3 | 0.098 | 0.96 |
| **G 1-3** | 0.00915 | 34.3 | 0 | NA |
| **G 2-1** | 33.6 | 33.4 | 0.025 | 0.72 |
| **G 2-2** | 25.1 | 35.0 | 0.082 | 0.59 |

(continued)

| Groups | dsRed+ RQR8+% | GFP+% in dsRed-RQR8-cells | GFP+RQR8-% in dsRed+ cells | dsRed+RQR8-% after one week |
|---|---|---|---|---|
| **G 2-3** | 0.00885 | 43.1 | 0 | NA |
| **G 3-1** | 18.3 | 32.4 | 0.21 | 1.85 |
| **G 3-2** | 16.5 | 33.1 | 0.20 | 2.43 |
| **G 3-3** | 0.00641 | 42.3 | 0 | NA |

**Example 6. Production, by means of 293T cells, of exosomes packaging specific proteins**

1. Experimental design and method

1.1 Experimental design

In this example, exosomes secreted in a cell culture supernatant were collected and purified and analyzed. In order to facilitate the detection of exosomes, cells were transfected with a specific plasmid (pDB30). The target protein expressed by the plasmid is a CD63-Nluc fusion protein, wherein CD63 (NM_001267698.1) is an exosome-specific tag, and Nluc (JQ513379.1) is a luciferase reporter gene. The expression of the reporter gene can be quantified by catalyzing the reaction of a substrate via the fusion protein and detecting the amount of fluorescence produced, which can thus reflect the secretion of exosomes and the packaging of the target protein. At the same time, the exosomal booster plasmid (pDB60) in the literature report concerning cell co-transfection has been experimentally proven to increase the production and secretion of exosomes.

In this example, two types of cells, 293T and Jurkat, were used, and the ability of the two cells to produce exosomes was respectively investigated.

The extraction of exosomes is based on the different physical and chemical properties of exosomes and cells. Separation and purification were carried out via a differential centrifugation method and analysis of yield properties was carried out.

1.2 Experimental method

Step (1) Construction of plasmids, wherein the map of plenti-GFP is described in Figure 1.

Table 12

| Plasmid name | Plasmid structure | Use | Plasmid source |
|---|---|---|---|
| pDB30-CD63 -Nluc (Figure 18) | PhCMV-CD63-nluc-pA | Encoding exosomal marker and fluorescent protein nluc, and assessing exosome yield | Synthesized by GENEWIZ |
| pDB60-Boost er (Figure 19) | PhCMV-STEA P3-T2A-SDC4-P2A-nad fragment-pA | Encoding three genes of exosomal booster: STEAP3-SDC4-NadB, and increasing exosome yield | Miaoling Biotechnology Co., Ltd (P2882) |

Step (2) Cell preparation and plasmid transfection

293T was cultured for 2-3 generations, and digested with trypsin, and the cells were counted. The cells were diluted to a density of $2.5 \times 10^5$/mL with a DMEM medium (10% serum, 1% P/S), and added to a 24-well plate (0.5 mL/well); and Jurkat was cultured for 2-3 generations, and the cells were counted. The cells were diluted to a cell density of $5 \times 10^5$/ml with a 1640 medium (10% serum, 1%P/S) (0.5 mL/well). The 24-well plate was placed at 37°C under 5% $CO_2$ for culturing for 24 h. The plasmids were mixed according to the proportion in Table 13, and were allowed to stand for 5 min, and PEI (Polysciences, 24765-2) was added, and same were uniformly mixed, incubated for 15 min, and added to a culture plate. The mixture was shaken gently, and cultured at 37°C under 5% $CO_2$ for 16 h. The supernatant of 293T cells was sucked out, fresh DMEM (+10% FBS +1% P/S) was added, and the resulting mixture was further cultured for 24 h; and 300 g of the suspension of Jurkat cells was centrifuged for 5 min, the supernatant was sucked out, and the cells were resuspended with a fresh 1640 (+10% FBS +1% P/S) medium, re-added to the 24-well plate, and cultured at 37°C under 5% $CO_2$ for 24 h.

Table 13

| Plasmid name | 1 | 2 | 3 | 4 | 5 |
|---|---|---|---|---|---|
| pDB60-Booster | 375 ng | 0 | 0 | 0 | 550 ng |
| pDB30-CD63-Nluc | 125 ng | 125 ng | 0 | 0 | 250 ng |
| plenti-GFP | 0 | 375 ng | 500 ng | 0 | 0 |
| PEI | 2.0 μg | 2.0 μg | 2.0 μg | 2.0 μg | 2.0 μg |

Step (3): Extraction and analysis of exosomes

The supernatant (293T) or the cell suspension (Jurkat) was collected, and 300g of same was centrifuged for 5 min to remove the cells; 2000g of the supernatant was further centrifuged for 10 min through gradient centrifugation. The supernatant was taken and 10,000 g thereof was centrifuged for 30min; and cells and cell debris were further removed, and the final supernatant was analyzed.

Fluorescence detection: The Nano-Glo® Dual- Luciferase® Reporter Assay system (Promega, N1610) luciferase reporter gene detection system was used to detect the expression level of a fluorescent protein in the supernatant and cell precipitate respectively. 80 μL of a sample to be detected was added to 96-well plates (white-well plates). After NanoDLR™ Stop & Glo Reagent: substrate : Stop& Glo Buffer = 1 : 100 were prepared and uniformly mixed, the mixture was left to stand at room temperature. An equal amount (80 μL) of NanoDLR™ Stop & Glo Reagent was added, 600 rpm, and subjected to a reaction involving shaking for 15 min in the dark at room temperature; a multifunctional microplate reader (Varioskan Lux, Thermo) was used to detect fluorescence, with a wavelength range of 400-600 nm, and a highest absorption peak of 460 nm (see Figure 20).

2. Experimental results and analysis

Experimental results (Figure 20): the vertical coordinate relative light unit (RLU) represents the relative test value of the amount of light produced in the sample. Quantitative analysis detected the expression of luciferase in the exosomes separated and collected from the supernatant of 293T cells. The detection of luciferase in the wavelength range of 400-600 nm (Figure 20A) and at the absorption peak at 460 nm (Figure 20B) shows a consistent trend. However, transfection with a plasmid with a multiple of reporter genes does not promote the expression of the target gene in exosomes. In addition, in the literature, gene expression that promotes exosome production and secretion does not play a role in stimulating exosomes under the present experimental conditions. In addition, the expression of the luciferase gene was not detected in the supernatant of Jurkat cells. This may be related to the low transfection efficiency of Jurkat cells and fewer exosomes being produced for packaging reporter genes.

Example 7. Production of exosomes through cell packaging to complete the intercellular delivery of materials

1. Experimental design and method

1.1 Experimental design

In this example, donor and receptor cells were selected, the donor cells were processed to make same able to produce an exosome that delivers a green fluorescent protein (GFP), it being possible to carry out intercellular material delivery in the form of an exosome was confirmed by means of detecting whether the receptor cells express the green fluorescent protein.

1.2 Experimental method

Step (1): Construction of a plasmid

The detection index was changed from Nluc to EGFP, which is convenient for detection, and the plasmid pDB30-CD63-EGFP was constructed. Since the plasmid pDB60-booster contains GFP, which interferes with the determination of target gene expression, a Booster gene was inserted into a vector pX330 (Addgene 92115) to construct a pX330-Booster plasmid.

Gene PCR amplification was performed: see Table 14 for the list of primers and templates.

Table 14

| Primer name | Primer sequence | Template name | PCR product |
|---|---|---|---|
| PX330-Gibson-R | tactgccaaaaccgcatcacggtacctctagagccatttgtctgc (SEQ ID No: 39) | PX330 plasmid | pX330-L (about 3500 bp) |
| PX330-Gibson-F | cataaacagataaggatccgaattcctagagctcgctgatcagc (SEQ ID No: 40) | | |
| Booster-Gibson-F | caaatggctctagaggtaccgtgatgcggttttggcagtacatc (SEQ ID No: 41) | pDB60-Booster plasmid | Booster(about4200 bp) |
| Booster-Gibson-R | Cagcgagctctaggaattcggatccttatctgtttatgtaatgattgcc (SEQ ID No: 42) | | |
| KpnI-GFP-F | Cggggtaccagagcccaggcccggcagccatggtgagcaagggcgaggag (SEQ ID No: 43) | plenti-GFP plasmid | GFP (about 750 bp) |
| BamHI-GFP-R | cgcggatccttacttgtacagctcgtccatgccg (SEQ ID No: 44) | | |

Construction and ligation of pDB30-CD63-GFP: a pDB30-CD63-Nluc plasmid and a GFP amplification product were subjected to double enzyme digestion with KpnI/BamHI (NEB R3142L, R3136L), and a gel was cut to obtain a fragment of about 7700 bp and a fragment of about 750 bp; the two fragments were ligated with a T4 ligase (NEB M0202L), the ligation product was transformed into bacteria, and the colony was sequenced and identified.

Construction and ligation of pX330-Booster: 1) PCR product pX330-L and a Booster amplification product were ligated by means of a Gibson ligation system (NEB E5510S), the product was transformed into bacteria, and the colony was sequenced and identified.

Step (2): Cell preparation and plasmid transfection

Receptor cells were 293T-dsRed cells (see Example 1 for cell preparation), which were cultured for 2-3 generations, and digested with trypsin. The cells were counted, diluted to a cell concentration of $3 \times 10^5$/mL with a DMEM medium (10% serum, 1% P/S), added to a 6-well plate (2 mL/well), and further cultured for 24 h at 37°C under 5% $CO_2$. At the same time, donor cells (293T) were prepared in the same way, added to a 24-well plate (0.5 mL/well), and cultured at 37°C under 5% $CO_2$ for 24 hours, and then, the donor cells were transfected with plasmids with reference to Table 15. The transfection method was the same as that in Example 6

Table 15

| Plasmid name | 1 | 2 | 3 | 4 | 5 | 6 |
|---|---|---|---|---|---|---|
| pX330-Booster | 375 ng | 0 | 375 ng | 550 ng | 0 | 0 |
| pDB30-CD63-GFP | 150 ng | 150 ng | 0 | 250 ng | 0 | 0 |
| plenti-GFP | 0 | 0 | 150 ng | 0 | 0 | 0 |
| PEI | 2.0 μg | 2.0 μg | 2.0 μg | 2.5 μg | 2.0 μg | 0 |
| Donor 293T | 3.0E5 | 3.0E5 | 3.0E5 | 3.0E5 | 3.0E5 | 0 |
| Receptor 293T-dsRed | 1.2E6 | 1.2E6 | 1.2E6 | 1.2E6 | 1.2E6 | 1.2E6 |

Step (3) Cell co-cultivation and flow analysis

8 h after transfection, the old medium was removed from donor cells, and the cells were digested with trypsin. A fresh medium was added, and the cell suspension was completely transferred to a 6-well plate for culturing receptor cells 293T-dsRed, and further cultured at 37°C under 5% $CO_2$ for 48 h. The cells in the 6-well plate were digested for flow analysis (flow cytometer: Invitrogen Attune NxT). The cells with red fluorescence and the cells with green fluorescence were counted respectively.

2. Experimental results and analysis

Since the receptor cells can stably express the red fluorescent protein, same can be obviously distinguished from the donor cells. When the receptor cells are cultured alone, almost all of the cells (99.55%, group 6 in Table 16) express the red fluorescent protein, but substantially do not express the green fluorescent protein. When the donor cells are introduced into the system, some donor cells that do not express the red fluorescent protein occur when the cell population is analyzed. The results show that the receptor cells account for a consistent proportion of the total cell population, about 75% to 80% (groups 1 to 5 in Table 16, $dsRed^+$/total).

When the donor cells are not transfected with a GFP plasmid, the green fluorescent signal cannot be detected in the donor cells (Table 16, group 5, $dsRed^-GFP^+/dsRed^-$). When the donor cells are transfected with a GFP-expressing plasmid, some of the donor cells express GFP (Table 16, groups 1 to 4, $dsRed^-GFP^+/dsRed^-$), and at the same time, green fluorescent signals are also detected in the receptor cells. Experiments show that, under co-culture conditions, a green fluorescent protein is delivered to the receptor cells, and statistical results show that about 1.2% to 2% of the receptor cells receive the delivered material (groups 1 to 4 in Table 16, $dsRed^+GFP^+/dsRed^+$). The low material delivery efficiency may be related to the low transfection efficiency of the donor cells, but compared with the control groups (about 0.3%, groups 5-6 in Table 16, $dsRed^+GFP^+/dsRed^+$), there is obvious material delivery.

Similar to Example 6, an exosome enhancer does not have a significant enhancement effect (comparison between groups 1 and 2 in Table 16), and increasing the amount of plasmid transfection does not enhance delivery (comparison between groups 1 and 4 in Table 16), probably because there is no further optimization for the enhancer or plasmid dosage under the current experimental conditions. In addition, fusion GFP without an exosomal tag can also be delivered to the receptor cells (comparison between groups 1 and 3 in Table 16). This may be because exosomes do not have strict restrictions on the packaging and secretion of contents, and all the contents in cytoplasm may be packaged and secreted for delivery.

Table 16

| | 1 | 2 | 3 | 4 | 5 | 6 |
|---|---|---|---|---|---|---|
| $dsRed^+$ /total number | 80.57% | 79.44% | 76.55% | 79.65% | 75.71% | 99.55% |
| $dsRed^-GFP^+$ /$dsRed^-$ | 19.61% | 20.56% | 25.47% | 17.88% | 0% | 0% |
| $dsRed^+GFP^+$ /$dsRed^+$ | 1.20% | 1.54% | 2.02% | 1.22% | 0.37% | 0.30% |

## Claims

1. A method for delivering biomacromolecules to target cells, the method comprising the following steps:

   d) introducing biomacromolecules into immune cells to assemble a delivery system containing biomacromolecules;
   e) bringing the delivery system of step a) into contact with target cells; and
   f) the target cells receiving the biomacromolecules delivered by the delivery system.

2. The method as claimed in claim 1, wherein the immune cells are T cells, B cells or NK cells.

3. The method as claimed in claim 2, wherein the immune cells are T cells.

4. The method as claimed in claim 3, wherein the T cells are CAR-T or TCR-T cells.

5. The method as claimed in any one of the preceding claims, wherein with respect to the immune cells, the biomacromolecules are exogenous biomacromolecules.

6. The method as claimed in any one of the preceding claims, wherein the biomacromolecules are selected from one or more of a polypeptide, a protein and a genetic material.

7. The method as claimed in claim 6, wherein the genetic material is DNA and/or RNA.

8. The method as claimed in claim 7, wherein the RNA is selected from one or more of mRNA, siRNA and gRNA.

9. The method as claimed in claim 7, wherein the RNA is gRNA.

10. The method as claimed in claim 9, wherein the gRNA is sgRNA or a duplex composed of crRNA and tracRNA.

**11.** The method as claimed in claim 9 or 10, wherein the gRNA and a coding gene encoding a Cas protein are delivered to the target cells.

**12.** The method as claimed in claim 7, wherein the DNA is selected from one or more of linear DNA, single-stranded DNA and plasmid DNA.

**13.** The method as claimed in claim 6, wherein the biomacromolecules are surface molecules, surface antigens, secreted molecules or cytokines.

**14.** The method as claimed in claim 13, wherein the biomacromolecules are CD19 surface molecules or CCL19 secreted molecules.

**15.** The method as claimed in any one of the preceding claims, wherein the method is performed *in vitro* or on *ex-vivo* cells.

**16.** The method as claimed in any one of claims 1-14, wherein step a) is performed *in vitro,* and steps b) and c) are performed *in vivo.*

**17.** The method as claimed in any one of claims 12-16, wherein the plasmid is a plasmid for assembling a virus.

**18.** The method as claimed in any one of claims 1-17, wherein the delivery system is a viral system.

**19.** The method as claimed in claim 18, wherein the viral system is selected from one or more of an adeno-associated virus, an adenovirus, a retrovirus, a lentivirus, a rabies virus and a herpes virus.

**20.** The method as claimed in claim 19, wherein the viral system is a lentivirus.

**21.** The method as claimed in any one of claims 1-16, wherein the delivery system is an exosome.

**22.** The method as claimed in claim 21, wherein the exosome, which encapsulates the polypeptide, protein and/or genetic material, is released to outside the immune cells and comes into contact with the target cells.

**23.** The method as claimed in any one of claims 12-22, wherein the plasmid further comprises a regulatory element.

**24.** The method as claimed in any one of the preceding claims, wherein the biomacromolecules of step a) are introduced into the immune cells by means of electrotransfection, or introduced into the immune cells by means of chemical reagents.

**25.** The method as claimed in any one of the preceding claims, wherein the target cells are tumor cells, pathogens, stem cells or somatic cells.

**26.** The method as claimed in claim 11, wherein the Cas protein is selected from Cas1, Cas1B, Cas2, Cas3, Cas4, Cas5, Cas6, Cas7, Cas8, Cas9, Cas10, Cas12 and Cas13, and mutants of the above-mentioned proteins, and fusion proteins or protein complexes including these proteins or mutants thereof.

**27.** The method as claimed in claim 26, wherein the Cas9 protein is a Cas9 protein derived from *Streptococcus pyogenes.*

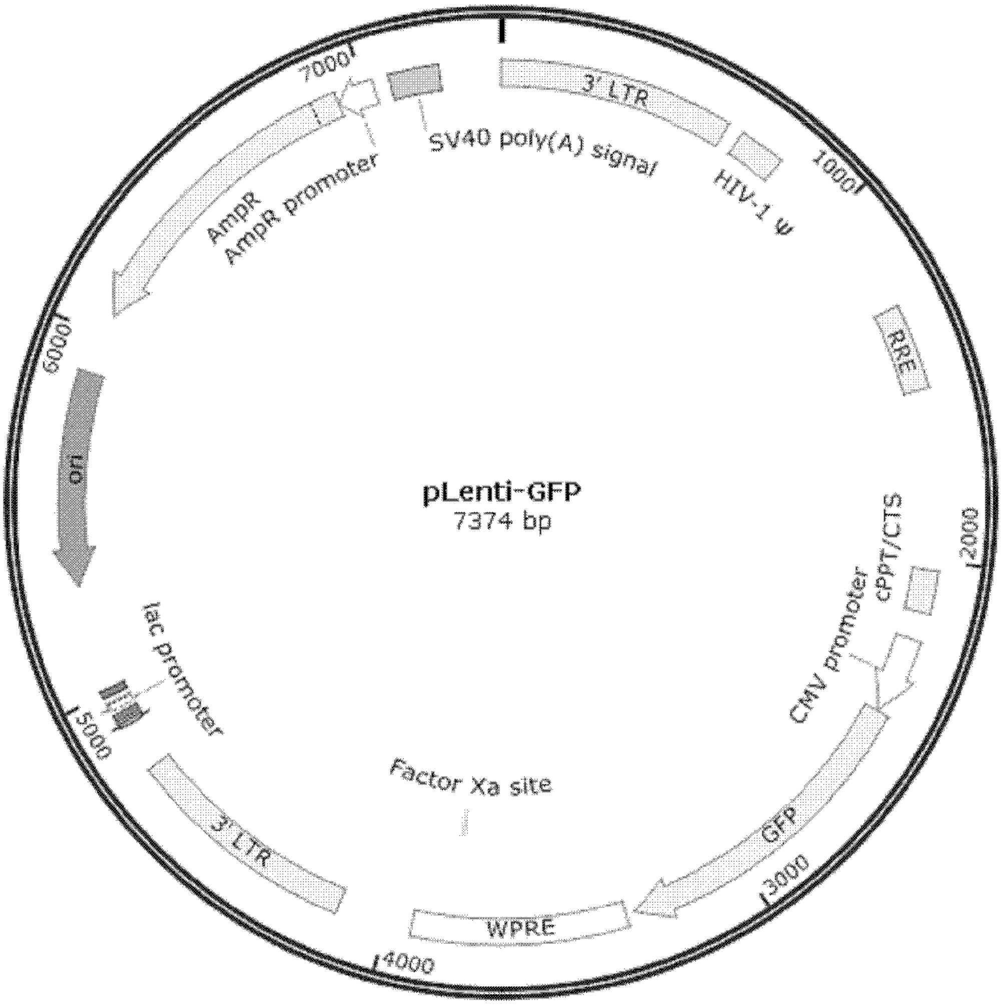
pLenti-GFP
7374 bp
3' LTR
HIV-1 ψ
1000
RRE
cPPT/CTS
2000
CMV promoter
GFP
3000
WPRE
4000
Factor Xa site
3' LTR
5000
lac promoter
ori
6000
AmpR
AmpR promoter
7000
SV40 poly(A) signal

*Fig. 1*

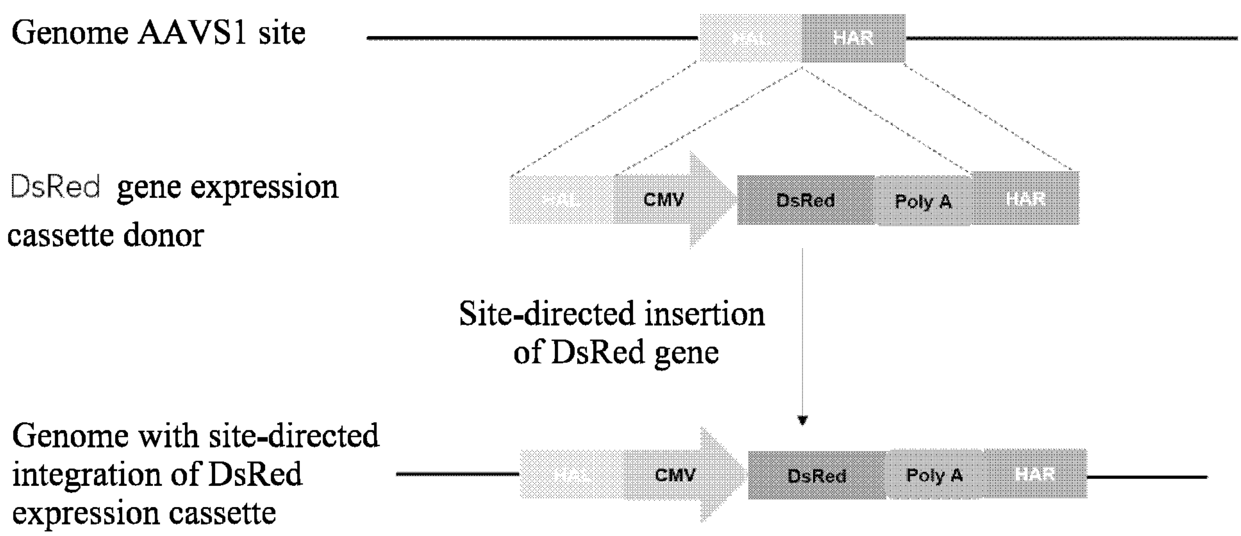
Genome AAVS1 site
HAL
HAR
DsRed gene expression
cassette donor
HAL
CMV
DsRed
Poly A
HAR
Site-directed insertion
of DsRed gene
Genome with site-directed
integration of DsRed
expression cassette
HAL
CMV
DsRed
Poly A
HAR

*Fig. 2*

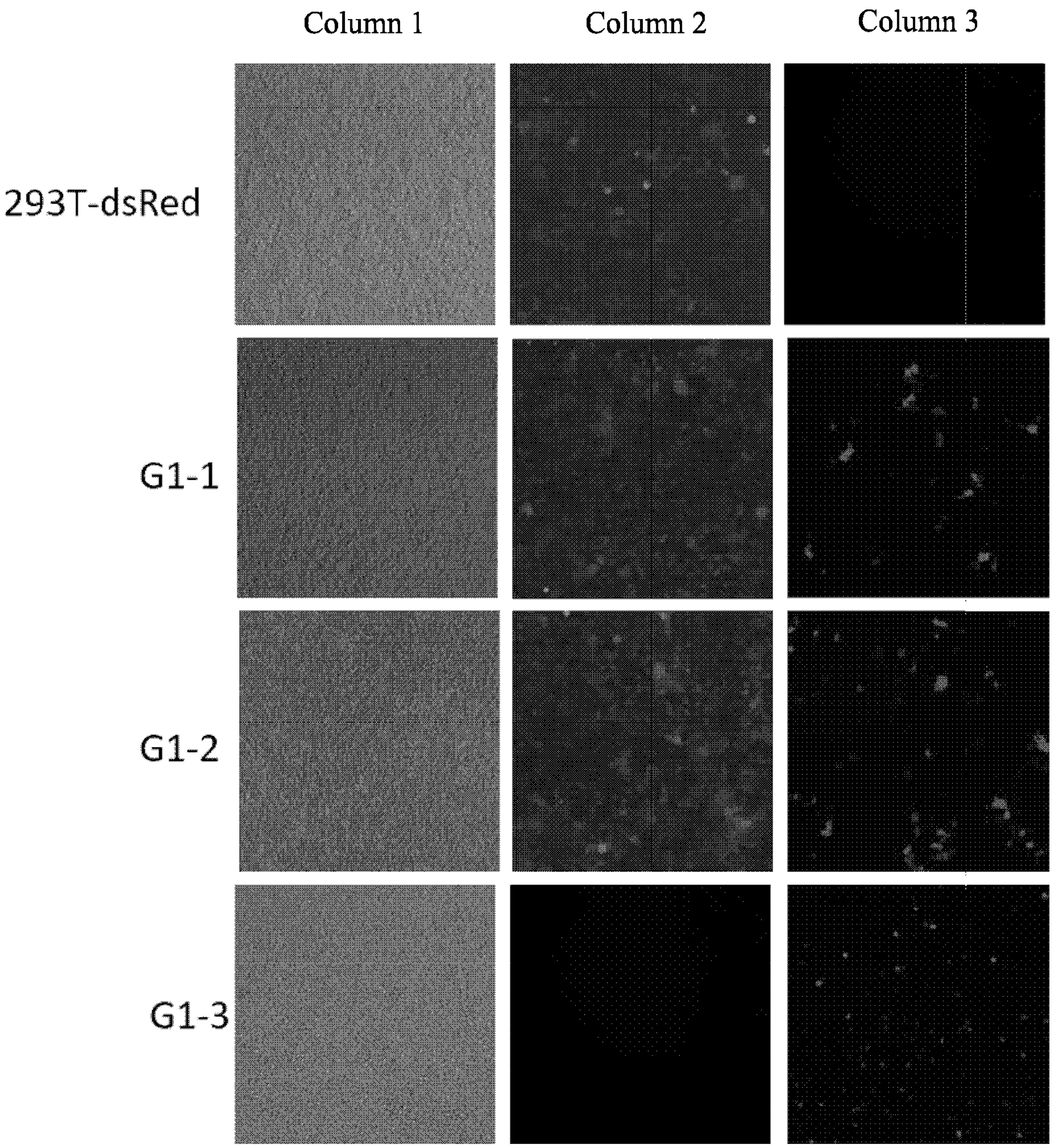


*Fig. 3*

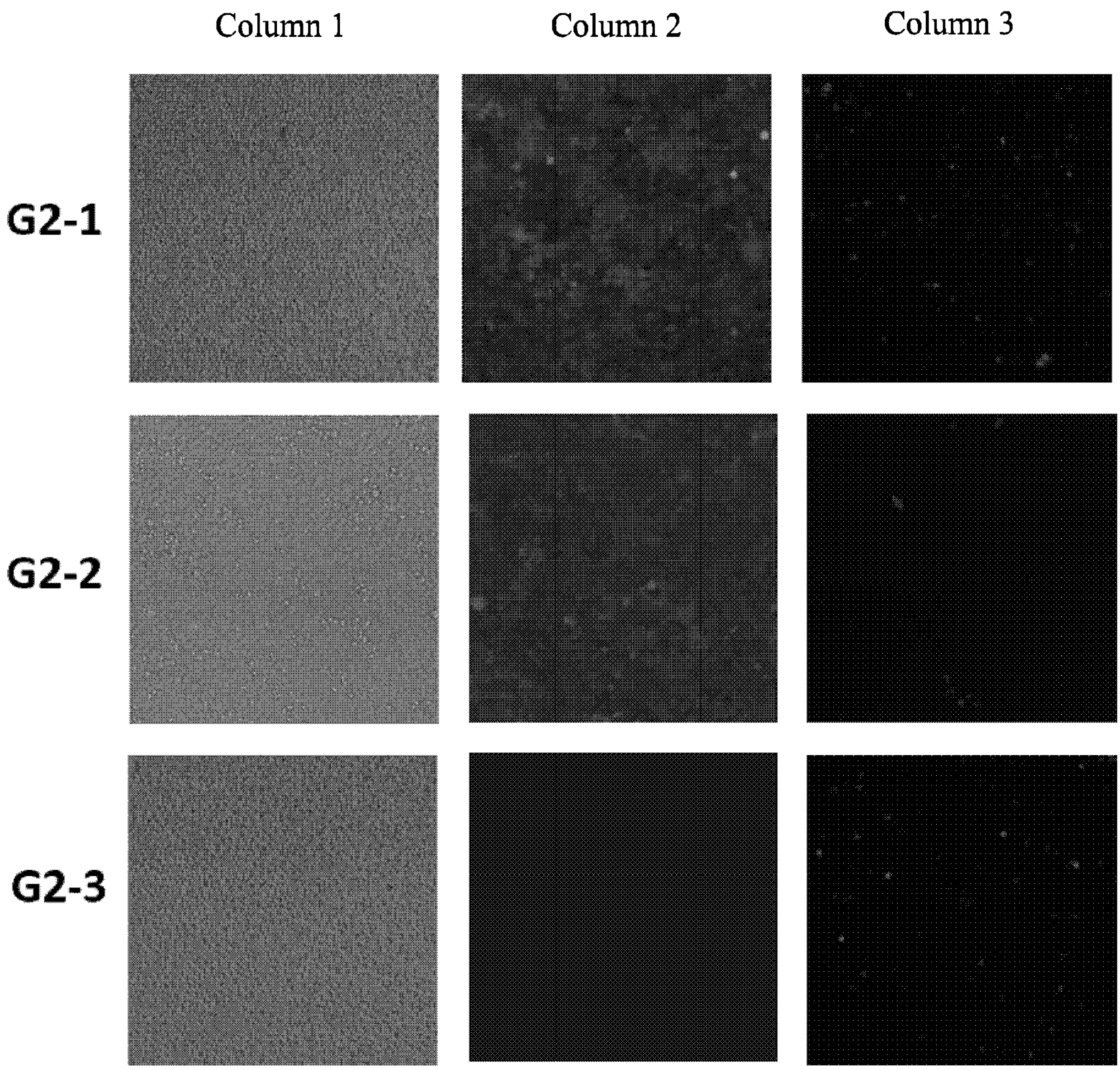


*Fig. 4*

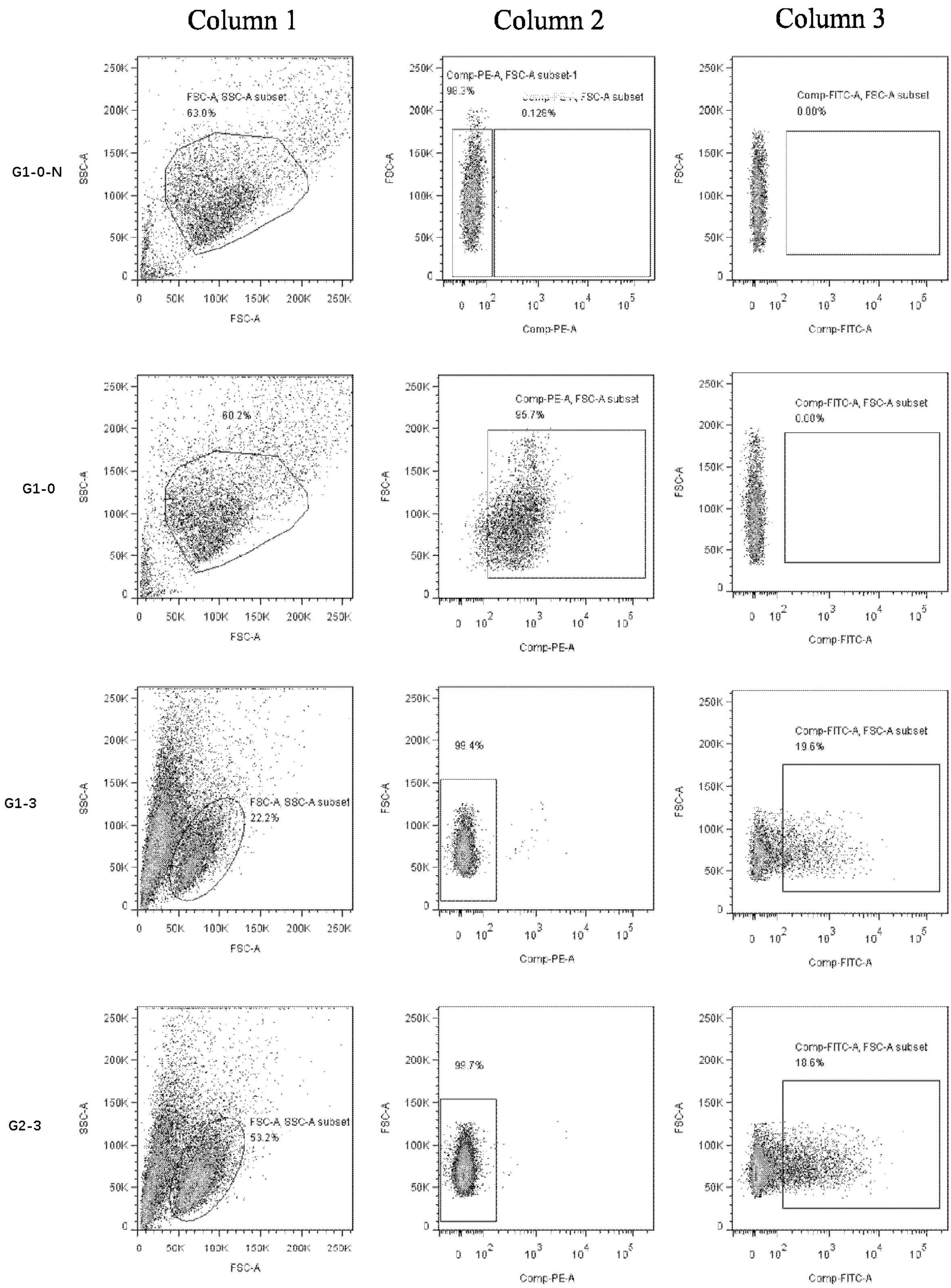
Column 1
Column 2
Column 3
G1-0-N
G1-0
G1-3
G2-3
FSC-A, SSC-A subset
63.0%
Comp-PE-A, FSC-A subset-1
98.3%
0.129%
Comp-FITC-A, FSC-A subset
0.00%
60.2%
Comp-PE-A, FSC-A subset
95.7%
Comp-FITC-A, FSC-A subset
0.00%
FSC-A, SSC-A subset
22.2%
99.4%
Comp-FITC-A, FSC-A subset
19.6%
FSC-A, SSC-A subset
53.2%
99.7%
Comp-FITC-A, FSC-A subset
18.6%
SSC-A
FSC-A
Comp-PE-A
Comp-FITC-A

*Fig. 5*

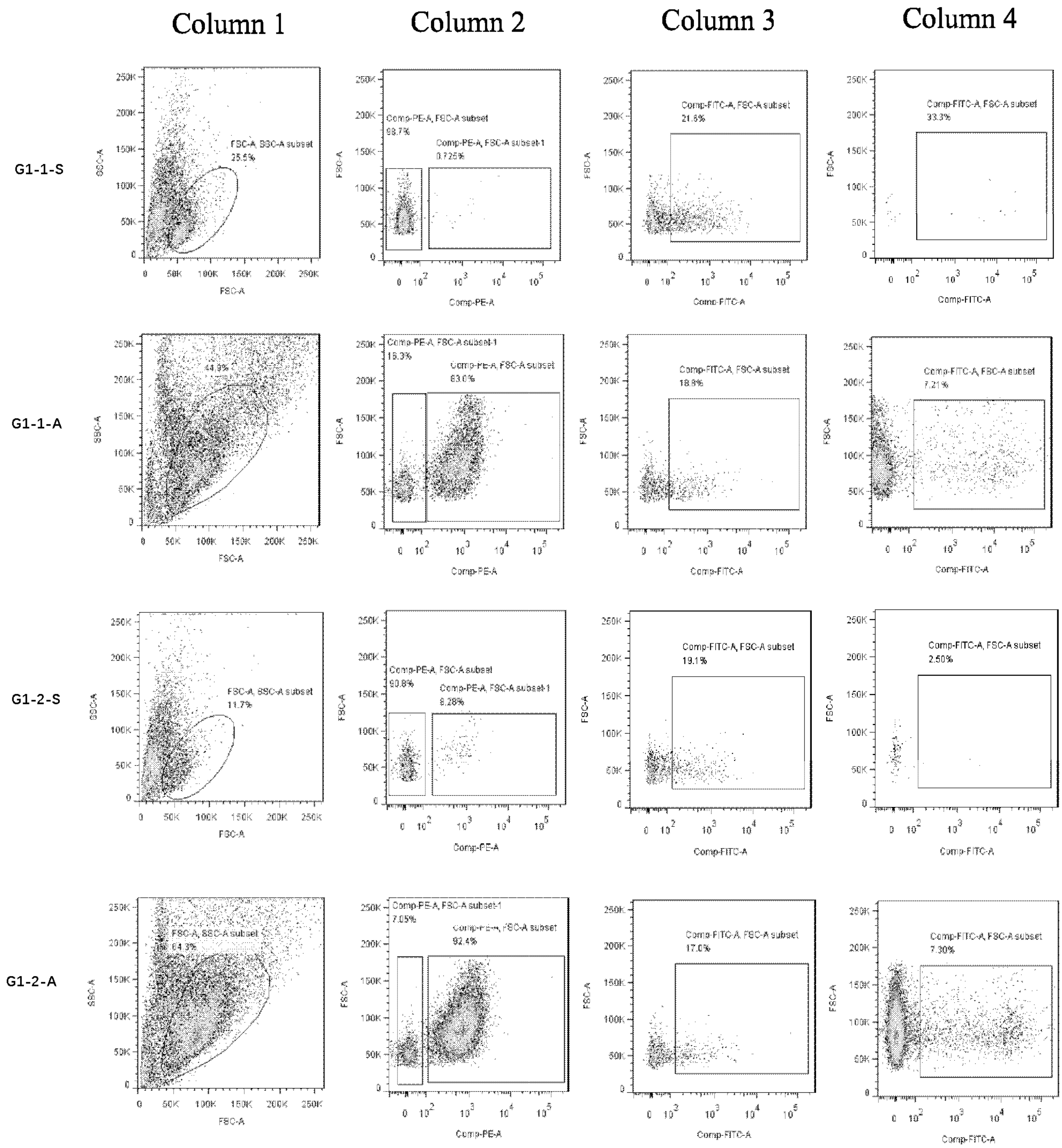
Column 1
Column 2
Column 3
Column 4
G1-1-S
G1-1-A
G1-2-S
G1-2-A
FSC-A, SSC-A subset
25.5%
44.9%
11.7%
64.3%
Comp-PE-A, FSC-A subset
98.7%
Comp-PE-A, FSC-A subset-1
0.725%
16.3%
83.0%
90.8%
8.28%
7.05%
92.4%
Comp-FITC-A, FSC-A subset
21.6%
18.8%
19.1%
17.0%
33.3%
7.21%
2.50%
7.30%
SSC-A
FSC-A
Comp-PE-A
Comp-FITC-A

*Fig. 6*

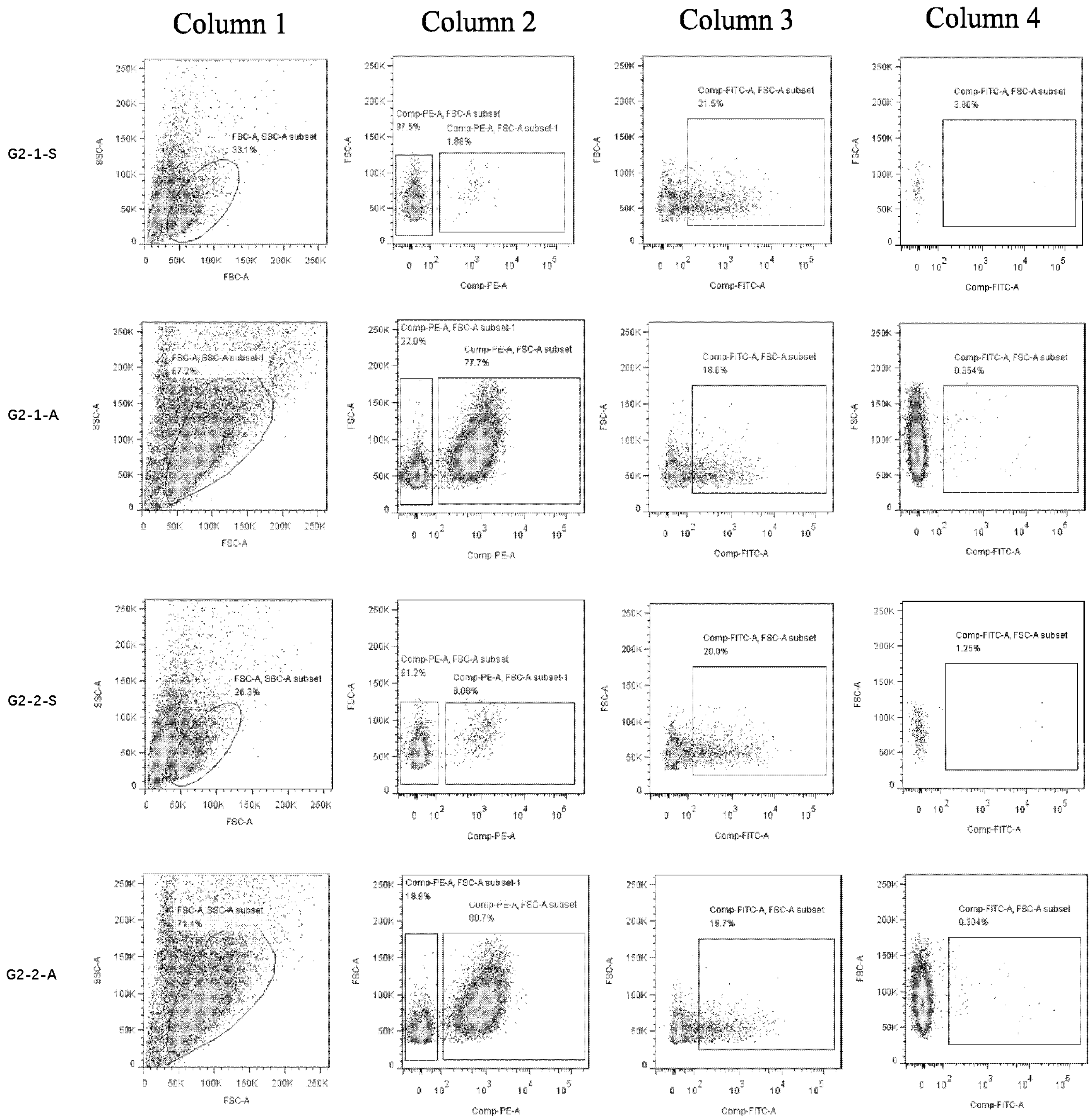
Column 1
Column 2
Column 3
Column 4
G2-1-S
G2-1-A
G2-2-S
G2-2-A
FSC-A, SSC-A subset
33.1%
Comp-PE-A, FSC-A subset
97.5%
Comp-PE-A, FSC-A subset-1
1.86%
Comp-FITC-A, FSC-A subset
21.5%
Comp-FITC-A, FSC-A subset
3.80%
FSC-A, SSC-A subset-1
67.2%
Comp-PE-A, FSC-A subset-1
22.0%
Comp-PE-A, FSC-A subset
77.7%
Comp-FITC-A, FSC-A subset
18.8%
Comp-FITC-A, FSC-A subset
0.354%
FSC-A, SSC-A subset
26.3%
Comp-PE-A, FSC-A subset
91.2%
Comp-PE-A, FSC-A subset-1
8.08%
Comp-FITC-A, FSC-A subset
20.0%
Comp-FITC-A, FSC-A subset
1.25%
FSC-A, SSC-A subset
71.4%
Comp-PE-A, FSC-A subset-1
18.9%
Comp-PE-A, FSC-A subset
80.7%
Comp-FITC-A, FSC-A subset
19.7%
Comp-FITC-A, FSC-A subset
0.304%
SSC-A
FSC-A
Comp-PE-A
Comp-FITC-A

*Fig. 7*

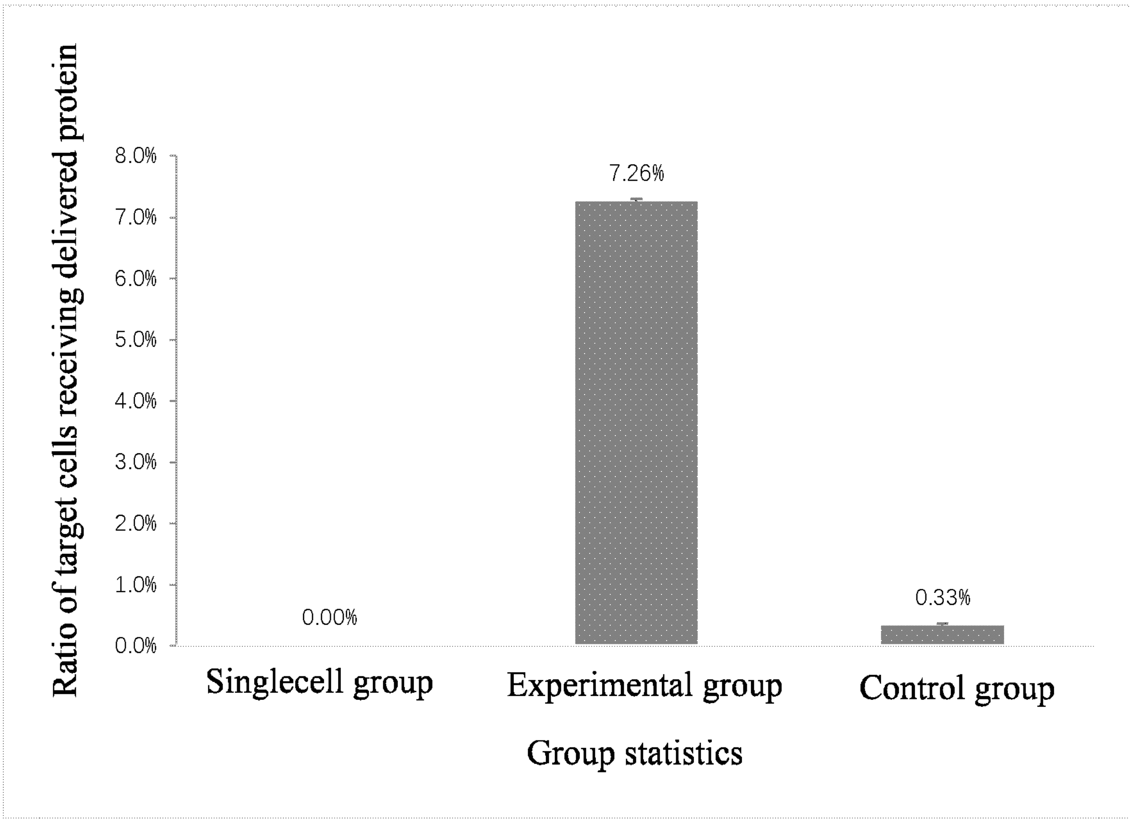
Ratio of target cells receiving delivered protein
8.0%
7.0%
6.0%
5.0%
4.0%
3.0%
2.0%
1.0%
0.0%
7.26%
0.00%
0.33%
Singlecell group
Experimental group
Control group
Group statistics

*Fig. 8*

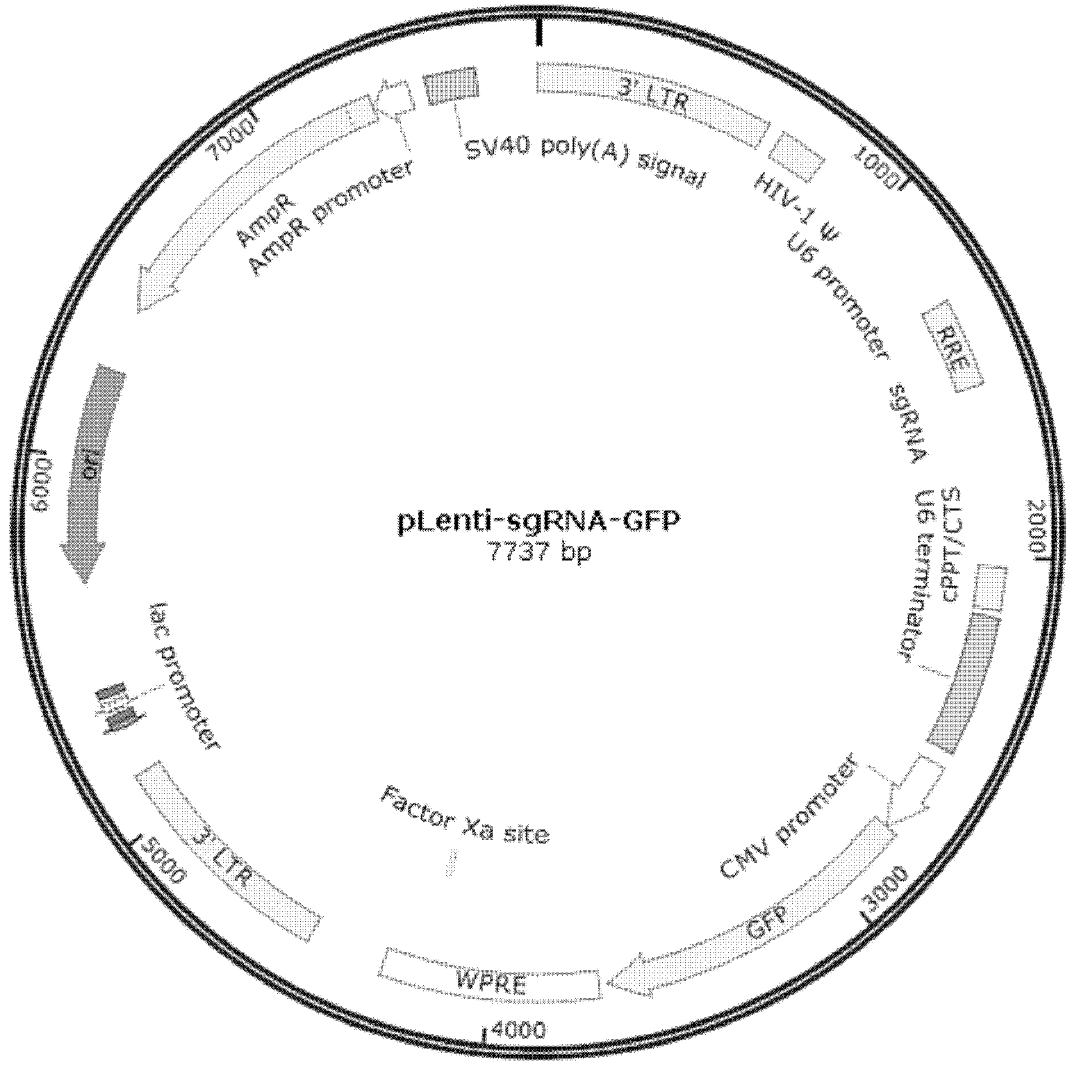
pLenti-sgRNA-GFP
7737 bp
3' LTR
1000
HIV-1 ψ
U6 promoter
RRE
sgRNA
cPPT/CTS
U6 terminator
2000
CMV promoter
3000
GFP
WPRE
4000
Factor Xa site
3' LTR
5000
lac promoter
6000
ori
7000
AmpR
AmpR promoter
SV40 poly(A) signal

*Fig. 9*

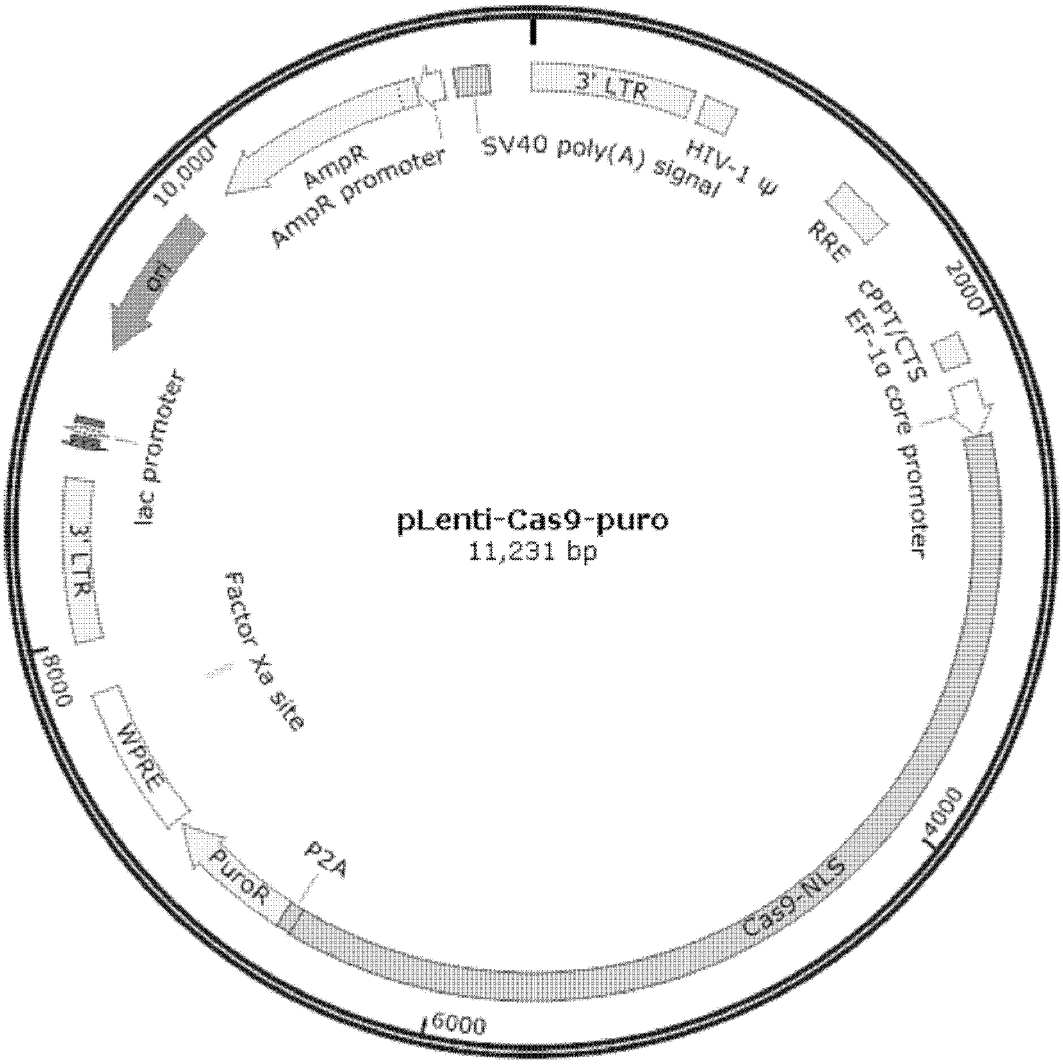
pLenti-Cas9-puro
11,231 bp
3' LTR
HIV-1 ψ
SV40 poly(A) signal
AmpR promoter
AmpR
10,000
ori
lac promoter
3' LTR
8000
Factor Xa site
WPRE
PuroR
P2A
6000
Cas9-NLS
4000
EF-1α core promoter
cPPT/CTS
2000
RRE

*Fig. 10*

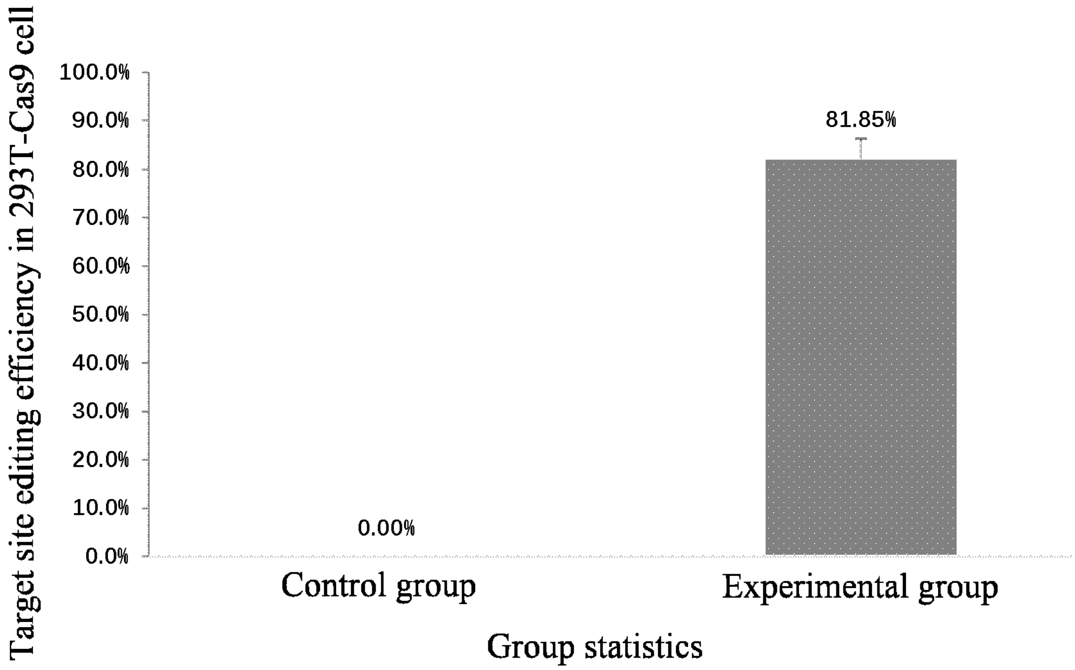
Target site editing efficiency in 293T-Cas9 cell
100.0%
90.0%
80.0%
70.0%
60.0%
50.0%
40.0%
30.0%
20.0%
10.0%
0.0%
0.00%
81.85%
Control group
Experimental group
Group statistics

*Fig. 11*

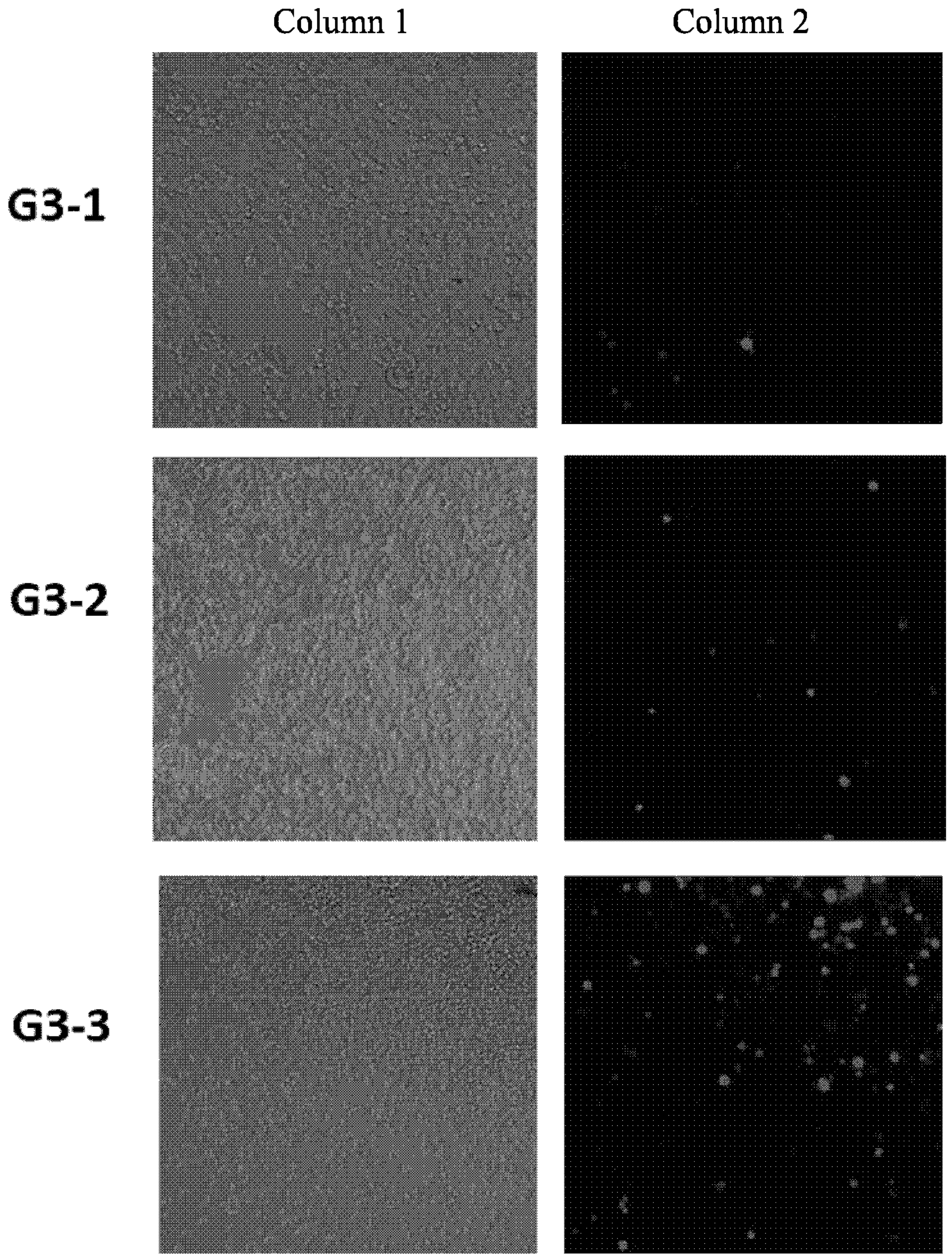


*Fig. 12*

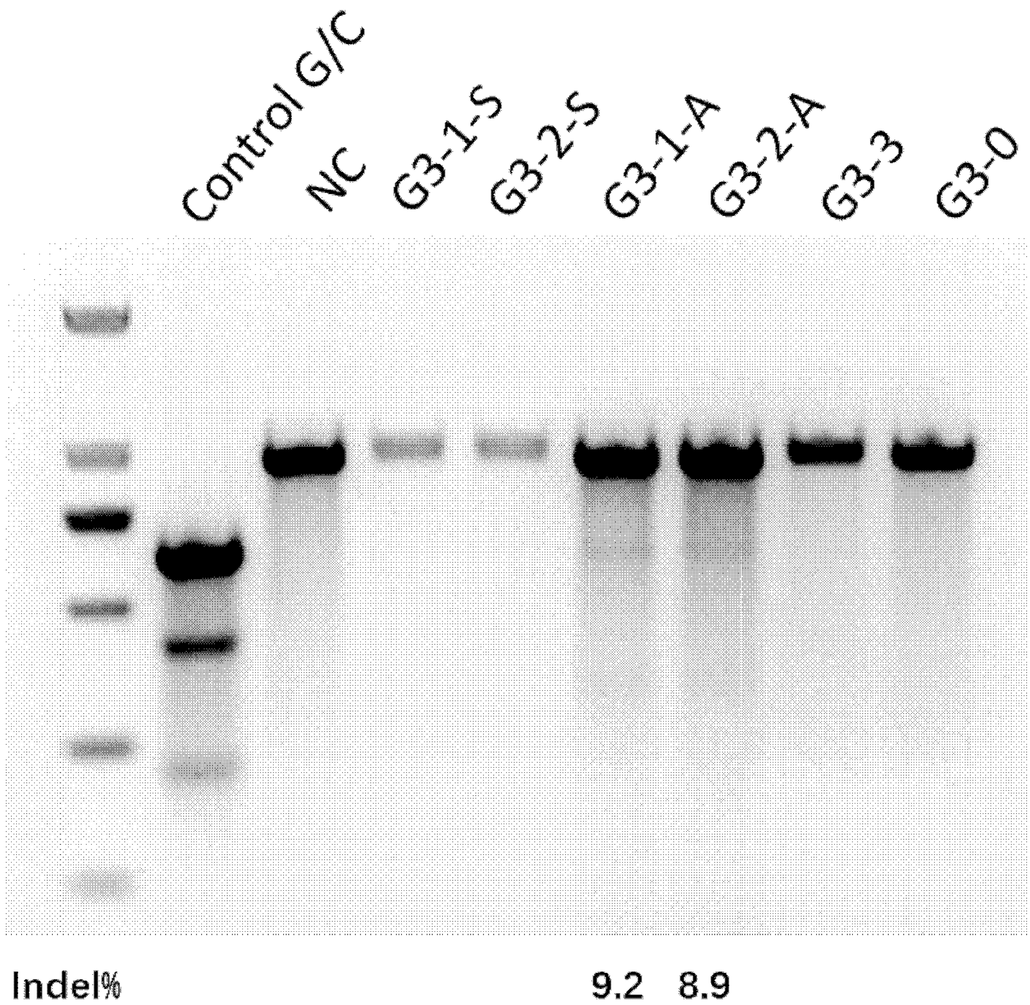


*Fig. 13*

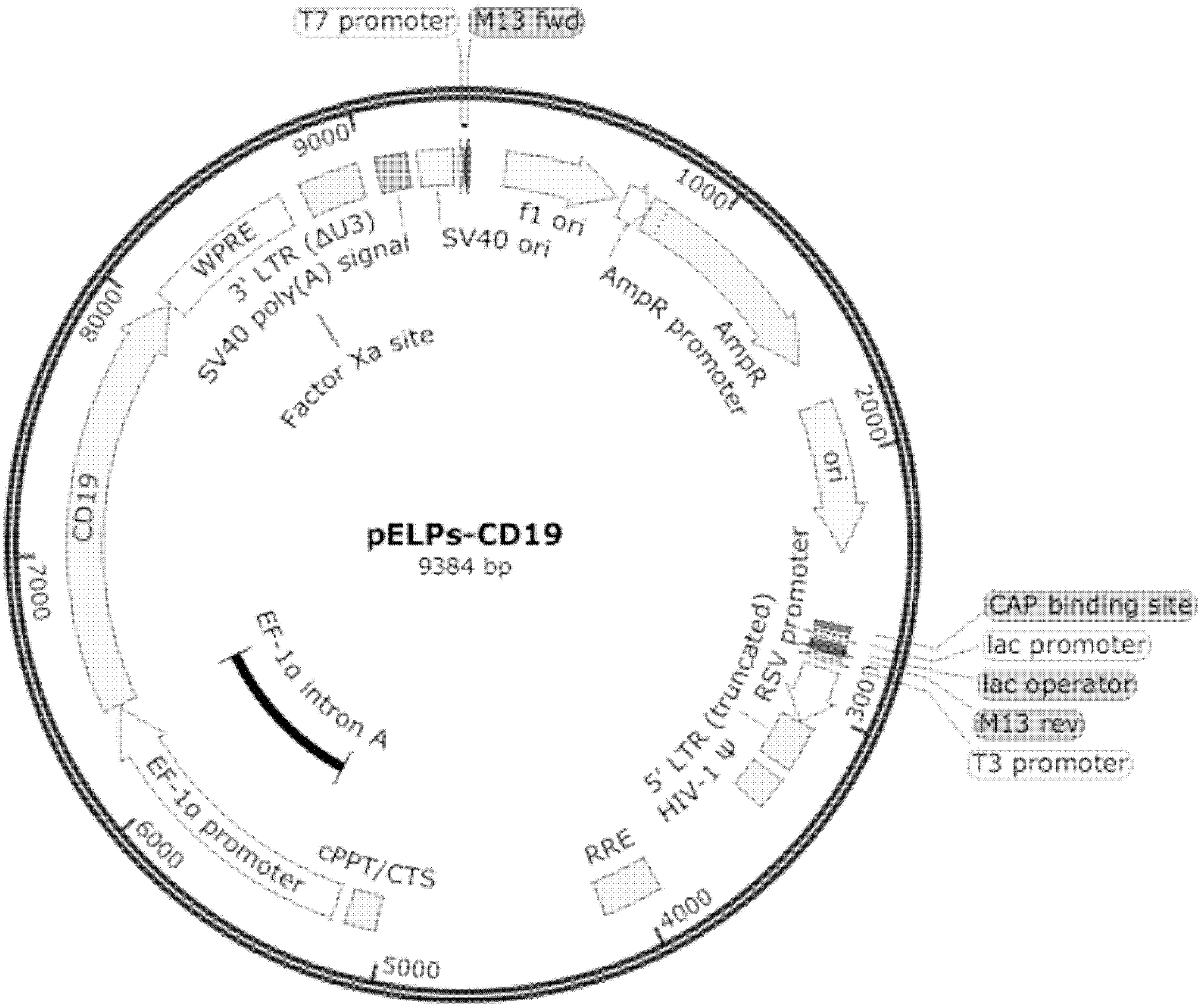
T7 promoter
M13 fwd
9000
1000
f1 ori
SV40 ori
AmpR promoter
AmpR
WPRE
3' LTR (ΔU3)
SV40 poly(A) signal
Factor Xa site
8000
2000
ori
CD19
pELPs-CD19
9384 bp
RSV promoter
5' LTR (truncated)
HIV-1 ψ
CAP binding site
lac promoter
lac operator
M13 rev
T3 promoter
3000
7000
EF-1α intron A
EF-1α promoter
6000
cPPT/CTS
RRE
4000
5000

*Fig. 14*

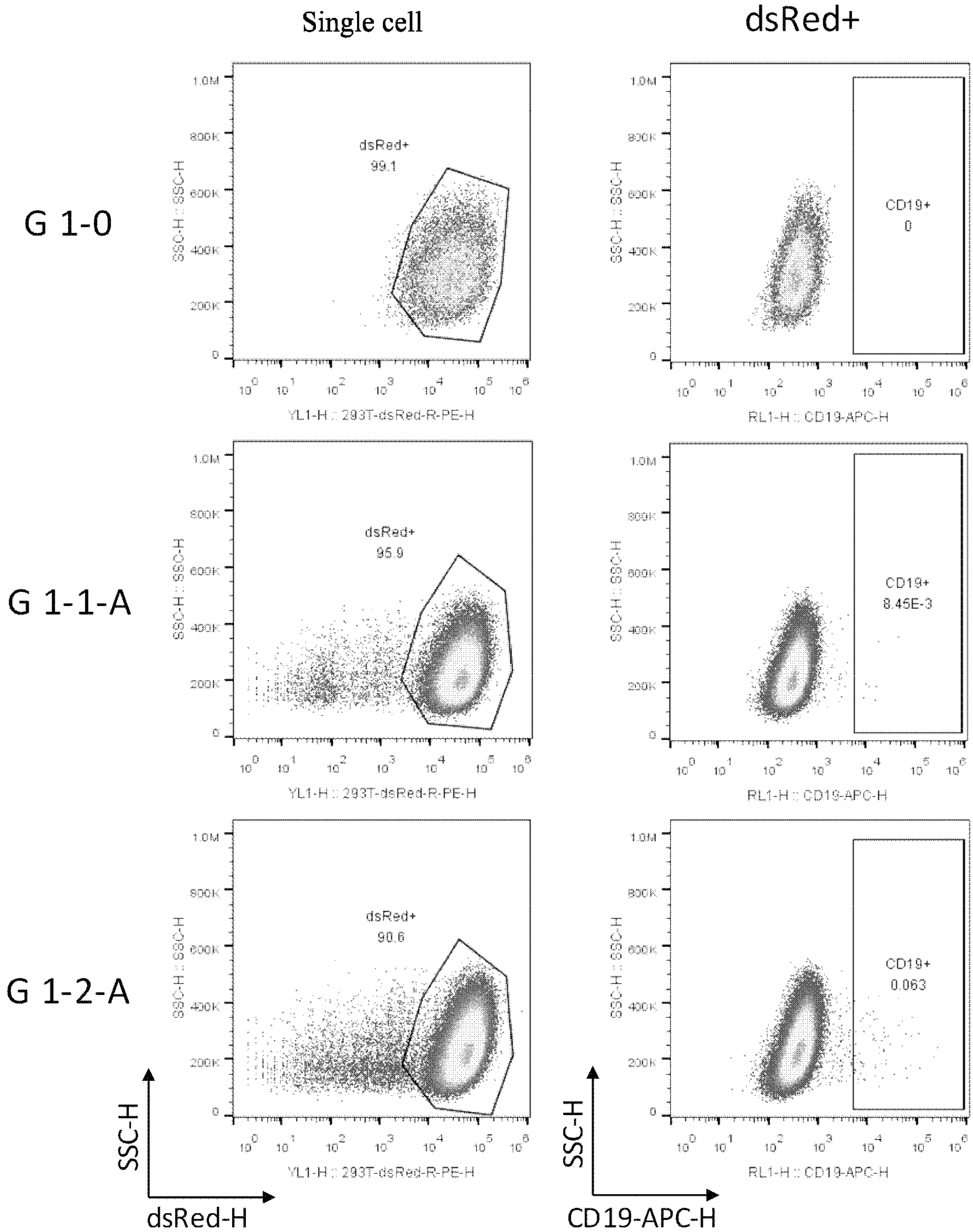
Single cell
dsRed+
G 1-0
G 1-1-A
G 1-2-A
dsRed+
99.1
95.9
90.6
CD19+
0
8.45E-3
0.063
SSC-H :: SSC-H
YL1-H :: 293T-dsRed-R-PE-H
RL1-H :: CD19-APC-H
SSC-H
dsRed-H
CD19-APC-H

*Fig. 15A*

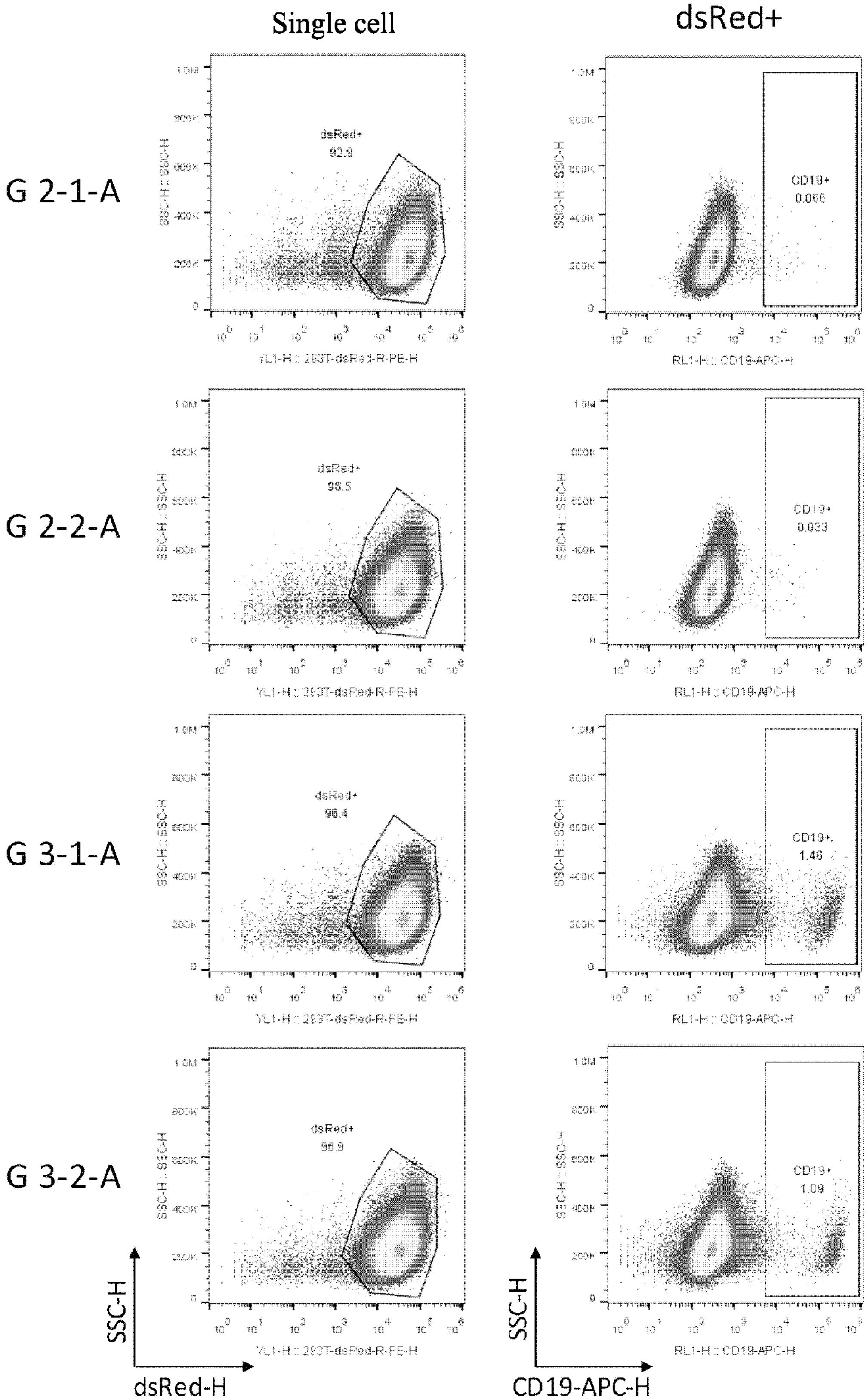
Single cell
dsRed+
G 2-1-A
G 2-2-A
G 3-1-A
G 3-2-A
dsRed+
92.9
dsRed+
96.5
dsRed+
96.4
dsRed+
96.9
CD19+
0.066
CD19+
0.033
CD19+
1.46
CD19+
1.09
YL1-H :: 293T-dsRed-R-PE-H
RL1-H :: CD19-APC-H
SSC-H :: SSC-H
SSC-H
dsRed-H
CD19-APC-H

*Fig. 15B*

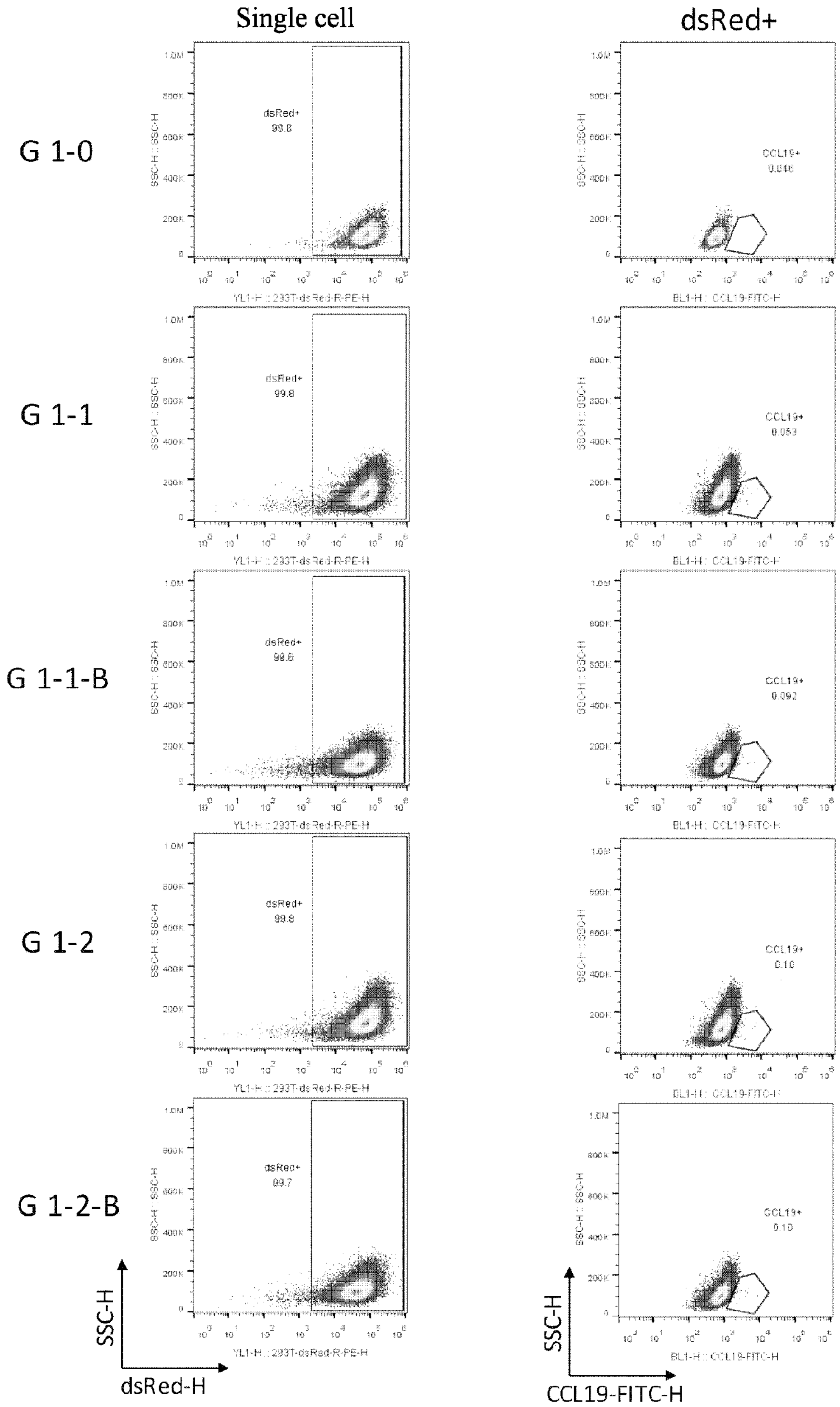
Single cell
dsRed+
G 1-0
G 1-1
G 1-1-B
G 1-2
G 1-2-B
dsRed+
99.8
dsRed+
99.8
dsRed+
99.8
dsRed+
99.8
dsRed+
99.7
CCL19+
0.046
CCL19+
0.053
CCL19+
0.092
CCL19+
0.10
CCL19+
0.10
SSC-H
dsRed-H
SSC-H
CCL19-FITC-H

*Fig. 16A*

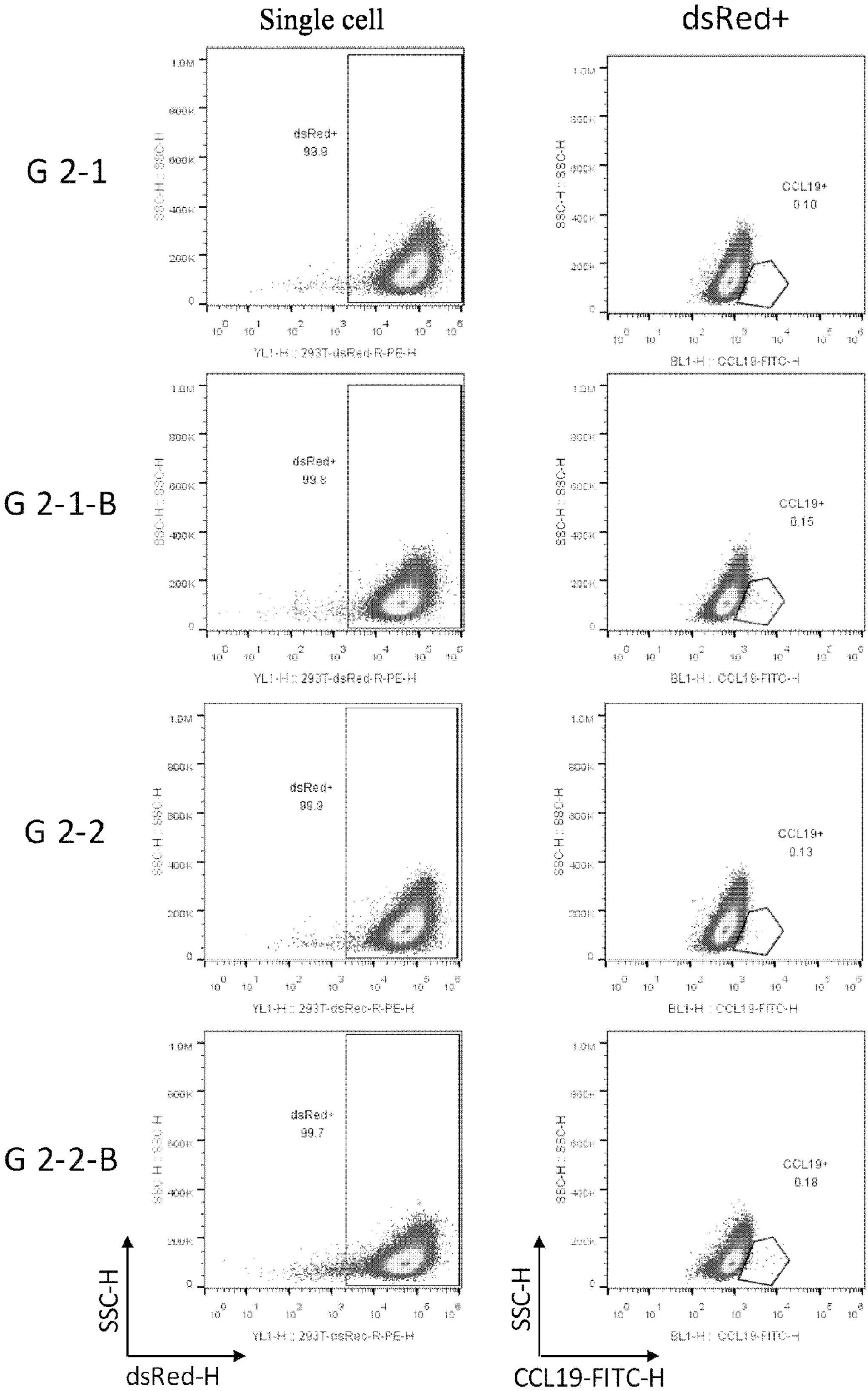
Single cell
dsRed+
G 2-1
G 2-1-B
G 2-2
G 2-2-B
dsRed+
99.9
99.8
99.9
99.7
CCL19+
0.10
0.15
0.13
0.18
YL1-H :: 293T-dsRed-R-PE-H
BL1-H :: CCL19-FITC-H
SSC-H :: SSC-H
SSC-H
dsRed-H
CCL19-FITC-H

*Fig. 16B*

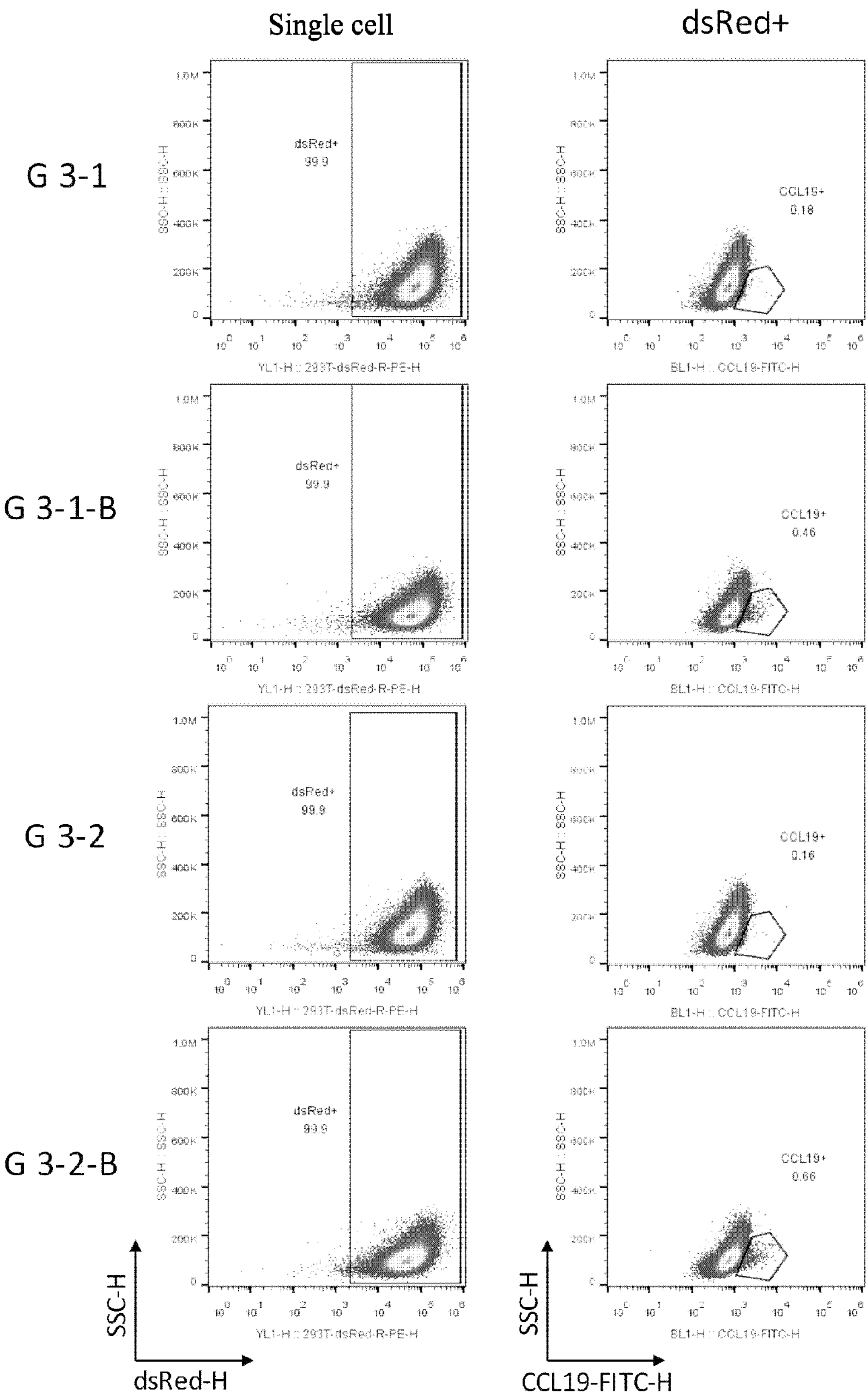
Single cell
dsRed+
G 3-1
G 3-1-B
G 3-2
G 3-2-B
dsRed+
99.9
CCL19+
0.18
0.46
0.16
0.66
YL1-H :: 293T-dsRed-R-PE-H
BL1-H :: CCL19-FITC-H
SSC-H :: SSC-H
SSC-H
dsRed-H
CCL19-FITC-H

*Fig. 16C*

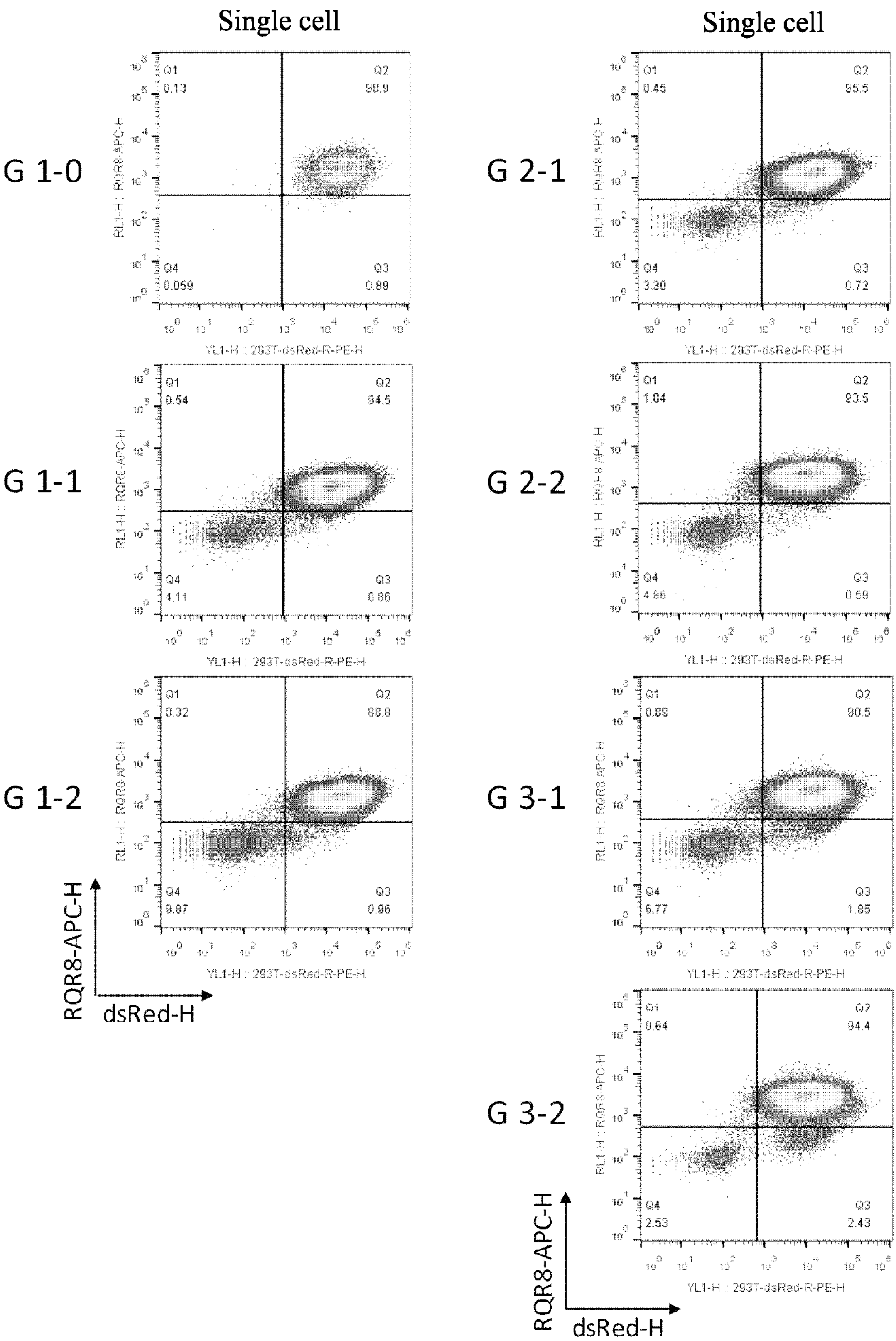
Single cell
Single cell
G 1-0
G 1-1
G 1-2
G 2-1
G 2-2
G 3-1
G 3-2
Q1 0.13
Q2 98.9
Q4 0.059
Q3 0.89
Q1 0.54
Q2 94.5
Q4 4.11
Q3 0.86
Q1 0.32
Q2 88.8
Q4 9.87
Q3 0.96
Q1 0.45
Q2 95.5
Q4 3.30
Q3 0.72
Q1 1.04
Q2 93.5
Q4 4.86
Q3 0.59
Q1 0.89
Q2 90.5
Q4 6.77
Q3 1.85
Q1 0.64
Q2 94.4
Q4 2.53
Q3 2.43
RL1-H :: RQR8-APC-H
YL1-H :: 293T-dsRed-R-PE-H
RQR8-APC-H
dsRed-H

*Fig. 17*

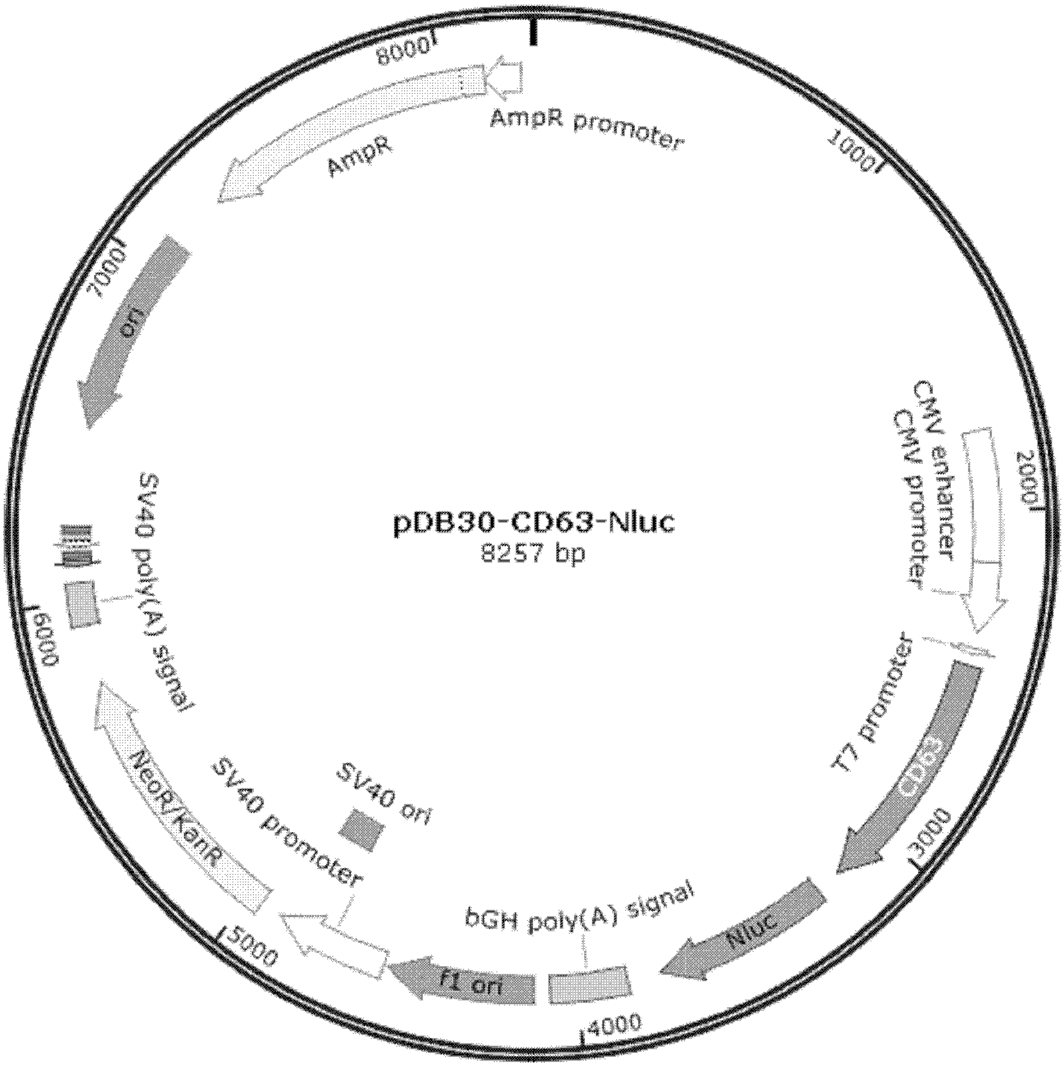
pDB30-CD63-Nluc
8257 bp
AmpR promoter
AmpR
ori
CMV enhancer
CMV promoter
T7 promoter
CD63
Nluc
bGH poly(A) signal
f1 ori
SV40 promoter
SV40 ori
NeoR/KanR
SV40 poly(A) signal
1000
2000
3000
4000
5000
6000
7000
8000

*Fig. 18*

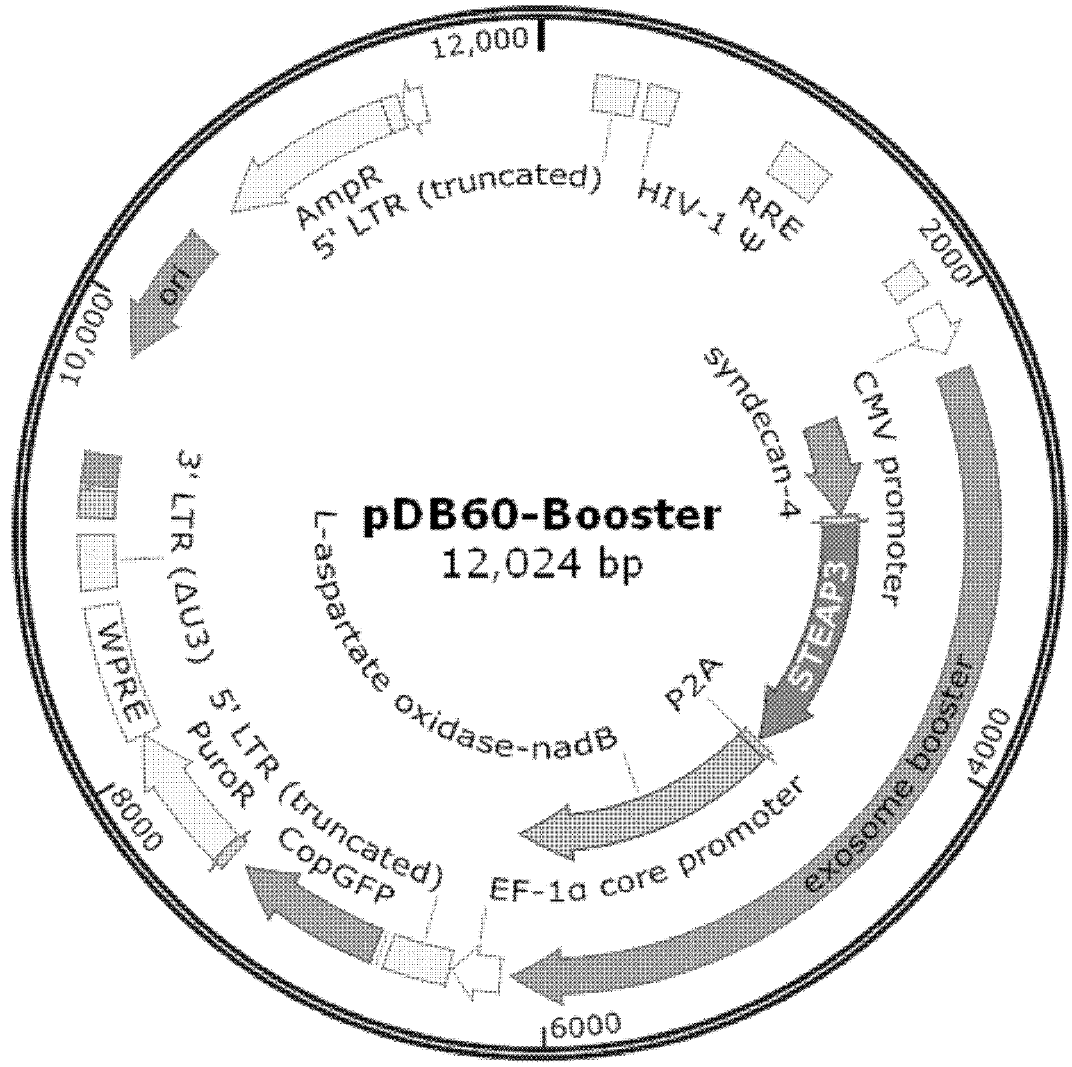
pDB60-Booster
12,024 bp
12,000
2000
4000
6000
8000
10,000
AmpR
5' LTR (truncated)
HIV-1 Ψ
RRE
ori
CMV promoter
syndecan-4
STEAP3
P2A
L-aspartate oxidase-nadB
exosome booster
EF-1α core promoter
CopGFP
5' LTR (truncated)
PuroR
WPRE
3' LTR (ΔU3)

*Fig. 19*

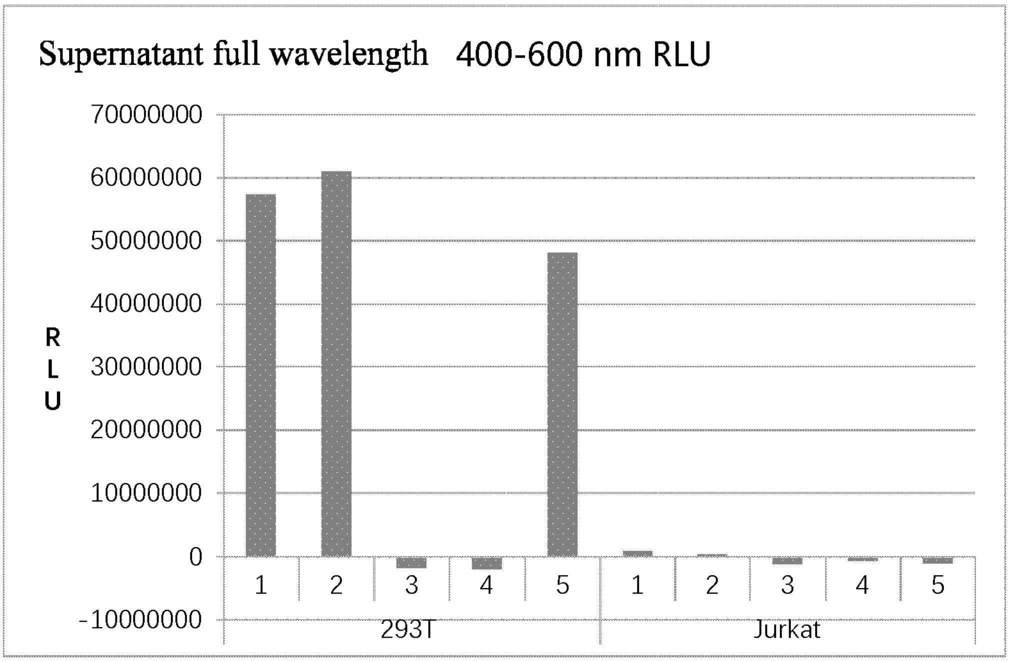


*Fig. 20A*

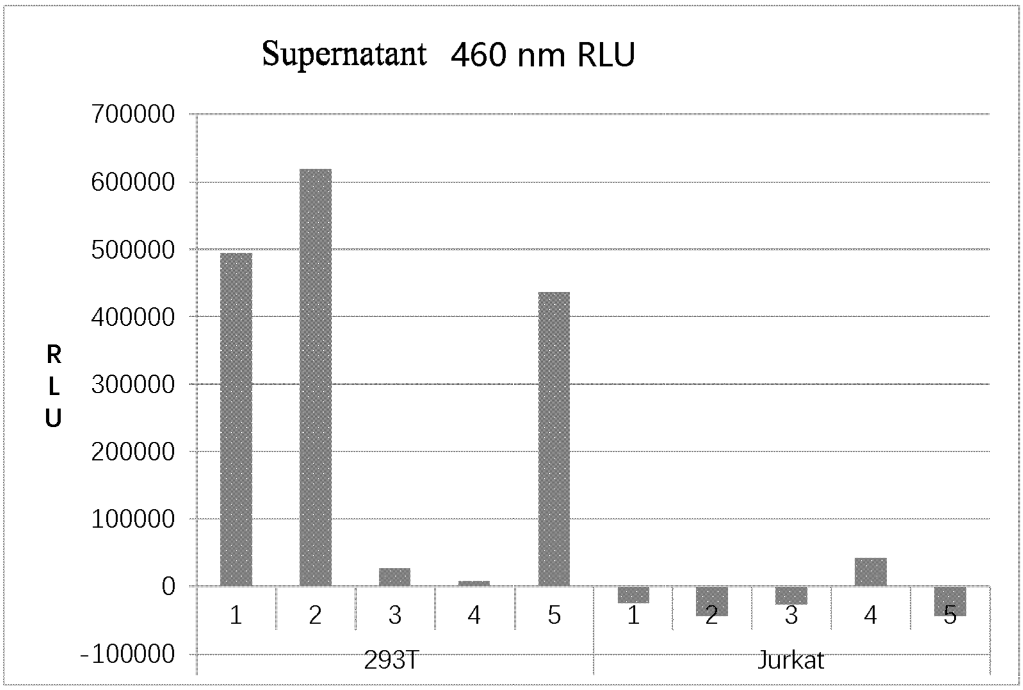


*Fig. 20B*

**INTERNATIONAL SEARCH REPORT**

International application No.
**PCT/CN2019/125565**

**A. CLASSIFICATION OF SUBJECT MATTER**

A61K 35/12(2015.01)i； C12N 15/12(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K; A61P; C12N

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, CNKI, 万方数据资源系统, 中国专利生物序列检索系统, 百度学术, PubMed, ISI Web of Knowledge, EMBL, Patentics: 申请人/发明人, 递送, 传递, 导入, 敲进, 转基因, 外泌体, 慢病毒, 病毒, 靶, 免疫细胞, B细胞, T细胞, NK细胞, DILIVER, KNOCKIN, INTRODUCE, TRANSGEN+, CRISPR, CAS9, CD19, CCL19, TARGET, B CELL, T CELL, NK CELL, EXOSOME, LENTIVIRUS, VIRUS, CAT-T, TCR-T, IMMUNOCYTE, IMMUNE CELL

**C. DOCUMENTS CONSIDERED TO BE RELEVANT**

| Category* | Citation of document, with indication, where appropriate, of the relevant passages | Relevant to claim No. |
|---|---|---|
| X | CN 108243607 A (SEATTLE CHILDREN'S HOSPITAL DBA SEATTLE CHILDREN'S RESEARCH INSTITUTE) 03 July 2018 (2018-07-03)<br>claims 1-59, 88-110, description, paragraphs [0179]-[0194] | 1-17, 23-25, 26-27 |
| A | CN 107530436 A (PHASERX INC) 02 January 2018 (2018-01-02)<br>entire document | 1-27 |
| A | CN 107074910 A (CELLIVERY THERAPEUTICS INC) 18 August 2017 (2017-08-18)<br>entire document | 1-27 |
| A | CN 108699557 A (NOVARTIS AG et al.) 23 October 2018 (2018-10-23)<br>entire document | 1-27 |
| A | CN 107759700 A (YINFENG BIOLOGICAL GROUP LTD.) 06 March 2018 (2018-03-06)<br>entire document | 1-27 |
| A | WO 2018031762 A1 (DUKE UNIVERSITY) 15 February 2018 (2018-02-15)<br>entire document | 1-27 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 March 2020** | **19 March 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2019/125565** |

**C. DOCUMENTS CONSIDERED TO BE RELEVANT**

| Category* | Citation of document, with indication, where appropriate, of the relevant passages | Relevant to claim No. |
|---|---|---|
| A | WO 2018081470 A1 (INTIMA BIOSCIENCE, INC. et al.) 03 May 2018 (2018-05-03)<br>entire document | 1-27 |
| A | MATTHEW J.J. et al. "Engineering of Primary Human B cells with CRISPR/CAS9 Targeted Nuclease"<br>*Scientific Reports,* Vol. 8, No. 1, 14 August 2018 (2018-08-14),<br>pp. 1-9 | 1-27 |

Form PCT/ISA/210 (second sheet) (January 2015)

**INTERNATIONAL SEARCH REPORT**

International application No.
**PCT/CN2019/125565**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-14,16-27**
because they relate to subject matter not required to be searched by this Authority, namely:

[1] Claims 1-14 and 16-27 relate to a method for delivering a biomacromolecule to a target cell, which belongs to disease treatment methods stipulated in PCT Rule 39.1(iv). The present report is formed on the basis of a use of an immune cell in preparing a drug which delivers a biomacromolecule to a target cell.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2019/125565**

| Patent document cited in search report | | | Publication date (day/month/year) | Patent family member(s) | | | Publication date (day/month/year) |
|---|---|---|---|---|---|---|---|
| CN | 108243607 | A | 03 July 2018 | AU | 2016318773 | A1 | 12 April 2018 |
| | | | | WO | 2017044487 | A1 | 16 March 2017 |
| | | | | EP | 3347026 | A1 | 18 July 2018 |
| | | | | JP | 2018528774 | A | 04 October 2018 |
| | | | | US | 2017087185 | A1 | 30 March 2017 |
| | | | | CA | 2997912 | A1 | 16 March 2017 |
| | | | | US | 10525082 | B2 | 07 January 2020 |
| | | | | EP | 3347026 | A4 | 08 May 2019 |
| CN | 107530436 | A | 02 January 2018 | EP | 3247328 | A1 | 29 November 2017 |
| | | | | AU | 2016209295 | A1 | 31 August 2017 |
| | | | | CA | 2974503 | A1 | 28 July 2016 |
| | | | | WO | 2016118697 | A9 | 29 September 2016 |
| | | | | WO | 2016118697 | A1 | 28 July 2016 |
| | | | | US | 2018221402 | A1 | 09 August 2018 |
| | | | | JP | 2018509387 | A | 05 April 2018 |
| CN | 107074910 | A | 18 August 2017 | WO | 2016028036 | A1 | 25 February 2016 |
| | | | | KR | 101971021 | B1 | 23 April 2019 |
| | | | | EP | 3180352 | A4 | 20 June 2018 |
| | | | | JP | 2017527280 | A | 21 September 2017 |
| | | | | AU | 2015304194 | B2 | 01 March 2018 |
| | | | | US | 2017240598 | A1 | 24 August 2017 |
| | | | | KR | 20170031243 | A | 20 March 2017 |
| | | | | EP | 3180352 | A1 | 21 June 2017 |
| | | | | AU | 2015304194 | A1 | 02 February 2017 |
| | | | | CA | 2957501 | A1 | 25 February 2016 |
| | | | | KR | 20190041551 | A | 22 April 2019 |
| | | | | US | 10323063 | B2 | 18 June 2019 |
| | | | | JP | 6559227 | B2 | 14 August 2019 |
| CN | 108699557 | A | 23 October 2018 | KR | 20180133840 | A | 17 December 2018 |
| | | | | EA | 201891338 | A1 | 28 December 2018 |
| | | | | PH | 12018501159 | A1 | 21 January 2019 |
| | | | | US | 2018362975 | A1 | 20 December 2018 |
| | | | | IL | 259576 | D0 | 31 July 2018 |
| | | | | CA | 3006432 | A1 | 08 June 2017 |
| | | | | AU | 2016362129 | A1 | 14 June 2018 |
| | | | | JP | 2018536436 | A | 13 December 2018 |
| | | | | SG | 11201804373V | A | 28 June 2018 |
| | | | | MX | 2018006767 | A | 14 March 2019 |
| | | | | WO | 2017093969 | A1 | 08 June 2017 |
| | | | | EP | 3384027 | A1 | 10 October 2018 |
| | | | | BR | 112018011089 | A2 | 04 December 2018 |
| CN | 107759700 | A | 06 March 2018 | | None | | |
| WO | 2018031762 | A1 | 15 February 2018 | EP | 3497221 | A1 | 19 June 2019 |
| | | | | US | 2019194633 | A1 | 27 June 2019 |
| WO | 2018081470 | A1 | 03 May 2018 | GB | 201906850 | D0 | 26 June 2019 |
| | | | | US | 2019374576 | A1 | 12 December 2019 |
| | | | | AU | 2017347848 | A1 | 23 May 2019 |
| | | | | CA | 3041835 | A1 | 03 May 2018 |
| | | | | EP | 3532079 | A2 | 04 September 2019 |

Form PCT/ISA/210 (patent family annex) (January 2015)

**INTERNATIONAL SEARCH REPORT**
**Information on patent family members**

International application No.
**PCT/CN2019/125565**

| Patent document cited in search report | Publication date (day/month/year) | Patent family member(s) | | | Publication date (day/month/year) |
|---|---|---|---|---|---|
| | | WO | 2018081476 | A9 | 19 July 2018 |
| | | GB | 2573663 | A | 13 November 2019 |
| | | AU | 2017347854 | A1 | 23 May 2019 |
| | | JP | 2019536447 | A | 19 December 2019 |
| | | US | 2019382799 | A1 | 19 December 2019 |
| | | WO | 2018081476 | A2 | 03 May 2018 |
| | | CN | 110545827 | A | 06 December 2019 |
| | | JP | 2019531755 | A | 07 November 2019 |
| | | WO | 2018081476 | A3 | 07 June 2018 |
| | | GB | 2573664 | A | 13 November 2019 |
| | | CA | 3041831 | A1 | 03 May 2018 |
| | | EP | 3532075 | A1 | 04 September 2019 |
| | | GB | 201906849 | D0 | 26 June 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NATHAN S et al.** *Current Hematologic Malignancy Reports,* 2017, vol. 10
- **ISABELLE R et al.** *Molecular Therapy,* 2017, vol. 25 (5
- **ADAM S. C et al.** *Molecular Biotechnology,* 2007, vol. 36 (3), 184-204
- **RYOSUKE K et al.** *Nature communications,* 2017, vol. 9, 1305
- **CHEN-YUAN K et al.** *Sci. Adv.,* 2018, vol. 4, eaau6762
- **CONG et al.** *Science,* 2013, vol. 339, 819-823
- **QI et al.** *Cell,* 2013, vol. 152, 1173-1183
- **CHRISTOPHER A. V et al.** *Nature Medicine,* 2018, (24), 1216-1224
- **JOHNNY H. H et al.** *Nature,* 2018, vol. 556 (7699), 57-63
- **JANA MUROVEC et al.** *Plant Biotechnology Journal,* 2017, vol. 15, 917-926
- **MA et al.** *ACS Synth. Biol.,* 2018, vol. 7 (4), 978-985
- **KOMOR et al.** *Sci. Adv.,* 2017, vol. 3, eaao4774
- **ZALATAN et al.** *Cell,* 2015, vol. 160, 339-350

SEQUENCE LISTING

<110> Cure Genetics Co., Limited

<120> METHOD FOR DELIVERING GENE IN CELLS

<130> AJ190048PCT

<160> 37

<170> PatentIn version 3.5

<210> 1
<211> 25
<212> DNA
<213> Artificial sequence

<400> 1
caccggtcac caatcctgtc cctag 25

<210> 2
<211> 25
<212> DNA
<213> Artificial sequence

<400> 2
aaaccagtgg ttaggacagg gatcc 25

<210> 3
<211> 25
<212> DNA
<213> Artificial sequence

<400> 3
attaacgctt acaatttcct gatgc 25

<210> 4
<211> 24
<212> DNA
<213> Artificial sequence

<400> 4
atgtgagcaa aaggccagca aaag 24

<210> 5
<211> 37
<212> DNA
<213> Artificial sequence

<400> 5
aatcctgtcc gtctagagat gcattagtta ttaatag 37

<210> 6
<211> 30
<212> DNA
<213> Artificial sequence

<400> 6

aaatgtggta aagcttctaa tcaattactg 30

<210> 7
<211> 36
<212> DNA
<213> Artificial sequence

<400> 7
ttagaagctt taccacattt gtagaggttt tacttg 36

<210> 8
<211> 48
<212> DNA
<213> Artificial sequence

<400> 8
aatcctgtcc gtctagagat gcattagtta ttaatagtaa tcaattac 48

<210> 9
<211> 40
<212> DNA
<213> Artificial sequence

<400> 9
aggaaattgt aagcgttaat ccaggaaccc ctgtagggaa 40

<210> 10
<211> 33
<212> DNA
<213> Artificial sequence

<400> 10
atctctagac ggacaggatt ggtgacagaa aag 33

<210> 11
<211> 44
<212> DNA
<213> Artificial sequence

<400> 11
aaatgtggta aagcttctaa tcaattactg ggagccggag tggc 44

<210> 12
<211> 40
<212> DNA
<213> Artificial sequence

<400> 12
tgctggcctt ttgctcacat ctgtgccgct ttctgtctgc 40

<210> 13
<211> 20
<212> DNA
<213> Artificial sequence

<400> 13

cggaactctg ccctctaacg 20

<210> 14
<211> 21
<212> DNA
<213> Artificial sequence

<400> 14
cttcttggcc acgtaacctg a 21

<210> 15
<211> 20
<212> DNA
<213> Artificial sequence

<400> 15
caccaaccac aacgaggact 20

<210> 16
<211> 20
<212> DNA
<213> Artificial sequence

<400> 16
tagggggcgt acttggcata 20

<210> 17
<211> 20
<212> DNA
<213> Artificial sequence

<400> 17
cttctccgac ggatgtctcc 20

<210> 18
<211> 20
<212> DNA
<213> Artificial sequence

<400> 18
aggtgggggt tagacccaat 20

<210> 19
<211> 110
<212> RNA
<213> Artificial sequence

<400> 19
gaggauguuc aauaacugug guuucagagc uaugcuggaa acagcauagc aaguugaaau 60

aaggcuaguc cguuaucaac uugaaaaagu ggcaccgagu cggugcuuuu 110

<210> 20
<211> 27
<212> DNA
<213> Artificial sequence

<400> 20
cctactggag ctcttacagg ttaatcc 27

<210> 21
<211> 29
<212> DNA
<213> Artificial sequence

<400> 21
ctctcgtttg tagctctgta agacttgtc 29

<210> 22
<211> 23
<212> DNA
<213> Artificial sequence

<400> 22
cacctcctcc gccgccagaa cac 23

<210> 23
<211> 23
<212> DNA
<213> Artificial sequence

<400> 23
aaacgtgttc tggcggcgga gga 23

<210> 24
<211> 23
<212> DNA
<213> Artificial sequence

<400> 24
caccatccta gcctgtgtag cgg 23

<210> 25
<211> 23
<212> DNA
<213> Artificial sequence

<400> 25
aaacccgcta cacaggctag gat 23

<210> 26
<211> 23
<212> DNA
<213> Artificial sequence

<400> 26
caccggatct gaactgccta cac 23

<210> 27
<211> 23
<212> DNA
<213> Artificial sequence

<400> 27
aaacgtgtag gcagttcaga tcc 23

<210> 28
<211> 23
<212> DNA
<213> Artificial sequence

<400> 28
caccccaatc ctagcctgtg tag 23

<210> 29
<211> 23
<212> DNA
<213> Artificial sequence

<400> 29
aaacctacac aggctaggat tgg 23

<210> 30
<211> 23
<212> DNA
<213> Artificial sequence

<400> 30
caccgatctg aactgcctac aca 23

<210> 31
<211> 23
<212> DNA
<213> Artificial sequence

<400> 31
aaactgtgta ggcagttcag atc 23

<210> 32
<211> 23
<212> DNA
<213> Artificial sequence

<400> 32
cacccaagcg gtggtggtag gct 23

<210> 33
<211> 23
<212> DNA
<213> Artificial sequence

<400> 33
aaacagccta ccaccaccgc ttg 23

<210> 34
<211> 23
<212> DNA
<213> Artificial sequence

```
<400>  34
caccttggtg gacacgttgc tga                                              23


<210>  35
<211>  23
<212>  DNA
<213>  Artificial sequence

<400>  35
aaactcagca acgtgtccac caa                                              23


<210>  36
<211>  23
<212>  DNA
<213>  Artificial sequence

<400>  36
cacccagggc catccaacac agc                                              23


<210>  37
<211>  23
<212>  DNA
<213>  Artificial sequence

<400>  37
aaacgctgtg ttggatggcc ctg                                              23
```